# EUROPEAN PATENT APPLICATION

(11) **EP 2 270 053 A1**
(43) Date of publication of application: **05.01.2011**
(21) Application number: 09006355.3
(22) Date of filing: 11.05.2009
(51) Int. Cl.: C07K 16/28, A61K 39/395, C12N 15/13, A61K 31/704, A61K 31/282, A61P 35/00

(54) **Humanized AXL antibodies**

(71) Applicant: U3 Pharma GmbH, 82152 Martinsried (DE)
(72) Inventor: Wirtz, Peter, 82131 Gauting (DE); Ruhe, Jens, 82152 Martinsried (DE); Takizawa, Takeshi, Shinagawa-ku Tokyo 140-8710 (JP); Takayama, Tomoko, Shinagawa-ku Tokyo 140-8710 (JP)
(74) Representative: Weiss, Wolfgang

(57) **Abstract**

The present invention refers to monoclonal humanized antibodies, which bind to the extracellular domain of the AXL receptor tyrosine kinase and which at least partially inhibit AXL activity.

## Description

The present invention refers to monoclonal humanized antibodies, which bind to the extracellular domain of the AXL receptor tyrosine kinase and which at least partially inhibit AXL activity.

### Background

The AXL (Ark, UFO, Tyro-7) receptor tyrosine kinase is a member of the Tyro-3 family of kinases with the other members being Mer (Eyk, Nyk, Tyro-12) and Sky (Rse, Tyro-3, Dtk, Etk, Brt, Tif). It is activated by binding of the heterophilic ligand Gas6, a 70-kDa protein homologous to the anticoagulation factor protein S. In contrast to other receptor tyrosine kinases, AXL tyrosine phosphorylation can also be induced by homophilic binding. AXL activation leads to signalling through PI-3-kinase/Akt (Franke et al., Oncogene 22: 8983-8998, 2003) and other major pathways like Ras/Erk and β-catenin/TCF (Goruppi et al., Mol. Cell Biol. 21: 902-915, 2001).

AXL is weakly expressed in a range of normal tissues, including brain, heart, skeletal muscle, the organ capsules and connective tissues of several other organs, and in monocytes, but not lymphocytes. Akt phosphorylation induced by AXL has been described in survival of fibroblasts (Goruppi et al., Mol Cell Biol 17: 4442-4453 1997), endothelial cells (Hasanbasic et al., Am J Physiol Heart Circ Physiol, 2004), vascular smooth muscle cells (Melaragno et al., J. Mol. Cell Cardiol. 37: 881-887, 2004) and neurons (Allen et al., Mol. Endocrinol. 13: 191-201 1999). Furthermore, AXL plays a role in cell-adhesion and chemotaxis. AXL knockouts display impaired platelet aggregate stabilization and thrombus formation as a result of reduced activation of the platelet integrin IIb3.

AXL overexpression has been demonstrated in various cancer types, e.g. breast (Meric et al., Clin. Cancer Res. 8: 361-367, 2002; Berclaz et al., Ann. Oncol. 12: 819-824, 2001), colon (Chen et al., Int. J. Cancer 83: 579-584, 1999; Craven et al., Int. J. Cancer 60: 791-797, 1995), prostate (Jacob et al., Cancer Detect. Prev. 23: 325-332, 1999), lung (Wimmel et al., Eur J Cancer 37: 2264-2274, 2001), gastric (Wu et al., Anticancer Res 22: 1071-1078, 2002), ovarian (Sun et al., Oncology 66: 450-457, 2004), endometrial (Sun et al., Ann. Oncol. 14: 898-906, 2003), renal (Chung et al., DNA Cell Biol. 22: 533-540, 2003), hepatocellular (Tsou et al., Genomics 50:331-340, 1998), thyroid (Ito et al., Thyroid 12:971-975, 2002; Ito et al., Thyroid 9: 563-567, 1999), and esophageal carcinoma (Nemoto et al., 1997), furthermore in CML (Janssen et al., A novel putative tyrosine kinase receptor with oncogenic potential. Oncogene, 6: 2113-2120, 1991; Braunger et al., Oncogene 14:2619-2631 1997; O'Bryan et al., Mol Cell Biol 11:5016-5031,1991), AML (Rochlitz et al., Leukemia 13: 1352-1358, 1999), osteosarcoma (Nakano et al., J. Biol. Chem. 270:5702-5705, 2003) melanoma (van Ginkel et al.,. Cancer Res 64:128-134, 2004) and in head and neck squamous cell carcinoma (Green et al., Br J Cancer. 2006 94:1446-5, 2006).

Moreover AXL has been identified as a metastasis-associated gene that is upregulated in aggressive breast cancer cell lines compared to non-invasive cells. In vitro, AXL activity was found to be required for migration and invasion, and this activity could be inhibited by antibody treatment (WO04008147). Similarly, abrogation of AXL activity in vivo, either via expression of a dominant negative version of AXL (Vajkoczy, P., et al., Proc. Natl. Acad. Science U.S.A. 103: 5799-5804. 2005) or by siRNA mediated downregulation of AXL (Holland et al., Cancer Res. 65: 9294-9303, 2005) prevented subcutaneous and orthotopic cell growth in murine xenograft experiments.

So far antibodies that bind to AXL and posses biological activity have been described. For example, one known antibody is capable of reducing AXL mediated cell invasion (WO04008147) whereas an other antibody has been reported to reduce AXULigand interaction. However, all known antibodies are polyclonal or non-humanized monoclonal antibodies. Non-humanized antibodies, polyclonal or monoclonal, are rapidly removed from circulation and usually cause systemic inflammatory effects, rendering them unsuitable for therapeutic administration.

Thus in light of the therapeutic potential of AXL there is a high need for monoclonal humanized AXL antibodies, antibody fragments or derivatives thereof that effectively and specifically block AXL mediated signal transduction and which are suitable for therapeutic treatment.

Accordingly a first aspect of the present invention relates to a monoclonal humanized antibody including a fragment or derivative thereof that binds to the extracellular domain of AXL, particularly of human AXL, and at least partially inhibits AXL activity.

Preferably the humanized antibody of the present invention possesses at least one or more of the following properties: the ability to reduce or block AXL-mediated signal transduction, the ability to reduce or block AXL phosphorylation, the ability to reduce or block cell proliferation, the ability to reduce or block angiogenesis, the ability to reduce or block cell migration, the ability to reduce or block tumor metastasis, and the ability to reduce or block AXL mediated anti-apoptosis, thereby increasing for example the sensitivity of a cell against treatment with an antineoplastic agent.

According to an especially preferred embodiment of the invention the humanized antibodies described herein show the ability to reduce and/or block ligand induced phosphorylation of AXL downstream signaling molecules such as ERK1/2, AKT, GSK-3β, TSC2, mTOR and/or S6K1.

Moreover the humanized antibodies of the present invention may exhibit high specificity for AXL, particularly human AXL and do not significantly recognize other Tyro-3 family members, e.g. MER and/or SKY and/or mammalian non-primate AXL, such as murine AXL.

The term "activity" as used herein refers to the biological function of AXL, which influences the phenotype of a cell, in particular but not limited to cancer phenotypes such as evasion of apoptosis, self sufficiency in growth signals, cell proliferation, tissue invasion and/or metastasis, insensitivity to anti-growth signals (anti-apoptosis) and/or sustained angiogenesis.

The term "AXL mediated signal transduction" means the activation of second messenger pathways, such as downstream signaling, triggered by direct or indirect interaction of AXL with second messenger molecules.

The term "AXL phosphorylation" refers to the phosphorylation of amino acid residues, preferably tyrosine residues, either by a second AXL protein (transphosphorylation) or by another protein having protein kinase activity.

The term "cell proliferation" refers to all AXL-involving processes underlying the reproduction of human cells, in particular but not limited to human cancer cells. They contribute to or result in the replication of cellular DNA, separation of the duplicated DNA into two equally sized groups of chromosomes, and the physical division (called cytokinesis) of entire cells, and shall be stimulated or mediated by non-catalytic or catalytic activities of AXL, preferably including AXL phosphorylation and/or AXL-mediated signal transduction.

The term "angiogenesis" refers to all AXL-involving processes that contribute to the growth of new blood vessels from pre-existing vessels, in particular but not limited to new tumor supplying blood vessels. These processes include multiple cellular events such as proliferation, survival, migration and sprouting of vascular endothelial cells, attraction and migration of pericytes as well as basal membrane formation for vessel stabilization, vessel perfusion, or secretion of angiogenic factors by stromal or neoplastic cells, and shall be stimulated or mediated by non-catalytic or catalytic activities of AXL, preferably including AXL phosphorylation and/or AXL-mediated signal transduction.

The term "metastasis" refers to all AXL-involving processes that support cancer cells to disperse from a primary tumor, penetrate into lymphatic and/or blood vessels, circulate through the bloodstream, and grow in a distant focus (metastasis) in normal tissues elsewhere in the body. In particular, it refers to cellular events of tumor cells such as proliferation, migration, anchorage independence, evasion of apoptosis, or secretion of angiogenic factors, that underly metastasis and are stimulated or mediated by non-catalytic or catalytic activities of AXL, preferably including AXL phosphorylation and/or AXL-mediated signal transduction.

The term "AXL mediated anti-apoptosis" refers to all AXL-involving processes that prevent human cells, preferably but not limited to human cancer cells from programmed cell death (apoptosis). In particular, it refers to processes that prevent human cells, preferably but not limited to human cancer cells from induction of apoptosis through growth factor withdrawal, hypoxia, exposure to chemotherapeutic agents or radiation, or initiation of the Fas/Apo-1 receptor-mediated signaling, and are stimulated or mediated by non-catalytic or catalytic activities of AXL, preferably including AXL phosphorylation and/or AXL-mediated signal transduction.

The terms "region" and "domain" are compatible with each other in the invention.

The antibodies designated as "11B7" and "11D5" may be also designated as "#11B7" and "#11D5", respectively, in the invention.

According to a second aspect of the invention the humanized antibodies described herein are derived from one of the chimeric (rat/human) anti-AXL antibodies 11 B7 (the amino acid sequence of its light and heavy chain are represented by SEQ ID NO: 135 and 136, respectively), 11 D5 (the amino acid sequence of its light and heavy chain are represented by SEQ ID NO: 137 and 138, respectively) or 10D12 or an antibody recognizing the same epitope on the extracellular domain of AXL. Particular preferred, the humanized antibodies are derived from 11 B7 and 11D5. Preferably the humanized antibody contains at least one mutation in the frame work region of at least one variable domain. Such mutations may be introduced by any method known to the person skilled in the art to alter amino acid sequences.

The mutation replaces preferably an amino acid in an frame work region of 11 B7 or 11 D5 by an amino acid which is conserved in human frame work regions. Methods for determining conserved or consensus amino acids in human frame work regions, such for example homology modelling using programs such as IgBLAST are known to the person skilled in the art.
According to another preferred embodiment, the humanized antibodies of the invention are derived from anti-AXL antibodies, preferably from anti-AXL antibodies 11 B7 or 11 D5, wherein the frame work regions of at least one variable domain of the antibody, preferably 11 B7 or 11 D5, been replaced by a human or humanized frame work region.

The binding activity of an antibody of the present invention to AXL can be determined by methods known to those skilled in the art. For example, the activity can be determined using surface plasmon resonance with Biacore, and/or by ELISA (enzyme-linked immunosorbent assays), EIA (enzyme immunoassays), RIA (radioimmunoassays), or fluorescent antibody techniques, e.g. FACS.

Preferably the humanized antibodies of the invention are less immunogenic if compared with polyclonal or monoclonal non-humanized anti-AXL antibodies such as rat 11 B7 or 11 D5. Beside other methods known to those skilled in the art reduced immunogenicity may be determined by ELISA based HAHA or HAMA assays (IBL-America, Minnesota / LiSrarFish, Italy). Moreover, preferably the inventive antibodies are not rapidly removed from blood circulation and do not cause systemic inflammatory effects if administered to a patient.

The antibodies of the invention may have at least one antigen binding site, e.g. one or two antigen binding sites. Further, the antibody preferably comprises at least one heavy immunoglobulin chain and at least one light immunoglobulin chain. An immunoglobulin chain comprises a variable domain and optionally a constant domain. A variable domain may comprise complementary determining regions (CDRs), e.g. a CDR1, CDR2 and/or CDR3 region, and framework regions. The term "complementary determining region" (CDR) is well-defined in the art (see, for example, Harlow and Lane, "Antibodies, a Laboratory Manual", CSH Press, Cold Spring Harbour, 1988) and refers to the stretches of amino acids within the variable region of an antibody that primarily makes contact with the antigen.

According to a further embodiment the humanized antibodies of the invention are derived from the anti-AXL antibody 11 B7 and may comprise a heavy chain amino acid sequence selected from the group consisting of SEQ ID NO:40 to SEQ ID NO:48 , or at least the variable domain thereof, or an amino acid sequence having a sequence identity of at least 90% thereto and represents a variable region having binding activity to Axl which is equivalent to that of one having a sequence identity of 100%
or a variable domain of a heavy chain amino acid sequence selected from the group consisting of SEQ ID NO:80 to SEQ ID NO:82 or an amino acid sequence having a sequence identity of at least 90% thereto and represents a variable region having binding activity to Axl which is equivalent to that of one having a sequence identity of 100%
and/or a light chain amino acid sequence selected from the group consisting of SEQ ID NO: 18 to SEQ ID NO:30, or at least the variable domain thereof or an amino acid sequence having a sequence identity of at least 90% thereto and represents a variable region having binding activity to Axl which is equivalent to that of one having a sequence identity of 100%,
or a variable domain of a light chain amino acid sequence selected from the group consisting of SEQ ID NO: 73 to SEQ ID NO:79 or an amino acid sequence having a sequence identity of at least 90% thereto and represents a variable region having binding activity to Axl which is equivalent to that of one having a sequence identity of 100%,
or a fragment thereof recognizing the same epitope on the extracellular domain of AXL. A description of the sequences according to the invention is presented below under "Description of the Sequences" and in the Sequence Listing.

In particular preferred embodiment the humanized h#11B7 antibodies are selected from the group consisting of h#11B7-T1, h#11B7-T2, h#11B7-T3, h#11B7-T4, h#11B7-T5, h#11B7-T6, h#11B7-T7, h#11B7-T8, h#11B7-T9. h#11B7-T10, h#11B7-T11, h#11B7-T12, h#11B7-T13, h#11B7-T14, h#11B7-T15, h#11B7-T16, h#11B7-T17, h#11B7-T18, h#11B7-T19, h#11B7-T20, h#11B7-T21, h#11B7-T22, h#11B7-T23, h#11B7-T24, h#11B7-T25, h#11B7-T26, or h#11B7-T27 antibodies. Table 1 (Example 10) summarizes by which combinations of specific humanized heavy and light chain amino acid sequences, respectively by which combination of corresponding expression vectors, the above humanized h#11B7 antibodies are characterized in the invention.

A humanized antibody derived from rat anti-human Axl monoclonal antibody #11B7 is not limited to the specific antibodies exemplified in the previous paragraphs, as far as said humanized antibody retains all of six CDRs and binding activity to the Axl antigen. Said humanized antibody retains CDRH1 (SNYWG; SEQ ID NO: 124), CDRH2 (YITYSGSTSYNPSLKS; SEQ ID NO: 125) and CDRH3 (TTFYY; SEQ ID NO: 126) in its heavy chain, and, CDRL4 (RASQDIGNYLR; SEQ ID NO: 121), CDRL5 (GATNLAA; SEQ ID NO: 122) and CDRL6 (LQSKESPWT; SEQ ID NO:123) in its light chain, respectively.

According to a further embodiment the humanized antibodies of the invention are derived from 11D5 and comprise a heavy chain amino acid sequence selected from the group consisting of SEQ ID NO:67 to SEQ ID NO:72, or at least the variable domain thereof or an amino acid sequence having a sequence identity of at least 90% thereto and represents a variable region having binding activity to Axl which is equivalent to that of one having a sequence identity of 100%,
or a variable domain of a heavy chain amino acid sequence selected from the group consisting of SEQ ID NO:114 to SEQ ID NO:120 or an amino acid sequence having a sequence identity of at least 90% thereto and represents a variable region having binding activity to Axl which is equivalent to that of one having a sequence identity of 100%
and/or a light chain amino acid sequence selected from the group consisting of SEQ ID NO:55 to SEQ ID NO:60, or at least the variable domain thereof or an amino acid sequence having a sequence identity of at least 90% thereto and represents a variable region having binding activity to Axl which is equivalent to that of one having a sequence identity of 100%,
or a variable domain of a light chain amino acid sequence selected from the group consisting of SEQ ID NO:83 to SEQ ID NO:113
or an amino acid sequence having a sequence identity of at least 90% thereto and represents a variable region having binding activity to Axl which is equivalent to that of one having a sequence identity of 100%, or a fragment thereof recognizing the same epitope on the extracellular domain of AXL.

In particular preferred embodiment the humanized h#11D5 antibodies are selected from the group consisting of h#11D5-T1, h#11D5-T2, h#11D5-T3, h#11D5-T4, h#11D5-T5 or h#11D5-T6 antibodies. Examples 11, 12 and 14 show by which combinations of specific humanized heavy and light chain amino acid sequences, respectively by which combination of corresponding expression vectors, the above humanized h#llD5 antibodies are characterized.

A humanized antibody derived from rat anti-human Axl monoclonal antibody #11 D5 is not limited to the specific antibodies exemplified in the previous paragraphs, as far as said humanized antibody retains all of six CDRs and binding activity to the Axl antigen. Said humanized antibody retains CDRH1 (SNYWG; SEQ ID NO: 130), CDRH2 (HITNSGNTTYNPSLKS; SEQ ID NO: 131) and CDRH3 (GAFDY; SEQ ID NO: 132) in its heavy chain, and, CDRL4 (RASQDIGNYLS; SEQ ID NO: 127), CDRL5 (GAIKLAV; SEQ ID NO: 128) and CDRL6 (LQYIQFPLT; SEQ ID NO: 129) in its light chain, respectively.

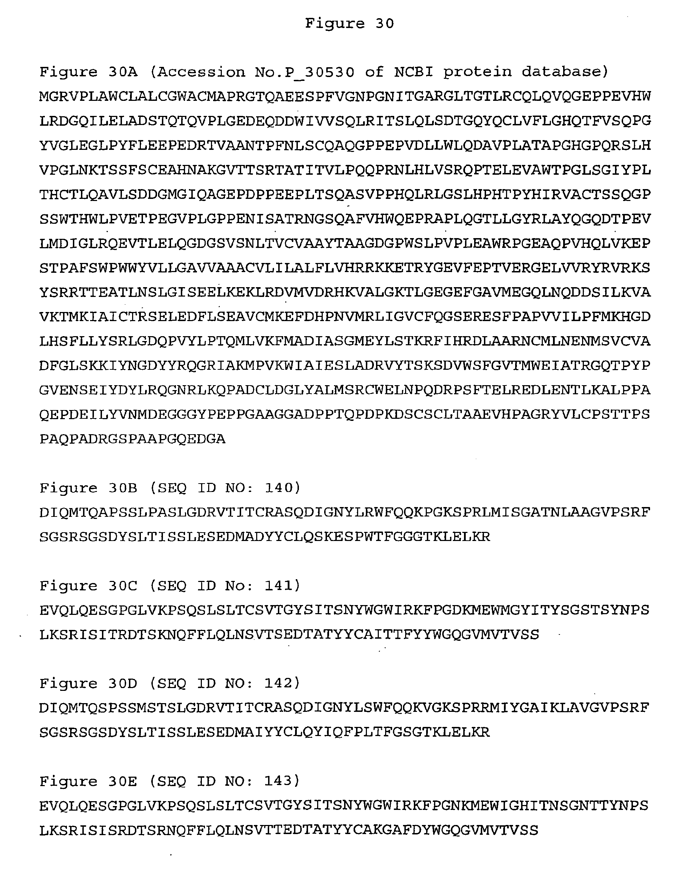

Another preferred embodiment of the invention is an antibody which (specifically) binds or recognizes one of IG domains closer to the carboxy terminus (the domain comprising amino acid residues of amino acid Nos. 129-220 in NCBI protein database ACCESSION No. P_30530: SEQ ID NO: 139; Figure 30A). Such a preferred antibody is not limited to a humanized antibody, but can be a human antibody or a functional fragment of one of them. More preferably, such a preferred antibody inhibits at least one of the biological activities that Axl has.

A (full) antibody of the present invention can be produced, for example, by carrying out the following steps: constructing a heavy chain expression vector and light chain expression vector, wherein each of said vector has an insert comprising a variable region and constant region; introducing said vectors into a host cell; culturing said cell; recovering antibody polypeptides from the culture supernatant (the conditioned medium), as exemplified in Examples 9 to 12.

As used herein, "sequence identity" between two polypeptide sequences, indicates the percentage of amino acids that are identical between the sequences. Methods how to determine the "sequence identity" between two given polypeptide sequences are known to the person skilled in the art. Preferred polypeptide sequences of the invention have a sequence identity of at least 90%, more preferably of al least 95% and even more preferably of at least 98%. The sequence identity can be determined, for example, by BLAST, an algorithm by Karlin and Altschul (Proc. Natl. Acad. Sci. USA, 90, pp5873(1993)) or FASTA (Methods in Enzymol., 183, pp63(1990)). A program designated BLASTN or BLASTX is available (J. Mol. Biol., 215, pp403(1990)).

The antibody of the invention may be of the IgA-, IgD-, IgE, IgG-or IgM-type, preferably of the IgG-or IgM-type including, but not limited to, the IgG1-, IgG2-, IgG3-, IgG4-, IgM1-and IgM2-type.

Inventive antibodies may be preferably designed by homology modeling using any suitable procedure known to the person skilled in the art Moreover, humanized forms of given antibodies, such as 11 B7 or 11D5, may be generated according to the methods known in the art such as chimerization or CDR grafting. Alternative methods for the production of humanized antibodies are well known in the art and are described in, e.g., EP-A1 0 239 400 and W09007861. Generally, a humanized antibody has one or more amino acid residues introduced into it from a source which is non-human. These non-human amino acid residues are often referred to as "import" residues, which are typically taken from an "import" variable domain. Humanization can be for example performed following the method of Winter and co-workers (Jones et al., Nature, 321:522-525 (1986); Riechmann et al., Nature, 332:323-327 (1988); Verhoeyen et al., Science, 239:1534-1536 (1988)), by substituting CDRs or CDR sequences of non human origin for the corresponding sequences of a human antibody. Accordingly, such "humanized" antibodies are chimeric antibodies (U.S. Pat. No. 4,816,567) wherein substantially less than an intact human variable domain has been substituted by the corresponding sequence from a non-human species. In practice, humanized antibodies are typically human antibodies in which some CDR residues and possibly some FR residues are substituted by residues from analogous sites in non human antibodies.

A human antibody can be derived from a phage-display library of human antibodies (Wormstone, I.M.et.al, Investigative Ophthalmology & Visual Science. (2002) 43 (7), p.2301-2308; Carmen, S.et.al., Briefings in Functional Genomics and Proteomics (2002), 1 (2), p.189-203; Siriwardena, D.et.al., Opthalmology (2002) 109 (3), p.427-431), which is well-known to the skilled person.

For example, a phage display method, which comprises having a variable region of human antibody expressing on the surface of phage as a single chain antibody, scFv, and selecting a phage which binds to an antigen, can be used (Nature Biotechnology (2005), 23, (9), p.1105-1116).

By analyzing the gene of the selected phage, the DNA sequence encoding the variable region of a human antibody binding to the antigen can be determined.

After the DNA sequence of the scFv is determined, a human antibody can be produced by constructing an expression vector comprising said sequence and introducing said vector into a suitable host cell to express said antibody (WO92001047, WO92020791, WO93006213, WO93011236, WO93019172, WO95001438, WO95015388 and Annu.Rev.Immunol (1994) 12, p.433-455 and Nature Biotechnology (2005) 23 (9), p.1105-1116).

Any human antibody, regardless of a method for the production thereof, can be an embodiment of the present invention, as far as said human antibody has binding activity to human Axl antigen, and all of the following six CDRs: CDRH1 (SNYWG; SEQ ID NO: 130), CDRH2 (HITNSGNTTYNPSLKS; SEQ ID NO: 131) and CDRH3 (GAFDY; SEQ ID NO: 132) in its heavy chain, and, CDRL4 (RASQDIGNYLS; SEQ ID NO: 127), CDRL5 (GAIKLAV; SEQ ID NO: 128) and CDRL6 (LQYIQFPLT; SEQ ID NO: 129) in its light chain, respectively, alternatively, as far as said human antibody has binding activity to human Axl antigen, and all of the following six CRDs: CDRH1 (SNYWG; SEQ ID NO: 124), CDRH2 (YITYSGSTSYNPSLKS; SEQ ID NO: 125) and CDRH3 (TTFYY; SEQ ID NO: 126) in its heavy chain, and, CDRL4 (RASQDIGNYLR; SEQ ID NO: 121), CDRL5 (GATNLAA; SEQ ID NO: 122) and CDRL6 (LQSKESPWT; SEQ ID NO:123) in its light chain, respectively.

For therapeutic purposes, the antibody may be conjugated with a therapeutic effector group, e.g. a radioactive group or a cytotoxic group.

For diagnostic purposes, the antibody may be labeled. Suitable labels include radioactive labels, fluorescent labels, or enzyme labels.

As discussed above, the antibody of the invention may exist in a variety of forms besides complete antibodies; including, for example, Fv, Fab, Fab' and F(ab')₂, diabody, minibody, divalent or multivalent antibody comprising a fragment of more than one antibody, as well as in single chains (scFV); see e.g. W08809344.

An antibody of the present invention can be a bi-specific antibody or mutli-specific antibody which is specific for more than one antigen or epitope.

A scFv can be obtained by fusing a variable region of an immunoglobulin heavy chain to the variable region of an immunoglobulin light chain through a polypeptide linker (Pluckthun, The Pharmacology of Monoclonal Antibodies, 113, edited by Rosenburg and Moore , Springer Verlag, New York, p.269-315 (1994) and Nature Biotechnology (2005), 23, p.1126-1136). A BiscFv can be obtained by fusing two different scFV through a polypeptide linker.

Methods for generating scFv are well-know to the person skilled in the art (US4,946,778, US5,260,203, US5,091,513, US5,455,030 etc). Preferably, a linker between two variable regions does not produce a conjugate (Huston, J.S.et al., Proc.Natl.Acad.Sci.U.S.A. (1988) 85, p.5879-5883). A scFV immunoglobulin heavy chain variable region and a scFV immunoglobulin light chain variable region can be derived from an identical antibody or two antibodies, one of which is different from the other. A polypeptide linker can be a single chain peptide consisting of, for example, 12 to 19 amino acid residues.

A DNA encoding a scFV can be generated by PCR which comprises amplifying a DNA fragment using a DNA encoding a full length or portion of an immunoglobulin heavy chain variable region or light chain variable region and two oligonucleotides corresponding to each of both terminuses as a template as a pair of primers, respectively, and then amplifying using a pair of primers which are designed so that a heavy chain variable region and light chain variable region are connected to one terminus and the other terminus of a polypeptide linker, respectively.

A functional fragment of an antibody including scFv can be produced by introducing a DNA encoding said scFv into a host cell and culturing said cell, as described elsewhere in the description.

One of the embodiments of the invention is a multimeric antibody that has higher binding affinity to an antigen than a monomeric antibody. The Unit of said multimeric antibody can be the same, or different from each other when a unit binds to an epitope of an antigen and another unit binds to a different epitope of the same antigen. Binding of IgG CH3 domain and two scFv, binding with streptoavidin and introduction of a helix-turn-helix motif can be applied for producing multimeric antibody.

If desired, the antibodies of the invention may be mutated in the variable domains of the heavy and/or light chains to alter a binding property of the antibody. For example, a mutation may be made in one or more of the CDR regions to increase or decrease the Kd of the antibody for AXL, or to alter the binding specificity of the antibody. Techniques in site directed mutagenesis are well-known in the art. See, e.g., Sambrook et al. and Ausubel et al., supra. Furthermore, mutations may be made at an amino acid residue that is known to be changed compared to germline in a variable region of an AXL antibody.

In another aspect, beside mutations with respect to humanization, further mutations may be introduced into one or more of the framework regions. A mutation may be made in a framework region or constant domain to increase the half-life of the AXL antibody. See, e.g., WO0009560. A mutation in a framework region or constant domain may also be made to alter the immunogenicity of the antibody, to provide a site for covalent or non-covalent binding to another molecule, or to alter such properties as complement fixation. Mutations may be made in each of the framework regions, the constant domain and the variable regions in a single mutated antibody. Alternatively, mutations may be made in only one of the framework regions, the variable regions or the constant domain in a single mutated antibody.

One of the embodiments of the invention is a polyclonal antibody comprising more than one antibody of the present invention.

One of the embodiments of the invention is a chemical modification of an antibody or its functional fragment of the present invention. Molecules such as polymer (polyethylene glycol or the like) can be used to generate a modified antibody or modified functional fragment.

In a further aspect, the humanized antibody according to the invention may have a constant domain with effector functions, whereby AXL expressing cells which have bound the antibody, antibody fragment or derivative thereof on the cell surface may be attacked by immune system functions. For example, the antibody may be capable of fixing complement and participating in complement-dependent cytotoxicity (CDC). Moreover, the antibody may be capable of binding to Fc receptors on effector cells, such as monocytes and natural killer (NK) cells, and participate in antibody-dependent cellular cytotoxicity (ADCC).

In yet a further aspect the antibodies described herein are applicable for therapeutic treatment, preferably for treatment of hyperproliferative diseases, cardiovascular diseases, in particular artherosclerosis and thrombosis, diabetes related diseases, in particular glomerular hypertrophy or diabetic nephropathy, and particularly of disorders associated with, accompanied by or caused by AXL expression, overexpression or hyperactivity. The hyperproliferative diseases are preferably selected from disorders associated with, accompanied by or caused by AXL expression, overexpression or hyperactivity, such as cancer, e.g. breast cancer, colon cancer, lung cancer, kidney cancer, follicular lymphoma, myeloid leukemia, skin cancer/melanoma, glioblastoma, ovarian cancer, prostate cancer, pancreatic cancer, Barrett's esophagus and esophageal cancer, stomach cancer, bladder cancer, cervical cancer, liver cancer, thyroid cancer, and head and neck cancer, or hyperplastic and neoplastic diseases or other AXL expressing or overexpressing hyperproliferative diseases.

The applicability of the antibodies of the present invention for the treatment of any Axl-related diseases such as hyperproliferative diseases can be demonstrated, indicated or suggested by *in vitro, in vivo* or *ex vivo* experiments, including those on ligand-induced autophosphorylation of Axl, effect on Axl-mediated downstream signal trancduction such as ligand-induced Akt and p42/44 MAP-kinase phosphorylation, cancer cell migration or proliferation, ligand-induced migration or proliferation of a cell expressing Axl, angiogenesis of tissues or cells, and growth or metastasis of human cancer or cancer cell transplanted onto non-human animals such as xenograft mice, as well as any other pre-clinical/non-clinical experiments and clinical trials.

In another aspect the antibodies of the present invention can be used for the co-administration with an antineoplastic agent for the treatment of one of the above mentioned disorders.

Co-administration as used herein includes the administration of an antibody of the present invention with an antineoplastic agent, preferably an apoptosis inducing antineoplastic agent. The term co-administration further includes the administration of the antibody of the present invention and the antineoplastic agent, preferably an apoptosis inducing antineoplastic agent, in the form of a single composition or in the form of two or more distinct compositions. Co-administration includes the administration of an antibody of the present invention with an antineoplastic agent, preferably an apoptosis inducing antineoplastic agent simultaneously (i.e. at the same time) or sequentially, (i.e. at intervals).

The invention further relates to a nucleic acid molecule encoding the antibody, antibody fragment or derivative thereof of the invention. The nucleic acid molecule of the invention encoding the above-described antibody, antibody fragment or derivative thereof may be, e.g. DNA, cDNA, RNA or synthetically produced DNA or RNA or recombinantly produced chimeric nucleic acid molecule comprising any of those nucleic acid molecules either alone or in combination. The nucleic acid molecule may also be genomic DNA corresponding to the entire gene or a substantial portion thereof or to fragments and derivatives thereof. The nucleotide sequence may correspond to the naturally occurring nucleotide sequence or may contain single or multiple nucleotide substitutions, deletions or additions. In a particular preferred embodiment of the present invention, the nucleic acid molecule is a cDNA molecule.

According to a further aspect, the present invention relates to an isolated nucleic acid molecule selected from the group consisting of:
(a) a nucleic acid sequence encoding an monoclonal antibody, antibody fragment or a derivative thereof of any of claims 1-12,
(b) a nucleic acid sequence as shown in in one of the SEQ ID NOs. selected from the group consisting of SEQ ID NO:5 to SEQ ID NO:17, SEQ ID NO:31 to SEQ ID NO:39, SEQ ID NO:49 to SEQ ID NO:54 and SEQ ID NO:60 to SEQ ID NO:66
(c) a nucleic acid sequence encoding a polypeptide selected from the group consisting of SEQ ID NO:18 to SEQ ID NO:30, SEQ ID NO:40 to SEQ ID NO:48, SEQ ID NO:55 to SEQ ID NO:60, SEQ ID NO:67 to SEQ ID NO:72, SEQ ID NO:73 to SEQ ID NO:120 and SEQ ID NO:121 to 132,
(d) a nucleic acid complementary to any of the sequences in (a) to (c), or
(e) a nucleic acid sequence capable of hybridizing to (a), (b), (c) or (d) under stringent conditions and encoding a polypeptide, wherein an antibody or functional fragment thereof comprising said polypeptide binds to the extracellular domain of AXL.

The term "hybridizing under stringent conditions" means that two nucleic acid fragments hybridize with one another under standardized hybridization conditions as described for example in Sambrook et al., "Expression of cloned genes in E. coli" in Molecular Cloning: A laboratory manual (1989), Cold Spring Harbor Laboratory Press, New York, USA. Such conditions are for example hybridization in 6.0xSSC at about 45° C. followed by a washing step with 2.0xSSC at 50° C, preferably 2.0xSSC at 65°C, or 0.2xSSC at 50°C, preferably 0.2xSSC at 65°C.

The invention also relates to a vector comprising a nucleic acid molecule of the invention. Said vector may be, for example, a phage, plasmid, viral or retroviral vector. Retroviral vectors may be replication competent or replication defective. In the latter case, viral propagation generally will occur only in complementing host cells.

The nucleic acid molecules of the invention may be joined to a vector containing selectable markers for propagation in a host. Generally, a plasmid vector is introduced in a precipitate such as a calcium phosphate precipitate or rubidium chloride precipitate, or in a complex with a charged lipid or in carbon-based clusters, such as fullerens. Should the vector be a virus, it may be packaged in vitro using an appropriate packaging cell line prior to application to host cells.

Preferably, the vector of the invention is an expression vector wherein the nucleic acid molecule is operatively linked to one or more control sequences allowing the transcription and optionally expression in prokaryotic and/or eukaryotic host cells. Expression of said nucleic acid molecule comprises transcription of the nucleic acid molecule, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the lac, trp or tac promoter in E. coli, and examples for regulatory elements permitting expression in eukaryotic host cells are the AOXI or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (Invitrogen) or pSPORTI (GIBCO BRL). Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (2001, Third Edition) N.Y. and Ausubel, Current Protocols in Molecular Biology, Green Publishing Associates and Wiley Interscience, N.Y. (1994). Alternatively, the nucleic acid molecules of the invention can be reconstituted into liposomes for delivery to target cells.

The invention further relates to a host comprising the vector of the invention. Said host may be a prokaryotic or eukaryotic cell or a non-human transgenic animal. The polynucleotide or vector of the invention which is present in the host may either be integrated into the genome of the host or it may be maintained extrachromosomally. In this respect, it is also to be understood that the nucleic acid molecule of the invention can be used for "gene targeting" and/or "gene replacement", for restoring a mutant gene or for creating a mutant gene via homologous recombination; see for example Mouellic, Proc. Nat!. Acad. Sci. USA, 87 (1990), 4712-4716; Joyner, Gene Targeting, A Practical Approach, Oxford University Press.

The host can be any prokaryotic or eukaryotic cell, such as a bacterial, insect, fungal, plant, animal, mammalian or, preferably, human cell. Preferred fungal cells are, for example, those of the genus Saccharomyces, in particular those of the species S. cerevisiae. The term "prokaryotic" is meant to include all bacteria which can be transformed or transfected with a polynucleotide for the expression of a variant polypeptide of the invention. Prokaryotic hosts may include gram negative as well as gram positive bacteria such as, for example, E. coli, S. typhimurium, Serratia marcescens and Bacillus subtilis. A polynucleotide coding for a mutant form of variant polypeptides of the invention can be used to transform or transfect the host using any of the techniques commonly known to those of ordinary skill in the art. Methods for preparing fused, operably linked genes and expressing them in bacteria or animal cells are well-known in the art (Sambrook, Molecular Cloning A Laboratory Manual, Cold Spring Harbor Laboratory (2001, Third Edition). The genetic constructs and methods described therein can be utilized for expression of variant antibodies, antibody fragments or derivatives thereof of the invention in, e.g., prokaryotic hosts. In general, expression vectors containing promoter sequences which facilitate the efficient transcription of the inserted nucleic acid molecule are used in connection with the host. The expression vector typically contains an origin of replication, a promoter, and a terminator, as well as specific genes which are capable of providing phenotypic selection of the transformed cells. The transformed prokaryotic hosts can be grown in fermentors and cultured according to techniques known in the art to achieve optimal cell growth. The antibodies, antibody fragments or derivatives thereof of the invention can then be isolated from the growth medium, cellular lysates, or cellular membrane fractions. The isolation and purification of the microbially or otherwise expressed antibodies, antibody fragments or derivatives thereof of the invention may be by any conventional means such as, for example, preparative chromatographic separations and immunological separations such as those involving the use of monoclonal or polyclonal antibodies.

In a preferred embodiment of the invention, the host is a bacterium, fungal, plant, amphibian or animal cell. Preferred animal cells include but are not limited to Chinese hamster ovary (CHO) cells (CHO cell, ATCC CCL-61) or a dihydrofolate reductaase-deficient strain thereof (Urlaub, G. and Chasin, L.A. Proc.Natl.Acad.Sci.U.S.A. (1980) 77, p.4126-4220), baby hamster kidney (BHK) cells, monkey kidney cells (COS) (Gluzman, Y. Cell (1981) 23, p.175-182 and ATCC CRL-1650), 3T3 cells or NIH3T3 cells derived from a mouse fibroblast (ATCC No.CRL-1658), NS0 cells and a number of other cell lines including human cells, for example Per.C6. In another preferred embodiment, said animal cell is an insect cell. Preferred insect cells include but are not limited to cells of the SF9 cell lines.

In a more preferred embodiment of the invention, said host is a human cell or human cell line. Said human cells include, but are not limited to Human embryonic kidney cells (HEK293, 293T, 293 freestyle). Furthermore, said human cell lines include, but are not limited to HeLa cells, human hepatocellular carcinoma cells (e. g., Hep G2), A549 cells.

The invention also provides transgenic non-human animals comprising one or more nucleic acid molecules of the invention that may be used to produce antibodies of the invention. Antibodies can be produced in and recovered from tissue or body fluids, such as milk, blood or urine, of goats, cows, horses, pigs, rats, mice, rabbits, hamsters or other mammals. See, e. g., U.S. Patent Nos. 5,827,690; 5,756,687; 5,750,172; and 5,741,957. As described above, non-human transgenic animals that comprise human immunoglobulin loci can be produced by immunizing with AXL or a portion thereof.

The invention additionally relates to a method for the preparation of an antibody, comprising culturing the host of the invention under conditions that allow synthesis of said antibody and recovering said antibody from said culture.

The transformed hosts can be grown in fermentors and cultured according to techniques known in the art to achieve optimal cell growth. Once expressed, the whole antibodies, their dimers, individual light and heavy chains, or other immunoglobulin forms of the present invention, can be purified according to standard procedures of the art, including ammonium sulfate precipitation, affinity columns, column chromatography, gel electrophoresis and the like; see, Scopes, "Protein Purification", Springer-Verlag, N.Y. (1982). The antibody or its corresponding immunoglobulin chain(s) of the invention can then be isolated from the growth medium, cellular lysates, or cellular membrane fractions. The isolation and purification of the, e.g., microbially expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention.

It will be apparent to those skilled in the art that the antibodies of the invention can be further coupled to other moieties for, e.g., drug targeting and imaging applications. Such coupling may be conducted chemically after expression of the antibody or antigen to site of attachment or the coupling product may be engineered into the antibody or antigen of the invention at the DNA level. The DNAs are then expressed in a suitable host system, and. the expressed proteins are collected and renatured, if necessary.

In a preferred embodiment of the present invention, the antibody is coupled to an effector, such as a radioisotope or a toxic chemotherapeutic agent. Preferably, these antibody conjugates are useful in targeting cells, e.g. cancer cells, expressing AXL, for elimination. The linking of antibodies/antibody fragments of the invention to radioisotopes e.g. provides advantages to tumor treatments. Unlike chemotherapy and other forms of cancer treatment, radioimmunotherapy or the administration of a radioisotope-antibody combination directly targets the cancer cells with minimal damage to surrounding normal, healthy tissue. Preferred radioisotopes include e.g. ³H, ¹⁴C, ¹⁵N, ³⁵S, ⁹⁰Y, ⁹⁹Tc, ¹¹¹In, ¹²⁵I, ¹³¹I.

Furthermore, the antibodies of the invention can be used to treat cancer when being conjugated with toxic chemotherapeutic drugs such as geldanamycin (Mandler et al., J. Natl. Cancer Inst., 92(19), 1549-51 (2000» and maytansin, for example, the maytansinoid drug, DM1 (Liu et al., Proc. Natl. Acad. Sci. U.S.A. 93:8618-8623 (1996) and auristatin-E or monomethylauristatin-E (Doronina et al., Nat. Biotechnol. 21:778-784 (2003) or calicheamicit);. Different linkers that release the drugs under acidic or reducing conditions or upon exposure to specific proteases are employed with this technology. The antibodies of the invention may be conjugated as described in the art.

One of the embodiments of the invention is a chemical modification of an antibody or its functional fragment of the present invention. Molecules such as polymer (polyethylene glycol or the like) can be used to generate a modified antibody or modified functional fragment.

The invention further relates to a pharmaceutical composition comprising the antibody, the nucleic acid molecule, the vector, the host of the invention or an antibody obtained by the method of the invention.

The term "composition" as employed herein comprises at least one compound of the invention. Preferably, such a composition is a pharmaceutical or a diagnostic composition.

It is preferred that said pharmaceutical composition comprises a pharmaceutically acceptable carrier and/or diluent. The herein disclosed pharmaceutical composition may be partially useful for the treatment of disorders associated with, accompanied by or caused by AXL expression, overexpression or hyperactivity, e.g. hyperproliferative diseases, cardiovascular diseases, in particular artherosclerosis and thrombosis, diabetes related diseases, in particular glomerular hypertrophy or diabetic nephropathy. Said disorders comprise, but are not limited to cancer, e.g. breast cancer, colon cancer, lung cancer, kidney cancer, follicular lymphoma, myeloid leukemia, skin cancer/melanoma, glioblastoma, ovarian cancer, prostate cancer, pancreatic cancer, Barrett's esophagus and esophageal cancer, stomach cancer, bladder cancer, cervical cancer, liver cancer, thyroid cancer, and head and neck cancer, or other hyperplastic or neoplastic diseases or other AXL expressing or overexpressing diseases.

The term "hyperactivity" herein refers to uncontrolled AXL signaling which may be caused by a lack and/or dysfunction of negative regulation. By way of example negative regulation comprises protein dephosphorylation, degradation and/or endocytosis. Moreover uncontrolled AXL signaling may be the result of genetic alterations, either somatic or germline, which result in changes of the AXL amino acid sequence.

Examples of suitable pharmaceutical carriers, excipients and/or diluents are well known in the art and include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical, intradermal, intranasal or intrabronchial administration. The compositions of the invention may also be administered directly to the target site, e.g., by biolistic delivery to an external or internal target site, like the brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Proteinaceous pharmaceutically active matter may be present in amounts between 1 µg and 100 mg/kg body weight per dose; however, doses below or above this exemplary range are envisioned, especially considering the aforementioned factors. If the regimen is a continuous infusion, it should also be in the range of 1 pg to 100 mg per kilogram of body weight per minute.

Progress can be monitored by periodic assessment. The compositions of the invention may be administered locally or systemically. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Furthermore, the pharmaceutical composition of the invention may comprise further agents depending on the intended use of the pharmaceutical composition.

It is particularly preferred that the pharmaceutical composition comprises at least one further active agent like, e.g. an additional antineoplastic agent, small molecule inhibitor, anti-tumor agent, chemotherapeutic agent or a combination of those agents.

The invention also relates to a pharmaceutical composition comprising an humanized anti-AXL-antibody of the invention in combination with at least one further antineoplastic agent. Said combination is effective, for example, in inhibiting abnormal cell growth.

Many antineoplastic agents are presently known in the art. In general the term includes all agents that are capable of prevention, alleviation and/or treatment of hyperproliferative disorders. In one embodiment, the antineoplastic agent is selected from the group of therapeutic proteins including but not limited to antibodies or immunomodulatory proteins. In another embodiment the antineoplastic agent is selected from the group of small molecule inhibitors or chemotherapeutic agents consisting of mitotic inhibitors, kinase inhibitors, alkylating agents, anti-metabolites, intercalating antibiotics, growth factor inhibitors, cell cycle inhibitors, enzymes, topoisomerase inhibitors, histone deacetylase inhibitors, anti-survival agents, biological response modifiers, anti-hormones, e. g. anti-androgens, and antiangiogenesis agents.

Specific examples of antineoplastic agents which can be used in combination with the antibodies provided herein include, for example, gefitinib, lapatinib, sunitinib, pemetrexed, bevacisumab, cetuximab, imatinib, trastuzumab, alemtuzumab, rituximab, erlotinib, bortezomib and the like. Other specific antineoplastic agents to be used in the compositions as described and claimed herein include for example, chemotherapeutic agents such as capecitabine, daunorubicin, daunomycin, dactinomycin, doxorubicin, epirubicin, idarubicin, esorubicin, bleomycin, mafosfamide, ifosfamide, cytosine arabinoside, bis-chloroethylnitrosurea, busulfan, mitomycin C, actinomycin D, mithramycin, prednisone, hydroxyprogesterone, testosterone, tamoxifen, dacarbazine, procarbazine, hexamethylmelamine, pentamethylmelamine, mitoxantrone, amsacrine, chlorambucil, methylcyclohexylnitrosurea, nitrogen mustards, melphalan, cyclophosphamide, 6-mercaptopurine, 6-thioguanine, cytarabine (CA), 5-azacytidine, hydroxyurea, deoxycoformycin, 4-hydroxyperoxycyclophosphoramide, 5-fluorouracil (5-FU), 5-fluorodeoxyuridine (5-FUdR), methotrexate (MTX), colchicine, taxol, vincristine, vinblastine, etoposide, trimetrexate, teniposide, cisplatin and diethylstilbestrol (DES). See, generally, The Merck Manual of Diagnosis and Therapy, 15th Ed. 1987, pp. 1206-1228, Berkow et al., eds., Rahway, N.J. In particular preferred are such antineoplastic agents that induce apoptosis.

The invention relates also to a polyclonal antibody comprising more than one antibody of the present invention.

When used with the described AXL antibodies, such antineoplastic agents may be used individually (e.g., 5-FU and an antibody), sequentially (e.g., 5-FU and an antibody for a period of time followed by MTX and an antibody), or in combination with one or more other such antineoplastic agents (e.g., 5-FU, MTX and an antibody, or 5-FU, radiotherapy and an antibody).

The term "antineoplastic agent" may also include therapeutic procedures, as for example irradiation or radiotherapy.

The pharmaceutical composition of the invention are preferably used in human medicine but may be used also for veterinary purposes. Additionally, the invention relates to the use of the antibody of the invention, the nucleic acid molecule, the vector, the host of the invention or an antibody obtained by the method of the invention for the preparation of a pharmaceutical composition for diagnosis, prevention or treatment of hyperproliferative diseases, cardiovascular diseases, in particular artherosclerosis and thrombosis, diabetes related diseases, in particular glomerular hypertrophy or diabetic nephropathy, and particularly of disorders associated with, accompanied by or caused by AXL expression, overexpression or hyperactivity.

A hyperproliferative disease as mentioned above includes any neoplasia, i.e. any abnormal and/or uncontrolled new growth of tissue. The term "uncontrolled new growth of tissue" as used herein may depend upon a dysfunction and/or loss of growth regulation. A hyperproliferative disease includes tumor diseases and/or cancer, such as metastatic or invasive cancers.

In a preferred embodiment of the use of the invention, said hyperproliferative disease is in particular breast cancer, colon cancer, lung cancer, kidney cancer, follicular lymphoma, myeloid leukemia, skin cancer/melanoma, glioblastoma, ovarian cancer, prostate cancer, pancreatic cancer, Barrett's esophagus and esophageal cancer, stomach cancer, bladder cancer, cervical cancer, liver cancer, thyroid cancer, and head and neck cancer, or hyperplastic or neoplastic diseases or other AXL expressing or overexpressing hyperproliferative diseases.

In yet another embodiment the present invention refers to the use of an humanized anti-AXL-antibody of the present invention for the manufacture of a medicament for the co-administration with an antineoplastic agent for the treatment of one of the above mentioned disorders.

According to a further preferred embodiment the present invention is directed to the use of an humanized anti-AXL antibody of the invention for the manufacture of a pharmaceutical composition for the treatment of drug resistant cancer.

Further the present invention relates to a diagnostic composition comprising the antibody of the invention, the nucleic acid molecule, the vector, the host of the invention or an antibody obtained by the method of the invention and optionally a pharmaceutically acceptable carrier.

The diagnostic composition of the invention is useful in the detection of an undesired expression, overexpression or hyperactivity of the mammalian AXL in different cells, tissues or another suitable sample, comprising contacting a sample with an antibody of the invention, and detecting the presence of AXL in the sample. Accordingly, the diagnostic composition of the invention may be used for assessing the onset or the disease status of a hyperproliferative disease.

Furthermore, malignant cells, such as cancer cells expressing AXL, can be targeted with the antibody of the invention. The cells which have bound the antibody of the invention might thus be attacked by immune system functions such as the complement system or by cell-mediated cytotoxicity, thereby reducing the number of or eradicating cancer cells. These considerations equally apply to the treatment of metastases and re-current tumors.

In another aspect of the present invention, the antibody of the invention is coupled to a labelling group. As already outlined above, such antibodies are particularly suitable for diagnostic applications. As used herein, the term "labelling group" refers to a detectable marker, e.g. a radiolabelled amino acid or biotinyl moieties that can be detected by marked avidin. Various methods for labelling polypeptides and glycoproteins, such as antibodies, are known in the art and may be used in performing the present invention. Examples of suitable labelling groups include, but are not limited to, the following: radioisotopes or radionuclides (e.g. ³H, ¹⁴C, ¹⁵N, ³⁵S, ⁹⁰Y, ⁹⁹Tc, ¹¹¹In, ¹²⁵I, ¹³¹I), fluorescent groups (e.g. FITC, rhodamine, lanthanide phosphors), enzymatic groups (e.g. horseradish peroxidase, β-galactosidase, luciferase, alkaline phosphatase), chemiluminescent groups, biotinyl groups, or predetermined polypeptide epitopes recognized by a secondary reporter (e.g. leucine zipper pair sequences, binding sites for secondary antibodies, metal binding domains, epitope tags).

In certain aspects, it may be desirable, that the labelling groups are attached by spacer arms of various lengths to reduce potential steric hindrance.

In another embodiment the present invention relates to a method of assessing for the presence of AXL expressing cells comprising contacting the antibody of the invention with cells or a tissue suspected of carrying AXL on their/its surface. Suitable methods for detection of AXL expression in a sample may be an Enzyme-Linked Immunosorbent Assay (ELISA) or Immunohistochemistry (IHC).

An ELISA assay may be carried out in a microtiter plate format, wherein e.g. wells of a microtiter plate, are adsorbed with an AXL antibody. The wells are rinsed and treated with a blocking agent such as milk protein or albumin to prevent nonspecific adsorption of the analyte. Subsequently the wells are treated with a test sample. After rinsing away the test sample or standard, the wells are treated with a second AXL antibody that is labelled, e.g. by conjugation with biotin. After washing away excess secondary antibody, the label is detected, e.g. with avidin-conjugated horseradish peroxidase (HRP) and a suitable chromogenic substrate. The concentration of the AXL antigen in the test samples is determined by comparison with a standard curve developed from standard samples.

For IHC, paraffin-embedded tissues may be used, wherein the tissues are, e.g. first deparaffinized in xylene and then dehydrated, e.g. with ethanol and rinsed in distilled water. Antigenic epitopes masked by formalin-fixation and paraffin-embedding may be exposed by epitope unmasking, enzymatic digestion or saponin. For epitope unmasking paraffin sections may be heated in a steamer, water bath or microwave oven for 20-40 min in an epitope retrieval solution as for example 2N HCl solution (pH 1.0). In the case of an enzyme digestion, tissue sections may be incubated at 37°C for 10-30 minutes in different enzyme solutions such as proteinase K, trypsin, pronase, pepsin etc. After rinsing away the epitope retrieval solution or excess enzyme, tissue sections are treated with a blocking buffer to prevent unspecific interactions. The primary AXL antibody is added at appropriate concentrations. Excess primary antibody is rinsed away and sections are incubated in peroxidase blocking solution for 10 min at room temperature. After another washing step, tissue sections are incubated with a secondary labeled antibody, e.g. labeled with a group that might serve as an anchor for an enzyme. Examples therefore are biotin labeled secondary antibodies that are recognized by streptavidin coupled horseradish peroxidase. Detection of the antibody/enzyme complex is achieved by incubating with a suitable chromogenic substrate.

In an additional embodiment the present invention relates to a method of blocking AXL function comprising contacting the antibody of the invention with cells or a tissue suspected of carrying AXL on their/its surface under conditions, wherein the antibody is capable of blocking AXL function. The contacting may be in vitro or in vivo.

The invention also relates to a method of treating a hyperproliferative disease, cardiovascular diseases, in particular artherosclerosis and thrombosis, diabetes related diseases, in particular glomerular hypertrophy or diabetic nephropathy, comprising, administering to a patient in need thereof a suitable dose of the antibody or antibody fragment or derivative thereof of the present invention. The hyperproliferative disease is preferably selected from disorders associated with, accompanied by or caused by AXL expression, overexpression or hyperactivity, such as cancer, e.g. breast cancer, colon cancer, lung cancer, kidney cancer, follicular lymphoma, myeloid leukemia, skin cancer/melanoma, glioblastoma, ovarian cancer, prostate cancer, pancreatic cancer, Barrett's esophagus and esophageal cancer, stomach cancer, bladder cancer, cervical cancer, liver cancer, thyroid cancer, and head and neck cancer, or hyperplastic and neoplastic diseases or other AXL expressing or overexpressing hyperproliferative diseases.

According to another preferred embodiment of the invention the cancer to be treated is a drug resistant cancer, preferably selected from the group of cancers cited above.

The invention further relates to a method of treating a disease wherein the antibody of the invention is administered to a mammal and wherein said disease is correlated directly or indirectly with the abnormal level of expression or activity of AXL.

Finally, the invention relates to a kit comprising an anti-AXL-antibody, preferably the antibody, antibody fragment or derivative thereof of the invention, the nucleic acid molecule encoding said components and/or the vector of the invention.

All embodiments covering the compounds disclosed herein can be used as single compounds or in combination for the preparation of a medicament.

### Brief Description of the Figures

**Figure 1****:** This figure describes the amino acid sequence of the humanized variable regions of the light chain of the rat anti-human AXL monoclonal antibody #11 B7, designated as h#11B7-T1L to h#11B7-T7L.
**Figure 2****:** This figure describes the amino acid sequence of the humanized variable regions of the light chain of the rat anti-human AXL monoclonal antibody #11B7, designated as h#11B7-T8L to h#11B7-T14L.
**Figure 3****:** This figure describes the amino acid sequence of the humanized variable regions of the light chain of the rat anti-human AXL monoclonal antibody #11 B7, designated as h#11B7-T15L to h#11B7-T20L.
**Figure 4****:** This figure describes the amino acid sequence of the humanized variable regions of the heavy chain of the rat anti-human AXL monoclonal antibody #11 B7, designated as h#11B7-T1H to h#11B7-T6H.
**Figure 5****:** This figure describes the amino acid sequence of the humanized variable regions of the heavy chain of the rat anti-human AXL monoclonal antibody #11 B7, designated as h#11B7-T7H to h#11B7-T12H.
**Figure 6****:** This figure describes the amino acid sequence of the humanized variable regions of the light chain of the rat anti-human AXL monoclonal antibody #11D5, designated as h#11D5-T1L to h#11D5-T7L.
**Figure 7****:** This figure describes the amino acid sequence of the humanized variable regions of the light chain of the rat anti-human AXL monoclonal antibody #11D5, designated as h#11D5-T8L to h#11D5-T14L.
**Figure 8****:** This figure describes the amino acid sequence of the humanized variable regions of the light chain of the rat anti-human AXL monoclonal antibody #11D5, designated as h#11D5-T15L to h#11D5-T21L.
**Figure 9****:** This figure describes the amino acid sequence of the humanized variable regions of the light chain of the rat anti-human AXL monoclonal antibody #11D5, designated as h#11D5-T22L to h#11D5-T28L.
**Figure 10****:** This figure describes the amino acid sequence of the humanized variable regions of the light chain of the rat anti-human AXL monoclonal antibody #11D5, designated as h#11D5-T29L to h#11D5-T35L.
**Figure 11****:** This figure describes the amino acid sequence of the humanized variable regions of the light chain of the rat anti-human AXL monoclonal antibody #11D5, designated as h#11D5-T36L and h#11D5-T37L.
**Figure 12****:** This figure describes the amino acid sequence of the humanized variable regions of the heavy chain of the rat anti-human AXL monoclonal antibody #11D5, designated as h#11D5-T1H to h#11D5-T7H.
**Figure 13****:** This figure describes the amino acid sequence of the humanized variable regions of the heavy chain of the rat anti-human AXL monoclonal antibody #11D5, designated as h#11D5-T8H to h#11D5-T13H.
**Figure 14****:** This figure describes nucleotide sequences of Primers EFF1 and EfsmR, Fragment B comprising the secretion signal and constant region of human kappa chain and poly (A) addition signal, a DNA fragment encoding a polypeptide which comprises a human IgG1 signal and constant domain, and Leader sequence, and, amino acid sequence of CDRs of the rat anti-human Axl monoclonal antibodies 11B7 and 11D5, chimeric #11 B7 antibody heavy and light chain (IgG1/kappa), chimeric #11D5 antibody heavy and light chain (IgG2a/Kappa) and Leader sequence

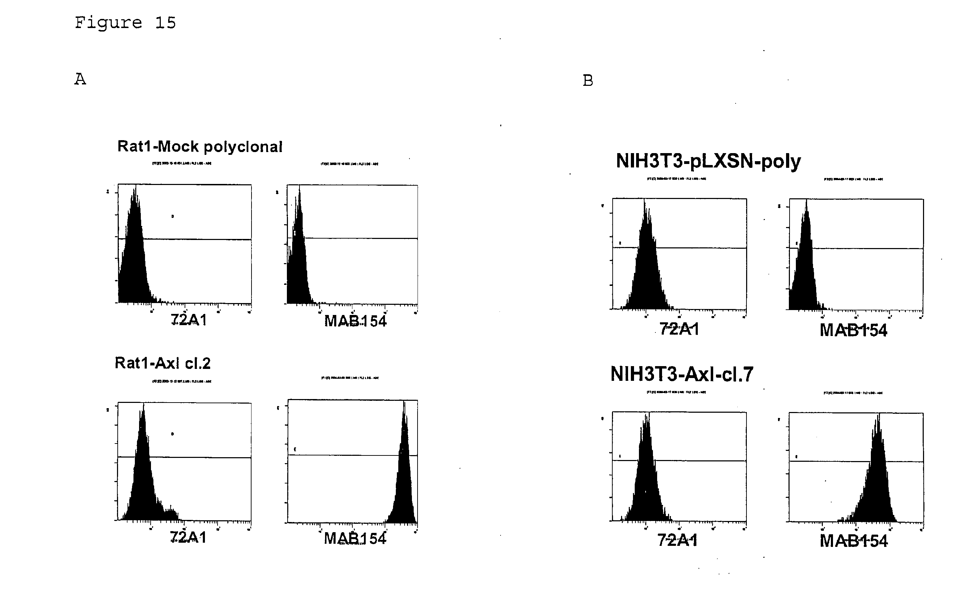

**Figure 15****. A** Flow cytometry analysis of cell surface AXL in Ratl-Mock and Ratl-AXL cl.2 fibroblasts. Polyclonal Ratl-Mock and clonal Ratl-AXL cl.2 cells, generated by infection of Ratl fibroblasts with pLXSN and pLXSN-hAXL ecotrophic virus, respectively, were collected and stained with mouse control antibody 72A1 (left panel) or mouse anti-AXL MAB154 primary antibody (right panel) at 3 µg/ml and PE-conjugated anti-mouse secondary antibody. See text for details. Staining of Ratl-AXL cl.2 cells results in a shift by three orders of magnitude and demonstrates AXL overexpression on the surface of these cells.
   **B.** Flow cytometry analysis of cell surface AXL in NIH3T3-Mock and NIH3T3-AXL cl.7 fibroblasts. Polyclonal NIH3T3-Mock and clonal NIH3T3-AXL cl.7 cells, generated by infection of NIH3T3 fibroblasts with pLXSN and pLXSN-AXL ecotrophic virus, respectively, were collected and stained with mouse control antibody 72A1 (left panel) or mouse anti-AXL MAB154 primary antibody (right panel) at 3 µg/ml and PE-conjugated anti-mouse secondary antibody. See text for details. Staining of NIH3T3-AXL cl.7 cells results in a shift by two orders of magnitude and demonstrates AXL overexpression on the surface of these cells.

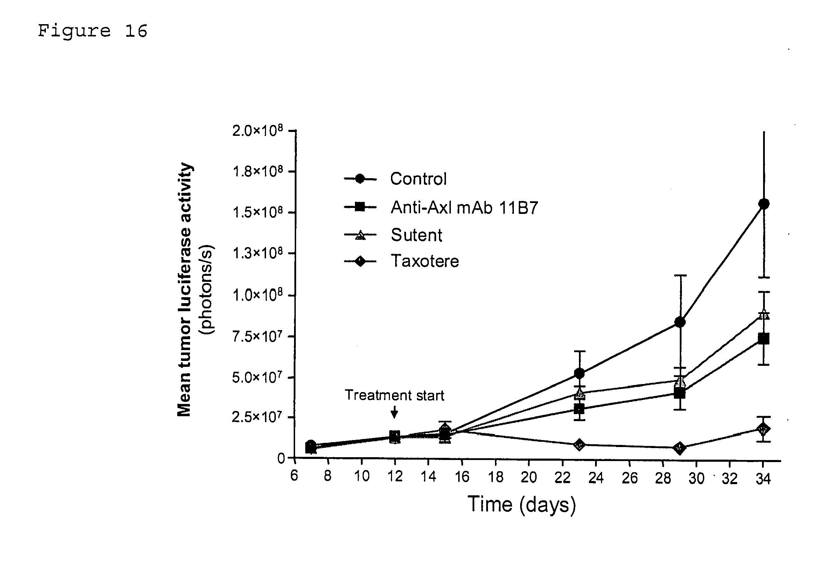

**Figure 16****.** Orthotopic xenograft model to investigate the effects of rat anti-AXL antibodies on human prostate carcinoma growth in nude mice. PC-3-LN prostate carcinoma cells were orthotopically implanted into the prostate of NMRI-nu/nu mice. Animals were randomized into 4 groups and received 25 mg/kg of the isotypic control antibody 1 D5 or the antagonistic rat anti-AXL antibody 11 B7, as well as 40 mg/kg Sutent or 12.5 mg/kg Taxotere. During the treatment period, the growth of orthotopically growing PC-3-LN tumors as well as peripheral metastases was monitored once weekly via in vivo bioluminescence imaging on day 15, day 23, day 29, and day 34. See text for details. Compared to the isotypic control antibody 1 D5, the antagonistic rat anti-AXL antibody 11 B7 reduced the overall growth of PC-3-LN prostate tumors in nude mice.

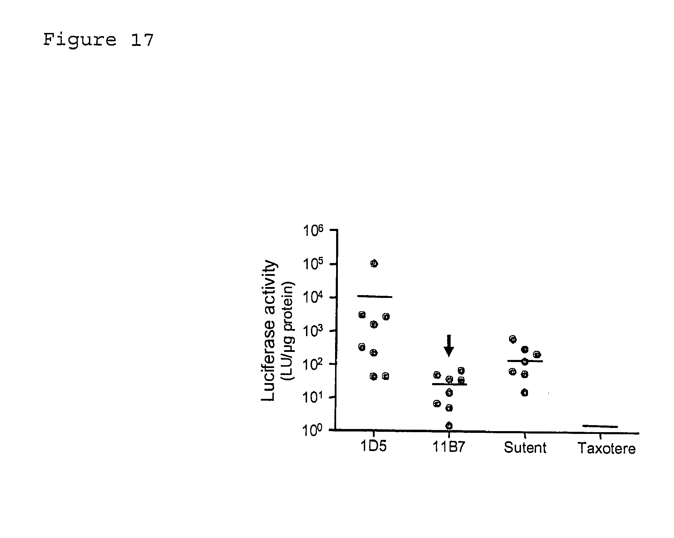

**Figure 17****.** Orthotopic xenograft model to investigate the effects of rat anti-AXL antibodies on human prostate carcinoma metastasis in nude mice. PC-3-LN prostate carcinoma cells were orthotopically implanted into the prostate of NMRI-nu/nu mice. Animals were randomized into 4 groups and received 25 mg/kg of the isotypic control antibody 1D5 or the antagonistic rat anti-AXL antibody 11 B7, as well as 40 mg/kg Sutent or 12.5 mg/kg Taxotere. Post necropsy, selected organs (liver, spleen, lung, femur, and a part of the lumbar spine) were collected and analyzed for the presence of metastases via bioluminescence imaging. See text for details. Compared to the isotypic control antibody 1D5, the antagonistic rat anti-AXL antibody 11 B7 of the invention reduced the occurrence of spleen metastases. Noteworthy, the anti-metastatic effect of 11 B7 in this experiment was stronger than that of Sutent.

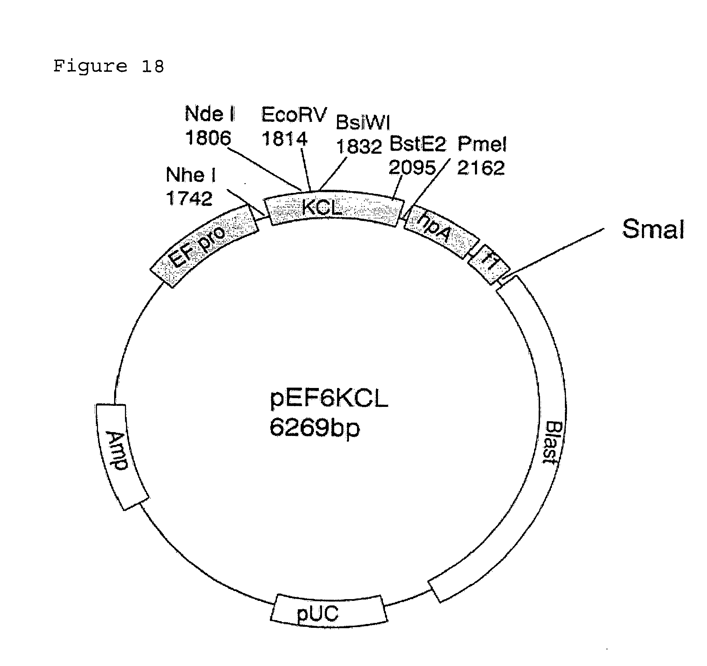

**Figure 18****:** This figure describes the construct of the vector pEF6KCL.

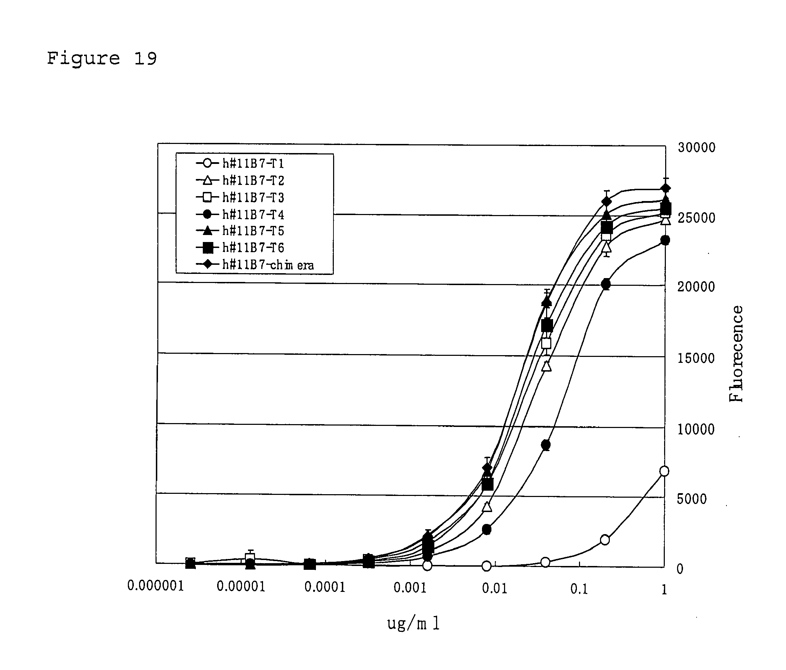

**Figure 19****:** This figure describes binding activity of the rat anti-human AXL humanized antibodies h#11B7-T1 to h#11B7-T6 determined by ELISA using human AXL-Fc coated plate.

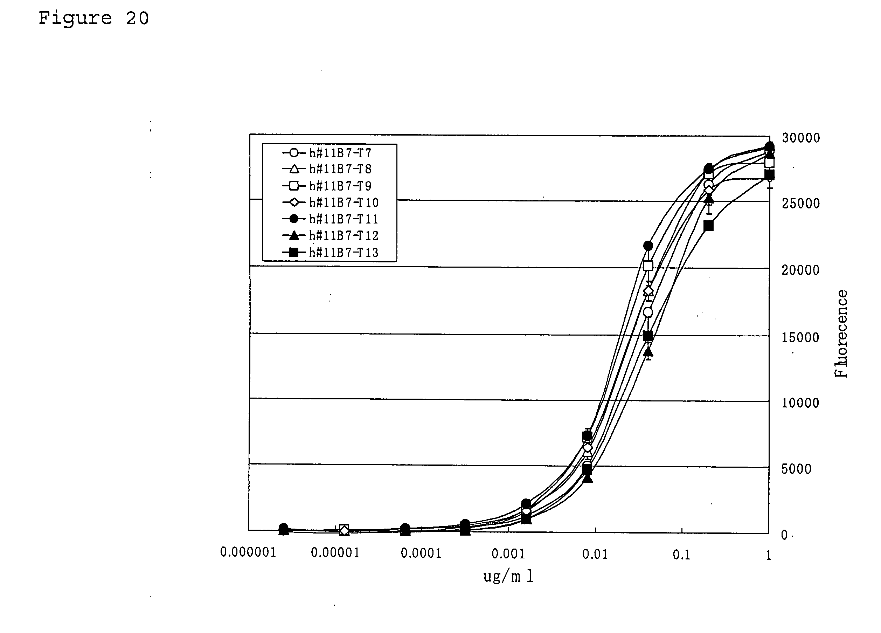

**Figure 20****:** This figure describes binding activity of the rat anti-human AXL humanized antibodies h#11B7-T7 to h#11B7-T13 determined by ELISA using human AXL-Fc coated plate.

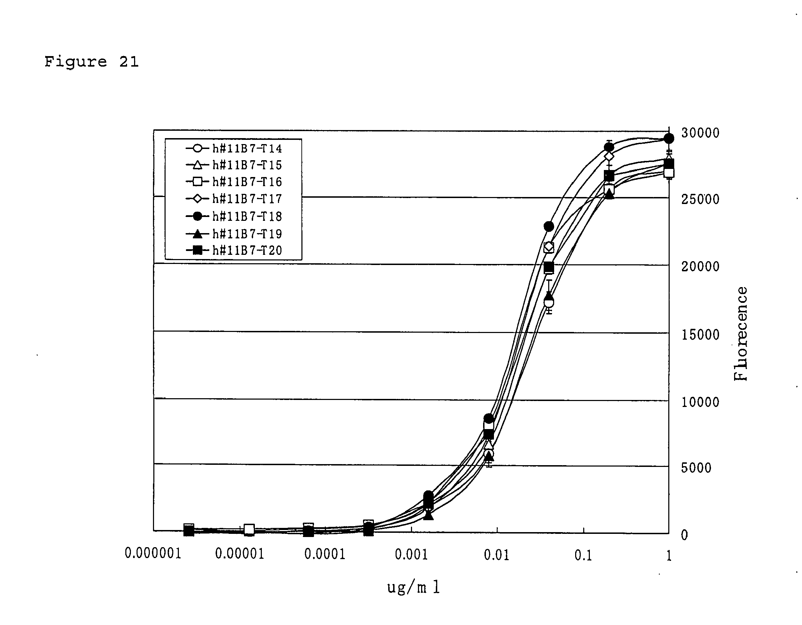

**Figure 21****:** This figure describes binding activity of the rat anti-human AXL humanized antibodies h#11B7-T14 to h#11B7-T20 determined by ELISA using human AXL-Fc coated plate.

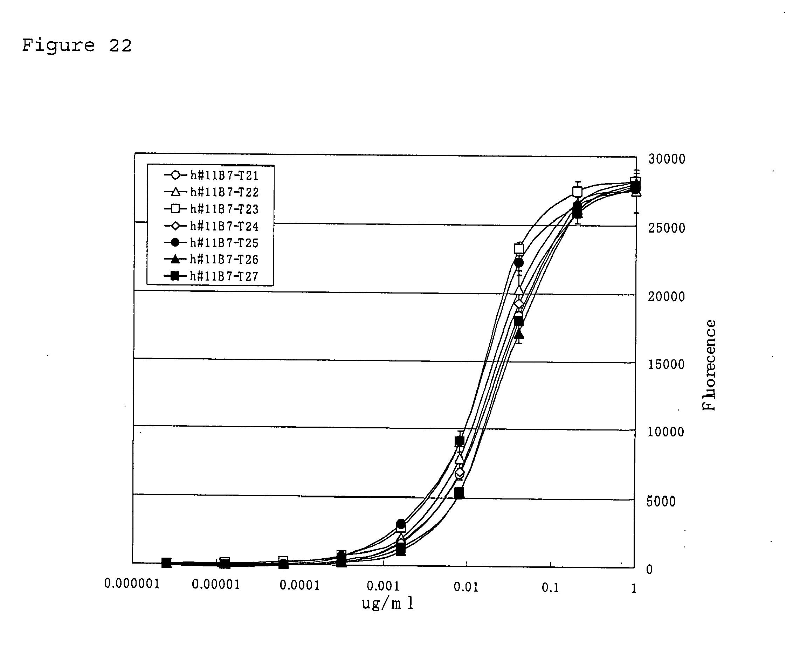

**Figure 22****:** This figure describes binding activity of the rat anti-human AXL humanized antibodies h#11B7-T21 to h#11B7-T27 determined by ELISA using human AXL-Fc coated plate.

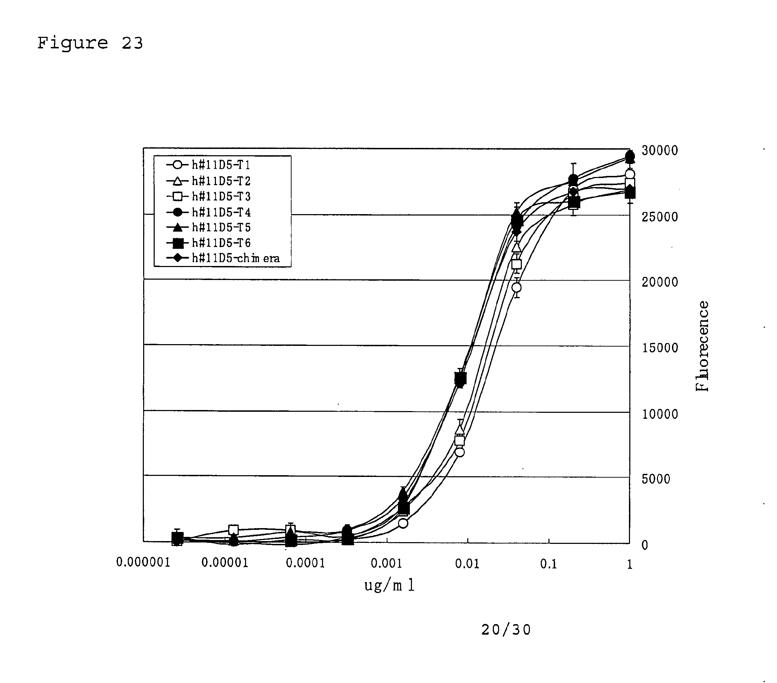

**Figure 23****:** This figure describes binding activity of the rat anti-human AXL humanized antibodies h#11D5-T1 to h#11D5-T6 determined by ELISA using human AXL-Fc coated plate.

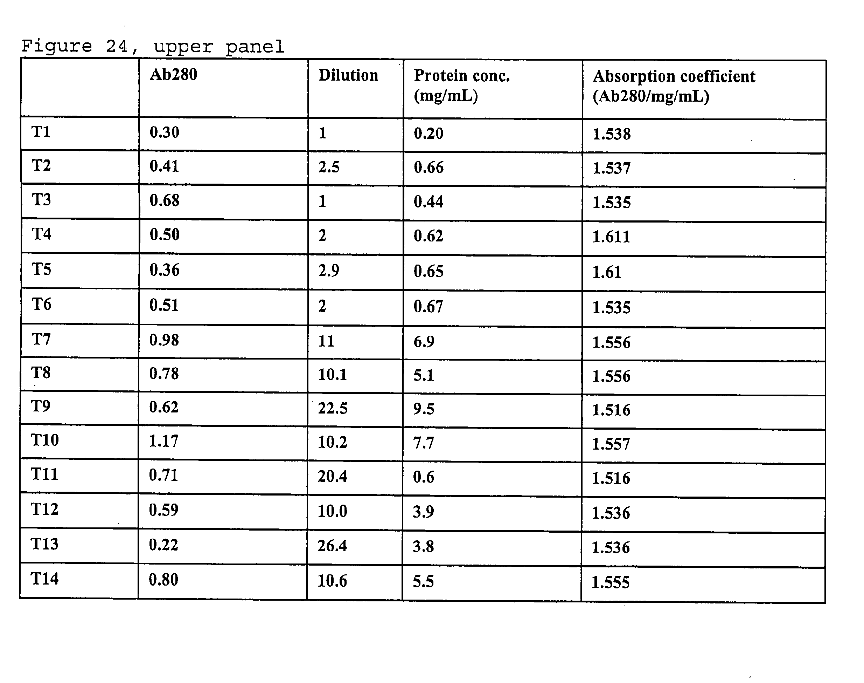

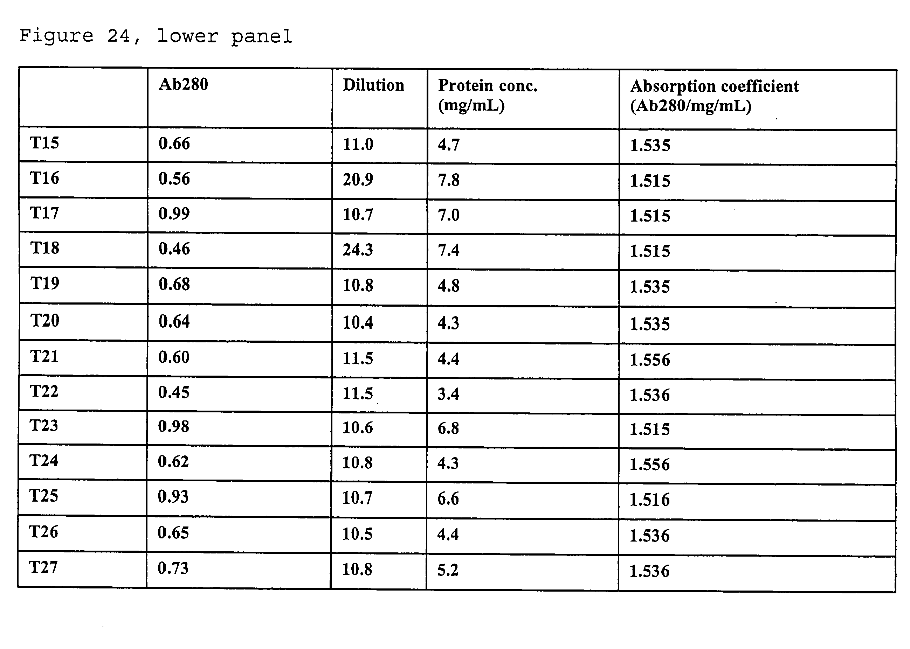

**Figure 24** (upper and lower panels): This figure describes protein concentration of the rat anti-human AXL humanized antibodies h#11B7-T1 to h#11B7-T27 determined by direct protein absorbance assay.

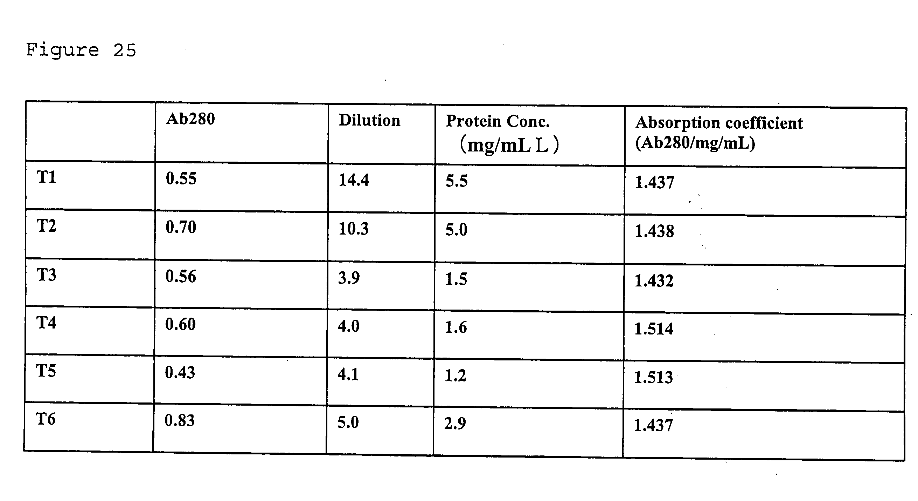

**Figure 25****:** This figure describes protein concentration of the rat anti-human AXL humanized antibodies h#11D5-T1 to h#11D5-T6 determined by direct protein absorbance assay.

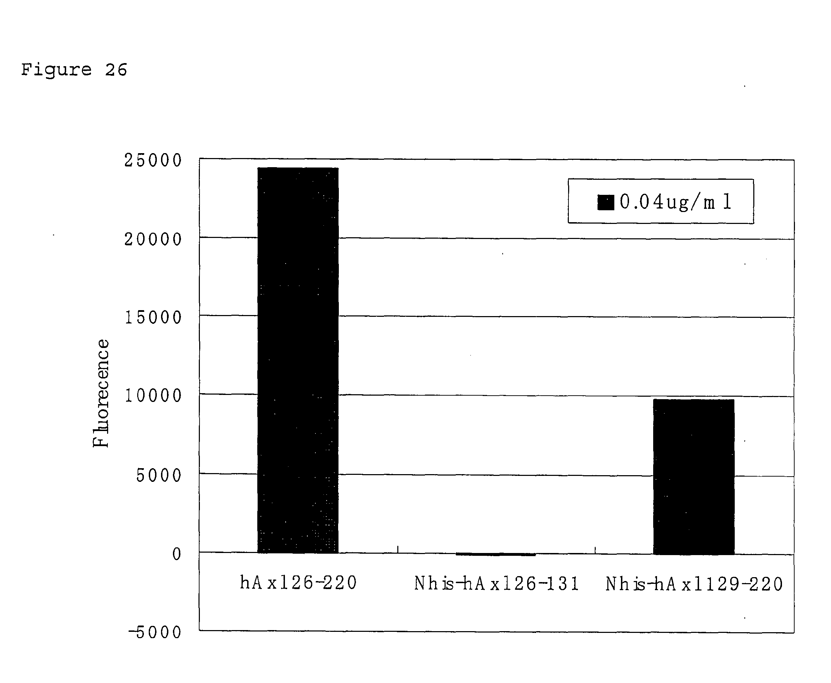

**Figure 26****:** This figure describes a determination of the domain to which the rat anti-human AXL monoclonal antibody #11 B7 binds on the human AXL antigen.

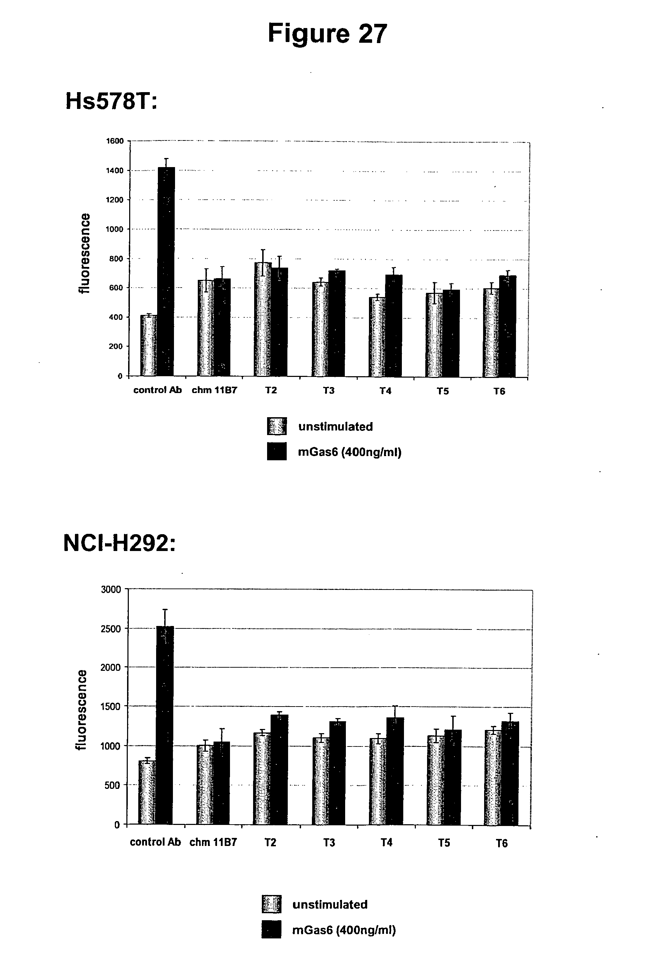

**Figure 27****. ELISA experiments to investigate the effects of humanized 11B7 anti-Axl antibodies on Axl receptor phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/Kappa) as well as the humanized anti-Axl antibodies h#11B7-T2, h#11B7-T3, h#11B7-T4, h#11B7-T5, and h#11B7-T6 (T2-T6) of the invention, treated with or without 400 ng/ml mGas6, and lysed. Lysates were transferred to anti-phospho-tyrosine antibody 4G10-coated Maxi-Sorp 96 well plates, which then were washed and incubated with 0.125 µg/ml biotinylated rat anti-Axl antibody 12B7, AP-conjugated streptavidin and AttoPhos substrate solution in order to collect fluorescence intensities. See text for details. Compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention were able to block or significantly reduce Gas6-mediated Axl activation in both cell lines as indicated by decreased Axl tyrosine phosphorylation levels in Gas6-stimulated versus corresponding non-stimulated cells.

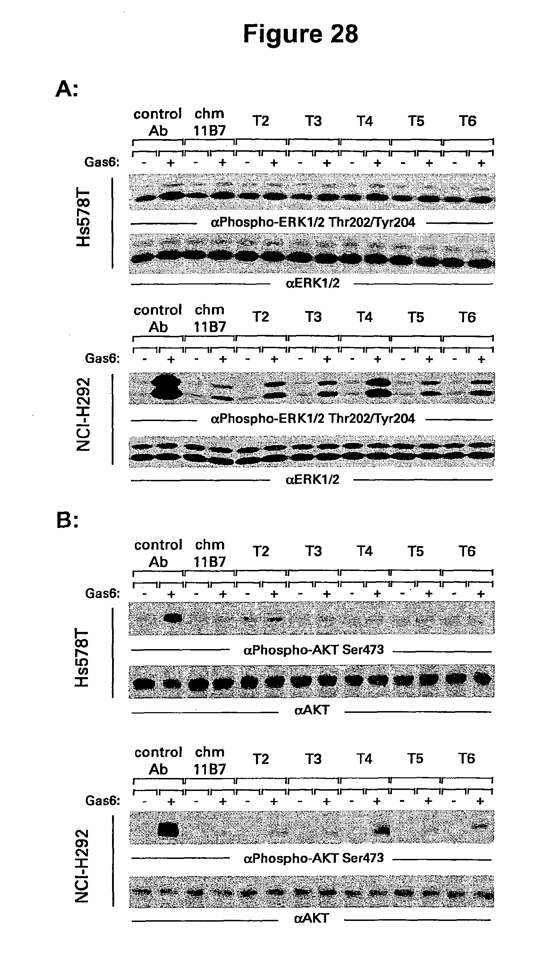

**Figure 28A****. Western Blot experiments to investigate the effects of humanized 11 B7 anti-Axl antibodies on ERK1/2 phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/Kappa) or the humanized anti-Axl antibodies h#11B7-T2, h#11B7-T3, h#11B7-T4, h#11B7-T5, and h#11B7-T6 of the invention prior to treatment with or without 400 ng/ml mGas6, and lysed. Whole cell lysates were separated by SDS-PAGE and analyzed by Western Blotting using anti-phospho-p44/42 MAP kinase (Thr202/Tyr204) antibody. Subsequently, the same filters were re-probed with anti-p44/42 MAP kinase antibody antibody. See text for details. Compared with Gammagard control antibody, the chimeric anti-Axl antibody chm 11 B7 as well as the humanized anti-Axl antibodies h#11B7-T2, h#11B7-T3, h#11B7-T4, h#11B7-T5, and h#11B7-T6 of the invention interfered with the weak, Gas6-induced increase of ERK1/2 activation in Hs578T breast cancer cells. Due to the high basal activity of ERK1/2 in this cell line, however, these effects which are indicated by decreased ERK1/2 Thr202/Tyr204 phosphorylation levels in Gas6-stimulated versus corresponding non-stimulated cells appear relatively moderate only. In contrast, the inhibitory effects of the chimeric anti-Axl antibody chm11B7 as well as the humanized anti-Axl antibodies h#11B7-T2, h#11B7-T3, h#11B7-T4, h#11B7-T5, or h#11B7-T6 on ERK1/2 Thr202/Tyr204 phosphorylation are much clearer reflected in NCI-H292 lung cancer cells.
**Figure 28B****. Western Blot experiments to investigate the effects of humanized 11 B7 anti-Axl antibodies on AKT phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11 B7 (IgG1/Kappa) or the humanized anti-Axl antibodies h#11B7-T2, h#11B7-T3, h#11B7-T4, h#11B7-T5, and h#11B7-T6 of the invention prior to treatment with or without 400 ng/ml mGas6, and lysed. Whole cell lysates were separated by SDS-PAGE and analyzed by Western Blotting using anti-AKT1/2/3 antibody. Subsequently, the same filter was re-probed with anti-phospho-AKT (Ser473) antibody. See text for details. Compared with Gammagard control antibody, the chimeric anti-Axl antibody chm 11 B7 as well as the humanized anti-Axl antibodies h#11B7-T2, h#11B7-T3, h#11B7-T4, h#11B7-T5, and h#11B7-T6 of the invention were able to significantly reduce Gas6-induced AKT activation in Hs578T breast cancer cells (top) and NCI-H292 lung cancer cells (bottom) as indicated by decreased AKT Ser473 phosphorylation levels in Gas6-stimulated versus corresponding non-stimulated cells.

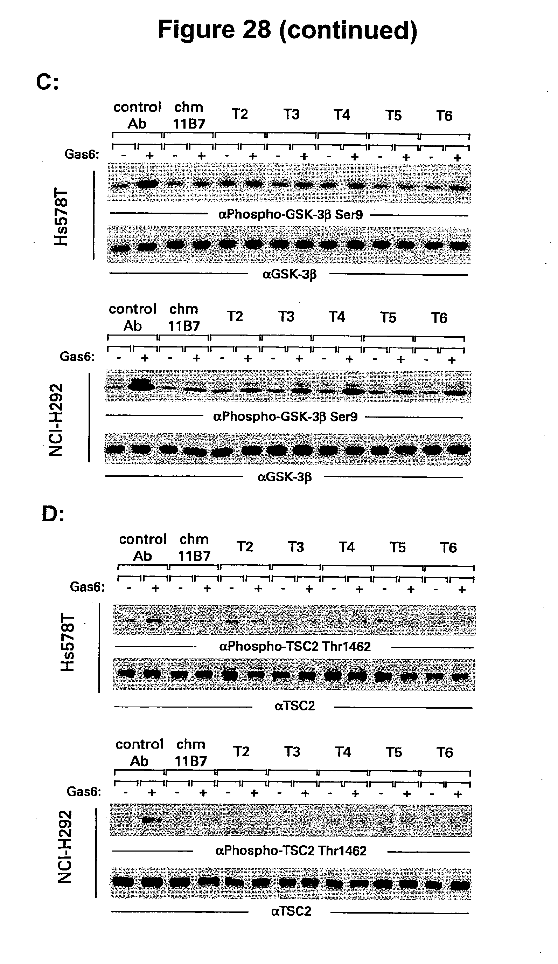

**Figure 28C****. Western Blot experiments to investigate the effects of humanized 11B7 anti-Axl antibodies on GSK-3**β **phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/kappa) or the humanized anti-Axl antibodies h#11B7-T2, h#11B7-T3, h#11B7-T4, h#11B7-T5, and h#11B7-T6 of the invention prior to treatment with or without 400 ng/ml mGas6, and lysed. Whole cell lysates were separated by SDS-PAGE and analyzed by Western Blotting using anti-phospho-GSK-3β (Ser9) antibody. Subsequently, the same filter was re-probed with anti-GSK-3β antibody. See text for details. Compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention were able to significantly reduce Gas6-induced GSK-3β activation in Hs578T breast cancer cells (top) and NCI-H292 lung cancer cells (bottom) as indicated by decreased GSK-3β Ser9 phosphorylation levels in Gas6-stimulated versus corresponding non-stimulated cells.
**Figure 28D****. Western Blot experiments to investigate the effects of humanized 11 B7 anti-Axl antibodies on TCS2 phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/kappa) or the humanized anti-Axl antibodies h#11B7-T2, h#11B7-T3, h#11B7-T4, h#11B7-T5, and h#11B7-6 of the invention prior to treatment with or without 400 ng/ml mGas6, and lysed. Whole cell lysates were separated by SDS-PAGE and analyzed by Western Blotting using anti-TSC2 antibody. Subsequently, the same filter was re-probed with anti-phospho-TSC2 (Thr1462). See text for details. Compared with Gammagard control antibody, the chimeric anti-Axl antibody chm11 B7 as well as the humanized anti-Axl antibodies h#11B7-T2, h#11B7-T3, h#11B7-T4, h#11B7-T5, and h#11B7-T6 of the invention were able to significantly reduce Gas6-induced phosphorylation of TCS2 on Thr1462 in Hs578T breast cancer cells (top) and NCI-H292 lung cancer cells (bottom) as indicated by decreased phosphorylation levels of this amino acid residue in Gas6-stimulated versus corresponding non-stimulated cells.

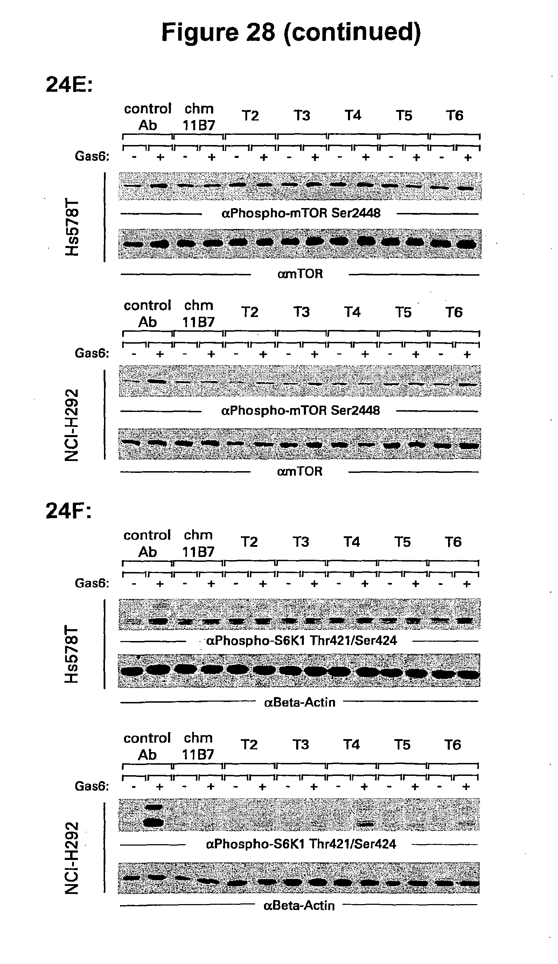

**Figure 28E****. Western Blot experiments to investigate the effects of humanized 11 B7 anti-Axl antibodies on mTOR phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/Kappa) or the humanized anti-Axl antibodies h#11B7-T2, h#11B7-T3, h#11B7-T4, h#11B7-T5, and h#11B7-T6 of the invention prior to treatment with or without 400 ng/ml mGas6, and lysed. Whole cell lysates were separated by SDS-PAGE and analyzed by Western Blotting using anti-phospho-mTOR (Ser2448) antibody. Subsequently, the same filter was re-probed with anti-mTOR antibody. See text for details. The inhibitory effects of the anti-Axl antibodies were relatively weak in Hs578T breast cancer cells (top). However, compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention were able to interfere with the Gas6-induced mTOR activation in NCI-H292 lung cancer cells as indicated by decreased mTOR Ser2448 phosphorylation levels in Gas6-stimulated versus corresponding non-stimulated cells (bottom).
**Figure 28F****. Western Blot experiments to investigate the effects of humanized 11B7 anti-Axl antibodies on S6K1 phosphorylation.** Hs578T breast cancer cells (top) and NCI-H292 lung cancer cells (bottom) were starved, pre-incubated with 10 µg/ml of Gammagard control antibody, the chimeric anti-Axl mAb chm11B7 (IgG1/Kappa) or the humanized anti-Axl antibodies h#11B7-T2, h#11B7-T3, h#11B7-T4, h#11B7-T5, and h#11B7-T6 of the invention prior to treatment with or without 400 ng/ml mGas6, and lysed. Whole cell lysates were separated by SDS-PAGE and analyzed by Western Blotting using anti-phospho-p70 S6 Kinase 1 (Thr421/Ser424) antibody. Subsequently, the same filter was re-probed with anti-β-actin antibody. See text for details. Compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention showed some inhibitory effects on Gas6-induced S6K1 activation in Hs578T breast cancer cells as indicated by decreased S6K1 Thr421/Ser424 phosphorylation levels in Gas6-stimulated versus corresponding non-stimulated cells (top). However, a much stronger decrease of Gas6-induced S6K1 Thr421/Ser424 phosphorylation and thus activation upon pre-treatment with the chimeric anti-Axl antibody chm11B7 as well as the humanized anti-Axl antibodies h#11B7-T2, h#11B7-T3, h#11B7-T4, h#11B7-T5, or h#11B7-T6 of the invention could be observed in NCI-H292 lung cancer cells (bottom).

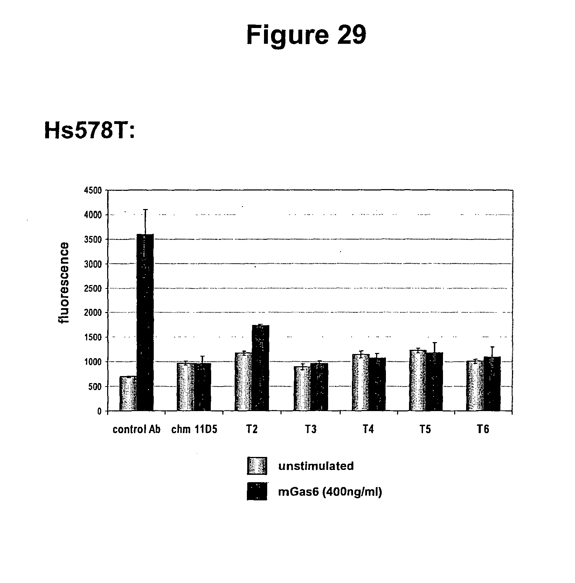

**Figure 29****. ELISA experiments to investigate the effects of humanized 11D5 anti-Axl antibodies on Axl receptor phosphorylation.** Hs578T breast cancer cells were starved, pre-incubated with 10 µg/ml of Gammagard control antibody, the chimeric anti-Axl mAb chm11D5 (IgG1/Kappa) as well as the humanized anti-Axl antibodies h#11D5-T2, h#11D5-T3, h#11D5-T4, h#11D5-T5, and h#11D5-T6 of the invention, treated with or without 400 ng/ml mGas6, and lysed. Lysates were transferred to anti-phospho-tyrosine antibody 4G10-coated Maxi-Sorp 96 well plates, which then were washed and incubated with 0.125 µg/ml biotinylated rat anti-Axl antibody 12B7, AP-conjugated streptavidin and AttoPhos substrate solution in order to collect fluorescence intensities. See text for details. Compared with Gammagard control antibody, the chimeric anti-Axl antibody chm11D5 as well as the humanized anti-Axl antibodies h#11D5-T2, h#11D5-T3, h#11D5-T4, h#11D5-T5, and h#11D5-T6 of the invention were able to block or significantly reduce Gas6-mediated Axl activation as indicated by decreased Axl tyrosine phosphorylation levels in Gas6-stimulated versus corresponding non-stimulated cells.
**Figure 30A****:** The amino acid sequence of human Axl, Accession No. P_30530 of NCBI protein database
**Figure 30B****:** The amino acid sequence of the variable region of the light chain of the rat anti-human Axl monoclonal antibody 11B7
**Figure 30C****:** The amino acid sequence of the variable region of the heavy chain of the rat anti-human Axl monoclonal antibody 11B7
**Figure 30D****:** The amino acid sequence of the variable region of the light chain of the rat anti-human Axl monoclonal antibody 11D5
**Figure 30E****:** The amino acid sequence of the variable region of the heavy chain of the rat anti-human Axl monoclonal antibody 11D5

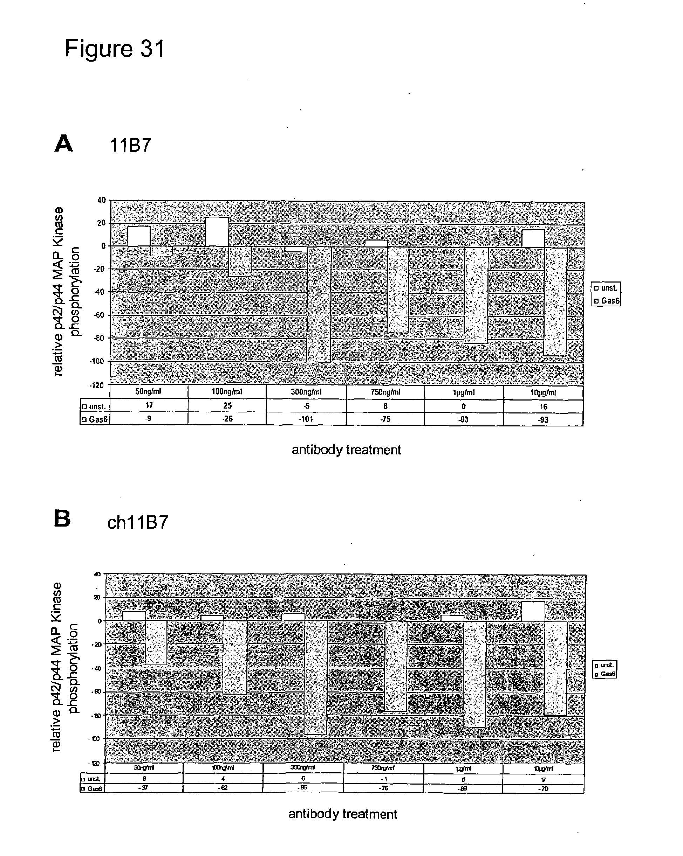

**Figure 31****. ELISA experiments to compare the effects of rat and chimeric anti-Axl antibodies on Axl phosphorylation.** CaSki cervical cancer cells were starved, pre-incubated with 50 ng/ml, 100 ng/ml, 300 ng/ml, 750 ng/ml, 1 jJg/ml, and 10 jJg/ml of rat anti-Axl antibody 11 B7 (A) or chimeric anti Axl antibody ch11 B7 (B), treated with or without 400 ng/ml mGas6, and lysed. Lysates were transferred to anti-phospho-tyrosine antibody 4G10-coated Maxi-Sorp 96 well plates. Afterwards, plates were washed and incubated with 0.5 IJg/ml of biotinylated rat anti-Axl antibody 12B7, AP-conjugated streptavidin, and AttoPhos substrate solution in order to collect fluorescence intensities. See text for details. As demonstrated by concentration-dependent decrease of the relative Axl phosphorylation in the cervical cancer cell line CaSki, the rat anti-Axi antibody 11B7 (A) and the chimeric anti-Axl antibody ch11B7 (B) of the invention were able to block ligand-induced activation of the receptor tyrosine kinase Axl to similar extent.

### Description of the Sequences

[SEQ ID NO:1] the nucleotide sequence of a primer for amplifying Fragment A, designated as Primer EFF1
[SEQ ID NO:2] the nucleotide sequence of a primer for amplifying Fragment A, designated as Primer EFsmaR
[SEQ ID NO:3] the nucleotide sequence of Fragment B comprising the secretion signal and constant region of human kappa chain, and the signal for addition of poly (A)
[SEQ ID NO:4] the nucleotide sequence of a DNA fragment comprising the nucleotide sequence encoding the amino acid sequence of the signal sequence and constant region of human IgG1
[SEQ ID NO:5] the nucleotide sequence encoding the amino acid sequence of the h#11B7-T1L, signal sequence(1-60), variable region(61-387), constant region(388-702)
[SEQ ID NO:6] the nucleotide sequence encoding the amino acid sequence of the h#11B7-T2L, signal sequence(1-60), variable region(61-387), constant region(388-702)
[SEQ ID NO:7] the nucleotide sequence encoding the amino acid sequence of the h#11B7-T3L, signal sequence(1-60), variable region(61-387), constant region(388-702)
[SEQ ID NO:8] the nucleotide sequence encoding the amino acid sequence of the h#11B7-T4L, signal sequence(1-60), variable region(61-387), constant region(388-702)
[SEQ ID NO:9] the nucleotide sequence encoding the amino acid sequence of the h#11B7-T5L, signal sequence(1-60), variable region(61-387), constant region(388-702)
[SEQ ID NO:10] the nucleotide sequence encoding the amino acid sequence of the h#11B7-T6L, signal sequence(1-60), variable region(61-387), constant region(388-702)
[SEQ ID NO:11] the nucleotide sequence encoding the amino acid sequence of the h#11B7-T7L, signal sequence(1-60), variable region(61-387), constant region(388-702)
[SEQ ID NO:12] the nucleotide sequence encoding the amino acid sequence of the h#11B7-T8L, signal sequence(1-60), variable region(61-387), constant region(388-702)
[SEQ ID NO:13] the nucleotide sequence encoding the amino acid sequence of the h#11B7-T9L, signal sequence(1-60), variable region(61-387), constant region(388-702)
[SEQ ID NO:14] the nucleotide sequence encoding the amino acid sequence of the h#11B7-T10L, signal sequence(1-60), variable region(61-387), constant region(388-702)
[SEQ ID NO:15] the nucleotide sequence encoding the amino acid sequence of the h#11B7-T11L, signal sequence(1-60), variable region(61-387), constant region(388-702)
[SEQ ID NO:16] the nucleotide sequence encoding the amino acid sequence of the h#11B7-T12L, signal sequence(1-60), variable region(61-387), constant region(388-702)
[SEQ ID NO:17] the nucleotide sequence encoding the amino acid sequence of the h#11B7-T13L, signal sequence(1-60), variable region(61-387), constant region(388-702)
[SEQ ID NO:18] the amino acid sequence of the h#11B7-T1L, signal sequence(1-20), variable region(21-129), constant region(130-234)
[SEQ ID NO:19] the amino acid sequence of the h#11B7-T2L, signal sequence(1-20), variable region(21-129), constant region(130-234)
[SEQ ID NO:20] the amino acid sequence of the h#11B7-T3L, signal sequence(1-20), variable region(21-129), constant region(130-234)
[SEQ ID NO:21] the amino acid sequence of the h#11B7-T4L, signal sequence(1-20), variable region(21-129), constant region(130-234)
[SEQ ID NO:22] the amino acid sequence of the h#11B7-T5L, signal sequence(1-20), variable region(21-129), constant region(130-234)
[SEQ ID NO:23] the amino acid sequence of the h#11B7-T6L, signal sequence(1-20), variable region(21-129), constant region(130-234)
[SEQ ID NO:24] the amino acid sequence of the h#11B7-T7L, signal sequence(1-20), variable region(21-129), constant region(130-234)
[SEQ ID NO:25] the amino acid sequence of the h#11B7-T8L, signal sequence(1-20), variable region(21-129), constant region(130-234)
[SEQ ID NO:26] the amino acid sequence of the h#11B7-T9L, signal sequence(1-20), variable region(21-129), constant region(130-234)
[SEQ ID NO:27] the amino acid sequence of the h#11B7-T10L, signal sequence(1-20), variable region(21-129), constant region(130-234)
[SEQ ID NO:28] the amino acid sequence of the h#11B7-T11L, signal sequence(1-20), variable region(21-129), constant region(130-234)
[SEQ ID NO:29] the amino acid sequence of the h#11B7-T12L, signal sequence(1-20), variable region(21-129), constant region(130-234)
[SEQ ID NO:30] the amino acid sequence of the h#11B7-T13L, signal sequence(1-20), variable region(21-129), constant region(130-234)
[SEQ ID NO:31] the nucleotide sequence encoding the amino acid sequence of the h#11B7-T1H, signal sequence(1-57), variable region(58-396), constant region(397-1386)
[SEQ ID NO:32] the nucleotide sequence encoding the amino acid sequence of the h#11B7-T2H, signal sequence(1-57), variable region(58-396), constant region(397-1386)
[SEQ ID NO:33] the nucleotide sequence encoding the amino acid sequence of the h#11B7-T3H, signal sequence(1-57), variable region(58-396), constant region(397-1386)
[SEQ ID NO:34] the nucleotide sequence encoding the amino acid sequence of the h#11B7-T4H, signal sequence(1-57), variable region(58-396), constant region(397-1386)
[SEQ ID NO:35] the nucleotide sequence encoding the amino acid sequence of the h#11B7-T5H, signal sequence(1-57), variable region(58-396), constant region(397-1386)
[SEQ ID NO:362] the nucleotide sequence encoding the amino acid sequence of the h#11B7-T6H, signal sequence(1-57), variable region(58-396), constant region(397-1386)
[SEQ ID NO:37] the nucleotide sequence encoding the amino acid sequence of the h#11B7-T7H, signal sequence(1-57), variable region(58-396), constant region(397-1386)
[SEQ ID NO:38] the nucleotide sequence encoding the amino acid sequence of the h#11B7-T8H, signal sequence(1-57), variable region(58-396), constant region(397-1386)
[SEQ ID NO:39] the nucleotide sequence encoding the amino acid sequence of the h#11B7-T9H, signal sequence(1-57), variable region(58-396), constant region(397-1386)
[SEQ ID NO:40] the amino acid sequence of the h#11B7-T1H, signal sequence(1-19), variable region(20-132), constant region(133-462)
[SEQ ID NO:41] the amino acid sequence of the h#11B7-T2H, signal sequence(1-19), variable region(20-132), constant region(133-462)
[SEQ ID NO:42] the amino acid sequence of the h#11B7-T3H, signal sequence(1-19), variable region(20-132), constant region(133-462)
[SEQ ID NO:43] the amino acid sequence of the h#11B7-T4H, signal sequence(1-19), variable region(20-132), constant region(133-462)
[SEQ ID NO:44] the amino acid sequence of the h#11B7-T5H, signal sequence(1-19), variable region(20-132), constant region(133-462)
[SEQ ID NO:45] the amino acid sequence of the h#11B7-T6H, signal sequence(1-19), variable region(20-132), constant region(133-462)
[SEQ ID NO:46] the amino acid sequence of the h#11B7-T7H, signal sequence(1-19), variable region(20-132), constant region(133-462)
[SEQ ID NO:47] the amino acid sequence of the h#11B7-T8H, signal sequence(1-19), variable region(20-132), constant region(133-462)
[SEQ ID NO:48] the amino acid sequence of the h#11B7-T9H, signal sequence(1-19), variable region(20-132), constant region(133-462)
[SEQ ID NO:49] the nucleotide sequence encoding the amino acid sequence of the h#11D5-T1L, signal sequence(1-60), variable region(61-387), constant region(388-702)
[SEQ ID NO:50] the nucleotide sequence encoding the amino acid sequence of the h#11D5-T2L, signal sequence(1-60), variable region(61-387), constant region(388-702)
[SEQ ID NO:51] the nucleotide sequence encoding the amino acid sequence of the h#11D5-T3L, signal sequence(1-60), variable region(61-387), constant region(388-702)
[SEQ ID NO:52] the nucleotide sequence encoding the amino acid sequence of the h#11D5-T4L, signal sequence(1-60), variable region(61-387), constant region(388-702)
[SEQ ID NO:53] the nucleotide sequence encoding the amino acid sequence of the h#11D5-T5L, signal sequence(1-60), variable region(61-387), constant region(388-702)
[SEQ ID NO:54] the nucleotide sequence encoding the amino acid sequence of the h#11D5-T6L, signal sequence(1-60), variable region(61-387), constant region(388-702)
[SEQ ID NO:55] the amino acid sequence of h#11D5-T1L, signal sequence(1-20), variable region(21-129), constant region(130-234)
[SEQ ID NO:56] the amino acid sequence of h#11D5-T2L, signal sequence(1-20), variable region(21-129), constant region(130-234)
[SEQ ID NO:57] the amino acid sequence of h#11D5-T3L, signal sequence(1-20), variable region(21-129), constant region(130-234)
[SEQ ID NO:58] the amino acid sequence of h#11D5-T4L, signal sequence(1-20), variable region(21-129), constant region(130-234)
[SEQ ID NO:59] the amino acid sequence of h#11D5-T5L, signal sequence(1-20), variable region(21-129), constant region(130-234)
[SEQ ID NO:60] the amino acid sequence of h#11D5-T6L, signal sequence(1-20), variable region(21-129), constant region(130-234)
[SEQ ID NO:61] the nucleotide sequence encoding the amino acid sequence of the h#11D5-T1H, signal sequence(1-57), variable region(58-396), constant region(297-1386)
[SEQ ID NO:62] the nucleotide sequence encoding the amino acid sequence of the h#11D5-T2H, signal sequence(1-57), variable region(58-396), constant region(297-1386)
[SEQ ID NO:63] the nucleotide sequence encoding the amino acid sequence of the h#11D5-T3H, signal sequence(1-57), variable region(58-396), constant region(297-1386)
[SEQ ID NO:64] the nucleotide sequence encoding the amino acid sequence of the h#11D5-T4H, signal sequence(1-57), variable region(58-396), constant region(297-1386)
[SEQ ID NO:65] the nucleotide sequence encoding the amino acid sequence of the h#11D5-T5H, signal sequence(1-57), variable region(58-396), constant region(297-1386)
[SEQ ID NO:66] the nucleotide sequence encoding the amino acid sequence of the h#11D5-T6H, signal sequence(1-57), variable region(58-396), constant region(297-1386)
[SEQ ID NO:67] the amino acid sequence of h#11D5-T1H, signal sequence(1-19), variable region(20-132), constant region(133-462)
[SEQ ID NO:68] the amino acid sequence of h#11D5-T2H, signal sequence(1-19), variable region(20-132), constant region(133-462)
[SEQ ID NO:69] the amino acid sequence of h#11D5-T3H, signal sequence(1-19), variable region(20-132), constant region(133-462)
[SEQ ID NO:70] the amino acid sequence of h#11D5-T4H, signal sequence(1-19), variable region(20-132), constant region(133-462)
[SEQ ID NO:71] the amino acid sequence of h#11D5-T5H, signal sequence(1-19), variable region(20-132), constant region(133-462)
[SEQ ID NO:72] the amino acid sequence of h#11D5-T6H, signal sequence(1-19), variable region(20-132), constant region(133-462)
[SEQ ID NO:73]the amino acid sequence of h#11B7-T14L, variable region
[SEQ ID NO:74] the amino acid sequence of h#11B7-T15L, variable region
[SEQ ID NO:75] the amino acid sequence of h#11B7-T16L, variable region
[SEQ ID NO:76] the amino acid sequence of h#11B7-T17L, variable region
[SEQ ID NO:77] the amino acid sequence of h#11B7-T18L, variable region
[SEQ ID NO:78] the amino acid sequence of h#11B7-T19L, variable region
[SEQ ID NO:79] the amino acid sequence of h#11B7-T20L, variable region
[SEQ ID NO:80] the amino acid sequence of h#11B7-T10H, variable region
[SEQ ID NO:81] the amino acid sequence of h#11B7-T11H, variable region
[SEQ ID NO:82] the amino acid sequence of h#11B7-T12H, variable region
[SEQ ID NO:83] the amino acid sequence of h#11D5-T7L, variable region
[SEQ ID NO:84] the amino acid sequence of h#11D5-T8L, variable region
[SEQ ID NO:85] the amino acid sequence of h#11D5-T9L, variable region
[SEQ ID NO:86] the amino acid sequence of h#11D5-T10L, variable region
[SEQ ID NO:87] the amino acid sequence of h#11D5-T11L, variable region
[SEQ ID NO:88] the amino acid sequence of h#11D5-T12L, variable region
[SEQ ID NO:89] the amino acid sequence of h#11D5-T13L, variable region
[SEQ ID NO:90] the amino acid sequence of h#11D5-T14L, variable region
[SEQ ID NO:91] the amino acid sequence of h#11D5-T15L, variable region
[SEQ ID NO:92] the amino acid sequence of h#11D5-T16L, variable region
[SEQ ID NO:93] the amino acid sequence of h#11D5-T17L, variable region
[SEQ ID NO:94] the amino acid sequence of h#11D5-T18L, variable region
[SEQ ID NO:95] the amino acid sequence of h#11D5-T19L, variable region
[SEQ ID NO:96] the amino acid sequence of h#11D5-T20L, variable region
[SEQ ID NO:97] the amino acid sequence of h#11D5-T21L, variable region
[SEQ ID NO:98] the amino acid sequence of h#11D5-T22L, variable region
[SEQ ID NO:99] the amino acid sequence of h#11D5-T23L, variable region
[SEQ ID NO:100] the amino acid sequence of h#11D5-T24L, variable region
[SEQ ID NO:101] the amino acid sequence of h#11D5-T25L, variable region
[SEQ ID NO:102] the amino acid sequence of h#11D5-T26L, variable region
[SEQ ID NO:103] the amino acid sequence of h#11D5-T27L, variable region
[SEQ ID NO:104] the amino acid sequence of h#11D5-T28L, variable region
[SEQ ID NO:105] the amino acid sequence of h#11D5-T29L, variable region
[SEQ ID NO:106] the amino acid sequence of h#11D5-T30L, variable region
[SEQ ID NO:107] the amino acid sequence of h#11D5-T31L, variable region
[SEQ ID NO:108] the amino acid sequence of h#11D5-T32L, variable region
[SEQ ID NO:109] the amino acid sequence of h#11D5-T33L, variable region
[SEQ ID NO:110] the amino acid sequence of h#11D5-T34L, variable region
[SEQ ID NO:111] the amino acid sequence of h#11D5-T35L, variable region
[SEQ ID NO:112] the amino acid sequence of h#11D5-T36L, variable region
[SEQ ID NO:113] the amino acid sequence of h#11D5-T37L, variable region
[SEQ ID NO:114] the amino acid sequence of h#11D5-T7H, variable region
[SEQ ID NO:115] the amino acid sequence of h#11D5-T8H, variable region
[SEQ ID NO:116] the amino acid sequence of h#11D5-T9H, variable region
[SEQ ID NO:117] the amino acid sequence of h#11D5-T10H, variable region
[SEQ ID NO:118 the amino acid sequence of h#11D5-T11H, variable region
[SEQ ID NO:119] the amino acid sequence of h#11D5-T12H, variable region
[SEQ ID NO:120] the amino acid sequence of h#11D5-T13H, variable region
[SEQ ID NO:121] the amino acid sequence of 11B7 Light chain CDRL4
[SEQ ID NO:122] the amino acid sequence of 11 B7 Light chain CDRL5
[SEQ ID NO:123] the amino acid sequence of 11B7 Light chain CDRL6
[SEQ ID NO:124] the amino acid sequence of 11B7 Heavy chain CDRH1
[SEQ ID NO:125] the amino acid sequence of 11 B7 Heavy chain CDRH2
[SEQ ID NO:126] the amino acid sequence of 11 B7 Heavy chain CDRH3
[SEQ ID NO:127] the amino acid sequence of 11 D5 Light chain CDRL4
[SEQ ID NO:128] the amino acid sequence of 11 D5 Light chain CDRL5
[SEQ ID NO:129] the amino acid sequence of 11 D5 Light chain CDRL6
[SEQ ID NO:130] the amino acid sequence of 11 D5 Heavy chain CDRH1
[SEQ ID NO:131] the amino acid sequence of 11 D5 Heavy chain CDRH2
[SEQ ID NO:132] the amino acid sequence of 11D5 Heavy chain CDRH3
[SEQ ID NO:133] nucleotide sequence of the Leader sequence
[SEQ ID NO:134] the amino acid sequence of the Leader sequence
[SEQ ID NO:135] the amino acid sequence of #11 B7-chimeric light chain
[SEQ ID NO:136] the amino acid sequence of #11 B7-chimeric heavy chain
[SEQ ID NO:137] the amino acid sequence of #11 D5-chimeric light chain
[SEQ ID NO:138] the amino acid sequence of #11D5-chimeric heavy chain
[SEQ ID NO: 139] the amon acid sequence of human Axl, Accession No. P_30530 of NCBI protein database, which is also described Figure 30A
[SEQ ID NO: 140] the amino acid sequence of the variable region of the light chain of the rat anti-human Axl monoclonal antibody 11B7, which is also described in Figure 30B, and which is identical to the amino acid sequence consisting of the amino acids No. 1 to 108 of the #11B7-chimeric light chain (SEQ ID NO: 135)
[SEQ ID NO: 141] the amino acid sequence of the variable region of the heavy chain of the rat anti-human Axl monoclonal antibody 11B7, which is also described in Figure 30C, and which is identical to the amino acid sequence consisting of the amino acids No. 1 to 113 of the #11 B7-chimeric heavy chain (SEQ ID NO: 136)
[SEQ ID NO: 142] the amino acid sequence of the variable region of the light chain of the rat anti-human Axl monoclonal antibody 11D5, which is also described in Figure 30D, and which is identical to the amino acid sequence consisting of the amino acid No. 1 to 108 of the #11D5-chimeric light chain (SEQ ID NO: 137)
[SEQ ID NO: 143] the amino acid sequence of the variable region of the heavy chain of the rat anti-human Axl monoclonal antibody 11D5, which is also described in Figure 30E, and which is identical to the amino acid sequence consisting of the amino acid No. 1 to 113 of the #11D5-chimeric heavy chain (SEQ ID NO: 138)

### Examples

### Example 1. Generation of AXL overexpressing Ratl fibroblasts as immunogen

The full length coding sequence for the human receptor tyrosine kinase AXL transcript variant 1 according to the National Center for Biotechnology Information (NCBI) reference sequence (NM_021913) was subcloned into pLXSN via flanking recognition elements for the restriction endonucleases EcoRI and BamHI, thereby resulting in the retroviral expression vector pLXSN-hAXL.
For the generation of antibodies that specifically bind to human receptor tyrosine kinase AXL, RatI fibroblasts stably overexpressing human AXL were generated by retroviral gene transfer. In brief, 3x105 Phoenix-E cells were seeded on 60 mm culture dishes and transfected with 2 µg/ml pLXSN vector or pLXSN-hAXL using the calcium phosphate method. After 24 h, medium was replaced by fresh medium in which Phoenix-E cells were incubated for 4 h. The supernatants of Phoenix-E cells releasing pLXSN or pLXSN-hAXL ecotrophic virus were harvested and used for the incubation of subconfluent Ratl cells (2x105 cells per 6 cm dish) for 3 h in the presence of Polybrene (4 mg/ml; Aldrich). Simultaneously, Phoenix-E cells were re-incubated with fresh medium, which after another 3 h was used for a second infection of the RatI fibroblasts in the presence of Polybrene (4 mg/ml; Aldrich). Likewise, a third infection cycle was performed. After changing the medium, selection of Ratl cells with G418 was started. Usually, stable clones were picked after selection for 21 days.
A panel of stable clones was propagated and quantified for membrane-localized human AXL expression by FACS analysis. In detail, 1x105 cells were harvested with 10 mM EDTA in PBS, washed once with FACS buffer (PBS, 3% FCS, 0.4% azide) and seeded on a 96 well round bottom plate. The cells were spun for 3 min at 1,000 rpm to remove supernatant and were resuspended with mouse anti-AXL primary antibody MAB154 (R&D Systems, 3 µg/ml). Cell suspensions were incubated on ice for 1 h, washed twice with FACS buffer and resuspended in 100 µl/well of PE-conjugated donkey anti-mouse secondary antibody (Jackson) diluted 1:50 in FACS buffer. The cell suspensions were incubated on ice and in the dark for 30 min, washed twice with FACS buffer and analyzed using an Epics XL-MCL flow cytometer (Beckman Coulter).

**Figure 15A** shows the FACS analysis of the polyclonal Ratl-Mock population stably infected with pLXSN empty vector and Ratl-AXL cl.2 stably infected with pLXSN-hAXL, and demonstrates AXL overexpression on the cell surface of this representative clone.
Additionally, in order to generate a suitable cellular model system for experimental purposes, NIH3T3 fibroblasts stably overexpressing AXL were generated in analogy to procedures described for Ratl. In brief 3x105 Phoenix-E cells were seeded on 60 mm culture dishes and transfected with 2 µg/ml pLXSN vector or pLXSN-AXL cDNA using the calcium phosphate method. After 24 h, medium was replaced by fresh medium in which Phoenix-E cells were incubated for 4 h. The supernatants of Phoenix-E cells releasing pLXSN or pLXSN-hAXL ecotrophic virus were harvested and used for the incubation of subconfluent NIH3T3 cells (2x105 cells per 6 cm dish) for 3 h in the presence of Polybrene (4 mg/ml; Aldrich). Simultaneously, Phoenix-E cells were re-incubated with fresh medium, which after another 3 h was used for a second infection of the NIH3T3 fibroblasts in the presence of Polybrene (4 mg/ml; Aldrich). Likewise, a third infection cycle was performed. After changing the medium, selection of NIH3T3 cells with G418 was started. Usually, stable clones were picked after selection for 21 days.
A panel of stable clones was propagated and quantified for membrane-localized AXL expression by FACS analysis. In detail, 1x105 cells were harvested with 10 mM EDTA in PBS, washed once with FACS buffer (PBS, 3% FCS, 0.4% azide) and seeded on a 96 well round bottom plate. The cells were spun for 3 min at 1000 rpm to remove supernatant and were resuspended with mouse anti-AXL primary antibody MAB154 (R&D Systems, 3 µg/ml). Cell suspensions were incubated on ice for 1 h, washed twice with FACS buffer and resuspended in 100 µl/well of PE-conjugated donkey anti-mouse secondary antibody (Jackson) diluted 1:50 in FACS buffer. The cell suspensions were incubated on ice and in the dark for 30 min, washed twice with FACS buffer and analyzed using an Epics XL-MCL flow cytometer (Beckman Coulter).

**Figure 15B** shows the FACS analysis of the polyclonal NIH3T3-Mock population stably infected with pLXSN empty vector and NIH3T3-AXL cl.7 stably infected with pLXSN-hAXL, and demonstrates AXL overexpression on the cell surface of this representative clone.

### Example 2. Generation of rat anti-AXL monoclonal antibodies

Monoclonal rat anti-AXL antibodies were raised by injection of approximately 10x106 frozen cells of Ratl-AXL cl.2 both i.p. and subcutaneously into Lou/C or Long Evans rats. After an 8-week interval, a final boost was given i.p and subcutaneously 3 d before fusion. Fusion of the myeloma cell line P3X63-Ag8.653 with the rat immune spleen cells was performed according to standard procedures and yielded 105 hybridomas. After 2 weeks, first supernatants from hybridomas were collected and tested in a primary FACS screen for binding to NIH3T3-AXL cl.7 fibroblasts versus NIH3T3-Mock control cells. Clones positive for AXL binding were further cultivated. From 50 ml supernatant of these clones, antibodies were purified and re-analyzed for specific binding to AXL on NIH3T3-AXL cl.7 fibroblasts versus NIH3T3-Mock control cells. Purified antibodies specifically binding to NIH3T3-AXL cl.7 fibroblasts but not NIH3T3-Mock control cells were furthermore tested in Akt-Kinase phosphorylation ELISAs, and ELISAs to determine the isotype were performed. For purification of rat antibodies, supernatants were spun for 20 minutes at 5,000g and subsequently sterile filtered. 500 µl of protein G sepharose FF were added and incubated at 4°C for at least 1 h on a spinning wheel. Sepharose was spun down, supernatant discarded and protein G matrix was washed twice with PBS prior to protein elution utilizing citrate buffer (100mM) pH 2.1. Elution fractions were immediately rebuffered to neutral pH by adding 1 M Tris pH 8.0 and dialyzed against PBS.
Of the oligoclonal antibodies tested, 91 specifically bound to NIH3T3-AXL cl.7 fibroblasts but not NIH3T3-Mock control cells, 9 inhibited Gas6-induced Akt phosphorylation in the same cells, whereas 71 stimulated Akt phosphorylation. Four antagonistic antibodies (I11B7, I10D12, 16E7, and III11D5, in the following examples referred to as 11B7, 10D12, 6E7 and 11D5, respectively), two agonistic antibodies (I11D7 and III2A1; in the following examples referred to as 11D7 and 2A1) and one control antibody (III1D5; in the following examples referred to as 1D5) were kryoconserved and subcloned.

### Example 3: Structure and characteristics of anti-AXL antibodies

### 3.1. Nucleotide sequences of rat antibody variable domains

Rat anti-AXL antibody variable domains were cloned from hybridoma cells. RNA was prepared utilizing the RNA-Extraction kit RNeasy (RNeasy midi-kit, Qiagen). cDNA encoding for the antibody genes was prepared using the 5'RACE kit (Invitrogen) according to manufacturer's instructions.
Briefly, first strand cDNA was synthesized from total or RNA using the gene-specific GSP1-primers and SuperScript™ II Reverse Transcriptase. After first strand cDNA synthesis, the original mRNA template is removed by treatment with the RNase Mix. A homopolymeric tail is then added to the 3'-end of the cDNA. PCR amplification is accomplished using Taq DNA polymerase, a nested, gene-specific primer (GSP2) that anneals to a site located within the cDNA molecule and an anchor primer provided with the kit. Following amplification 5' RACE products were cloned into the pLXSN-ESK vector for sequencing. To facilitate cloning the Anchor Primer (AP) included a recognition sequence for Sal I, GSP2 primers contained a XhoI site.

GSP1 primer:

| | |
|---|---|
| kappa_GSP1: | GATGGATGCATTGGTGCAGC |
| new_kappa_GSP1: | atagatacagttggtgcagc |
| heavy_GSP1: | CAGGGTCACCATGGAGTTA |

GSP2 primer:
Xhol-hGSP2: CCGCTCGAGCGGGCCAGTGGATAGACAGATGG
Xhol-kGSP2: CCGCTCGAGCGGCCGTTTCAGCTCCAGCTTGG

Utilization of GSP primers for rat anti-AXL Mab cloning:

| | |
|---|---|
| 11B7: | kappa GSP1; Xhol-kGSP2 |
| | heavy GSP1; Xhol-hGSP2 |
| | |
| 10D12: | kappa_GSP1, new_kappa_GSP1; Xhol-kGSP2 |
| | heavy GSP1; Xhol-hGSP2 |
| | |
| 11D5: | new_kappa_GSP1; Xhol-kGSP2 |
| | heavy GSP1; Xhol-hGSP2 |

### 3.2. Aminoacid sequence rat anti-AXL antibody variable domains

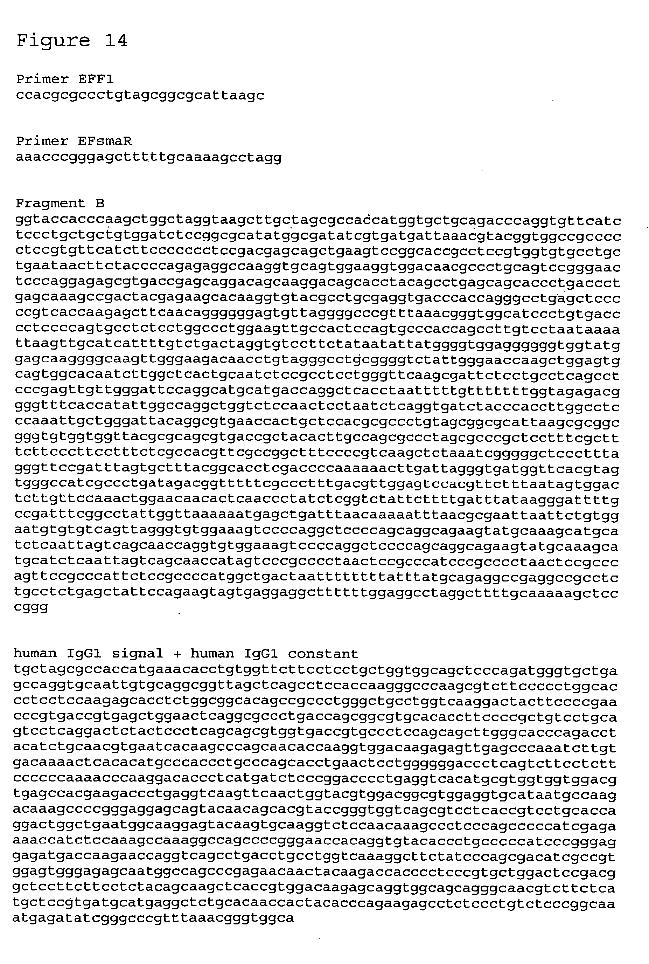

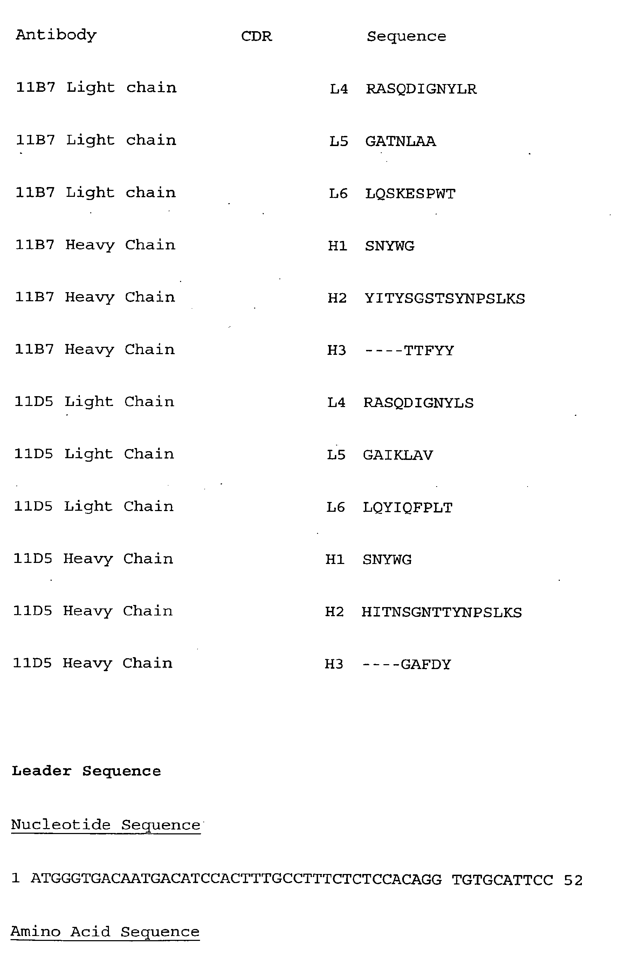

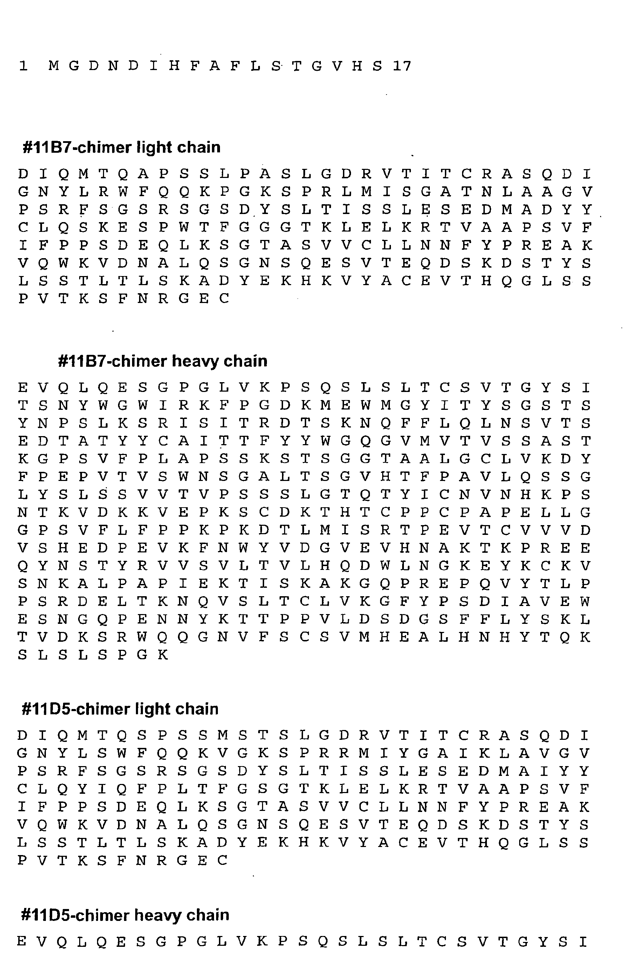

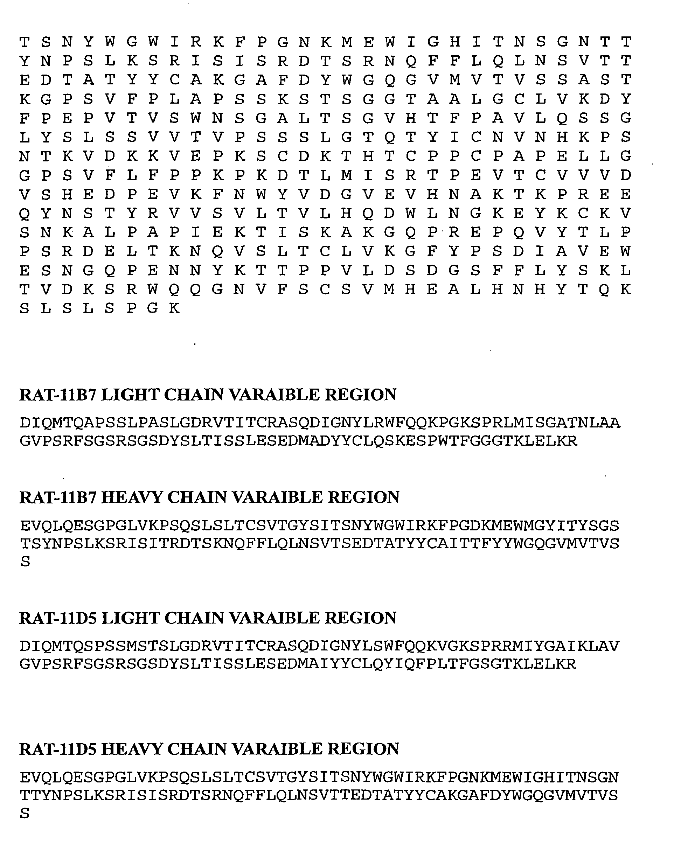

Rat antibody variable domain sequences were translated from sequenced genes cloned into the pLXSN-ESK vectors. The given amino acid sequences start at position one of the variable domain. The complementarity determining regions (CDRs) required for the specific binding of the antibody to its target are defined according to Kabat (Kabat et al. Sequences of Proteins of Immunological Interest, Fifth Edition. NIH Publication No. 91-3242, 1991). The Kabat definition is based on the sequence variability within the variable domains. Anti-AXL specific CDR regions of the antibodies are listed in Figure 14 and SEQ ID NO: 121 to SEQ ID NO: 132. The individual CDRs include the following positions:

| | |
|---|---|
| CDR-L1: | 24-34 |
| CDR-L2: | 50-56 |
| CDR-L3: | 89-97 |
| | |
| CDR-H1: | 31-35b |
| CDR-H2: | 50-65 |
| CDR-H3: | 95-102 |

The amino acid sequence of the rat 11 B7 antibody light and heavy chain variable regions are represented by SEQ ID NOs: 140 and 141, respectively. The subclasses of the light and heavy chains of the rat 11B7 antibody are kappa and IgG1, respectively.

The amino acid sequence of the rat 11 D5 antibidy light and heavy chain variable regions are represented by SEQ ID NOs: 142 and 143, respectively. The subclasses of the light and heavy chains of the rat 11D5 antibody are kappa and IgG2a, respectively.

### 3.3 Rat antibody expression and purification:

Hybridomas were cultured in Celline CL 1000 bioreactors (Integra Biosciences) at 37°C, 5-7% CO2 using DMEM including 4.5g/L glucose; 1% Glutamine, 1 % Pyrovate 1 % Pen/Strep. FCS supplementation is 1 % FCS for the nutrient compartment and 5 % low IgG FCS for the cell compartment. Harvest and media exchange is performed twice a week. Cell splitting 1/1 - >1/3 depending on cell growth. Productivity is tested once a week via SDS-PAGE analysis. Supernatants are stored at -20°C until purification. Mycoplasma test of running cultures is done once a week.

Antibodies are purified using Protein A or G Sepharose FF (GE-Healthcare) via an Äkta Explorer 100 System (GE-Healthcare). Columns are individually packed for each purification. The column size is adjusted to the expected productivity and size of each batch (usually 50 - 500 mg). Protein containing solutions are kept on ice or at 4°C wherever possible. Sterile buffers and double destilled water are used for the entire process.
Supernatants are thawed, buffered with 50 mM TRIS pH 8.5, centrifuged, filtered through a 0.22 µm membrane and loaded onto the column. After washing with 8 column volumes (CV) 50 mM PO4, pH8.5 the antibody is eluted within 10 CV 100 mM Glycin, pH 3.3. Eluate fractions are rebuffered immediately to neutral pH by adding 1/5 1M Tris pH 8.0 (1 ml Tris per 4ml eluate fraction) and analysed by rSDS-PAGE subsequently. Fractions containing pure antibody are pooled, dialysed against PBS at 4°C and sterile filtered.

Buffer system requirements are adjusted according to the individual properties of each antibody. In particular, rat IgG2a antibody 11D5 was bound to ProteinG 4 FF matrix (GE-Healthcare) and washed under high salt conditions (2M NaCl). Rat antibody IgG1 11 B7 was purified via rProteinA (GE-Healthcare) under high salt conditions according to 11D5. Antibody elution was performed at pH 5.5. Flow rate for rat antibody purification has to be kept low for increased binding efficiency.

As a second purification step either ion exchange chromatography (under individual, suitable conditions) or preparative size exclusion chromatography (PBS, pH 7.4) can be implemented.

The standard protocol for quality control of the purified antibodies includes:
rSDS-PAGE gel analysis; Coommassie or silver stained
BCA test (Pierce #23227 BCA Protein Assay Kit; rat IgG standard #31233)
Analytical size exclusion (Superdex 200 Tricorn 10/300 GL,∼250 mg in 250 µl; 0.5 ml/min, Äkta Explorer 100)
Endotoxin test (LAL, Cambrex QCL-1000® Chromogenic LAL Endpoint Assay # US50-648U)
Cell based activity assays (FACS binding; pAkt; pAXL)

Purified antibodies are stored in PBS, pH 7.4 under steril conditions at 4°C or -20°C depending on their stability.

One of the obtained rat antibodies, 11 B7, was applied to Examples 5, 6 and 16.

### 3.4. Antibody Affinity determination by FACS scatchard

Human AXL overexpression NIH3T3 cells were harvested by incubation with 10 mM EDTA in PBS and resuspended at 6 million cells per ml in FACS buffer (PBS pH 7.4, 3% FCS, 0.1 % NaN3). In a round-bottom microtiter plate, 100 µl of cell suspension were added to 100µl of antibody solution containing antibodies 11B7, 11D5, ch11B7-IgG1, ch11B7-IgG2, ch11D5-IgG1 or ch11D5-IgG2 at concentrations between 40 and 0.002 µg/ml (266 and 0.01 nM) in FACS buffer. Antibody binding was allowed to proceed for 2 hours on ice. Then, cells were washed twice with 250 µl FACS buffer per well, and resuspended in 200µl of secondary antibody (anti-rat-PE; Jackson) diluted 1:50 in FACS buffer. After 45 minutes of incubation, cells were again washed twice in FACS buffer and resuspended in 500ml PBS for FACS analysis. Analysis was carried out on a Beckman-Coulter FACS FC500. To determine the apparent affinity constant KDapp, mean fluorescence values were plotted against the ratio of mean fluorescence and the corresponding antibody concentration ([M]). The calculated KDapp resulted from the inverse slope of the straight line are listed below:

| Clone | KD value (nM) |
|---|---|
| 11B7 | 0,38 |
| ch11B7 | 0,6 |
| 11D5 | 0,81 |
| Ch11D5 | 0,4 |

The KD values listed above include those of chimeric antibodies obtained in Example 4.

### Example 4: Chimerization of rat anti-AXL antibodies:

Human kappa light chain and heavy chain IgG1/2 genes were cloned from peripheral blood mononuclear cells (PBMC) of a human volunteer as described below:
PBMCs were prepared from whole blood. Blood was diluted 1/2,5 in PBS/ 2 mM EDTA with 10 U/ml heparin at RT, layered over 15 ml Biocoll solution covered by a diaphragm ( 35 ml/ tube) [Biocoll from Biochrom # L6115]. Samples were centrifuged at RT for 30 min at 400xg and serum (∼15 ml) was discarded. Interface containing PBMCs was carefully recovered using a Pasteur pipette. PBMCs were washed 2x in PBS/2 mM EDTA (first wash 100ml, second wash 50 ml) and spun down at 300xg for 10 min. Cell pellet was resuspended in RPMI/10% FCS (25 ml) and yielded 5.5x10^7 PBMCs. RNA was prepared from PBMCs using RNeasy kit from Qiagen (# 75142) according to manufacturer's instructions. Purified RNA (30 µg) was stored in aliquots at -80°C.
cDNA for antibody IgG gamma 1 and 2 as well as kappa chains were prepared from isolated RNA by RT-PCR using Superskript III Reverse Transkriptase (invitrogen # 18080 - 93) according to manufacturers instructions using the following primers:

| | |
|---|---|
| 1) RT-gamma: | GCG TGT AGT GGT TGT GCA GAG |
| 2) RT-gamma2: | ggg ctt gcc ggc cgt g |
| 3) RT-kappa: | TGG AAC TGA GGA GCA GGT GG |
| 4) 5'Blp: | AGA TAA GCT TTG CTC AGC GTC CAC CAA GGG CCC ATC GGT |
| 5) 3'Bam(GAG): | AGA TGG ATC CTC ATT TAC CCG GAG ACA GGG AGA G |
| 6) 5'Bsi: | AGA TAA GCT TCG TAC GGT GGC TGC ACC ATC TGT CTT CAT |
| 7) 3'Bam(CTT): | AGA TGG ATC CCT AAC ACT CTC CCC TGT TGA AGC TCT |

Primers were dissolved at 100µM. RT-PCR reactions were performed using 2pmol oligo RTy and RTκ respectively, adding 1 µg RNA, 10 mM dNTP mix and heat for 5 min to 65 °C. 4µl first strand buffer, 1µl 0.1M DTT, 1 µl RNase inhibitor (40 U/µl Fermentas # E00311) and 2 µl Superscript III RT were added, mixed and incubated at 50°C for 1 h followed by a heat inactivation step for 15 min at 70 °C.
2µl of first strand reaction were used for second step PCR using Taq polymerase (Eurochrom # EME010001) to yield double stranded DNA of antibody constant domains. The primer 5'Blp and 3'Bam(GAG) were used to amplify. γchain, and 5'Bsi and 3'Bam(CTT) were used to amplify κ-chain constant regions using the following PCR settings:.

| κ-chain amplification: | |
|---|---|
| 94°C | 120 sec |
| | |
| 94°C | 30 sec |
| 55°C | 30 sec |
| 72°C | 45 sec cycle 35 times |
| | |
| 72°C | 10 min |

| γ̃chain amplification: | |
|---|---|
| 94°C | 120 sec |
| | |
| 94°C | 30 sec |
| 45°C | 30 sec |
| 72°C | 60 sec cycle 5 times |
| | |
| 94°C | 30 sec |
| 50°C | 30 sec |
| 72°C | 60 sec cycle 35 times |
| | |
| 72°C | 10 min |

The PCR products were analysed on a TAE buffered 2% agarose gel. A single band of -350 bp for kappa light chain and a single band of - 1000 bp for the heavy chains γ1 and γ2 were found. The PCR products were purified by Qiagen gel extraction kit, (QIAGEN, #28784) according to the manufacturer's instructions. To clone the PCR fragments into the multiple cloning site of the pcDNA3 vector (Invitrogen), pcDNA3 vector and PCR fragments were digested with HindIII (5') and BamHI (3') restriction endonucleases. Restriction sites were encoded within the PCR. primers. Digested fragments were purified using the Qiagen PCR purification kit (QIAGEN, 28104), and DNA encoding the γ1, γ2 and κ chains were ligated into the pcDNA3 vector facilitating T4 DNA ligase at 16°C overnight. Ligase was inactivated for 10 min. at 65°C. Ligated DNA plasmids were directly transformed into CaCl2 competent E.coli using standard protocol and plated onto Ampicillin containing LB-plates. After incubation at 37°C overnight single colonies were picked, suspended in 10 µl H2O and proofed for containing the respective antibody chain carrying plasmid by PCR (5 µl suspended cells, Taq polymerase, primer 5BIp and 3Bam(GAG) γ1/γ2 and 5Bsi and 3Bam(CTT) for κ colonies:

| | |
|---|---|
| 94°C | 120 sec |
| | |
| 94°C | 30 sec |
| 55°C | 30 sec |
| 72°C | 60 sec cycle 35 times |
| | |
| 72°C | 10 min |

Samples were analysed on 1.5% agarose gel for PCR products. Antibody gene containing colonies were selected to inoculate 5 ml LB/Ampicillin medium. After incubation at 37 °C overnight E.coli were harvested and DNA was prepared using Qiagen miniprep kit (QIAGEN, # 12123). A control digest (HindIII, BamHI) showed all κ and γ chain gene inserts at the expected size; sequences were verified by DNA sequencing at Medigenomix.

Rat variable domains were amplified by PCR from pLXSN-ESK vector and cloned into g1/g2 and k pcDNA3 vectors to yield the chimeric full length antibodies. Variable VL domains were amplified with the following primers, containing a HindIII and Bsml site at the 5'end and a BsiWI site at the 3'end:
VL-11 B7-5': AGA TAA GCT TGT GCA TTC CGA CAT CCA GAT GAC CCA GGC TCC
VL-11B7-3': AGA TCG TAC GTT TCA GCT CCA GCT TGG TGC CTC
VL-11 D5-5': AGA TAA GCT TGT GCA TTC CGA CAT CCA GAT GAC CCA GTC TCC ATC
VL-11D5-3': AGA TCG TAC GTT TCA GCT TGG TCC CAG

Variable VH domains were amplified with the following primers, containing a HindIII and BsmI site at the 5'end and a BlpI site at the 3'end:
VH-11B7/11D5-5': AGA TAA GCT TGT GCA TTC CGA GGT GCA GCT TCA GGA GTC AGG
VH-11B7/11D5-3': AGA TGC TGA GCT GAC AGT GAC CAT GAC TCC TTG GCC

BsiWI for the light chain and the BlpI for the heavy chain are single sites at the 5'end of the constant regions to enable the direct fusion with the 3'end of the variable domain genes.
Fused to the Leader sequence SEQ ID No.: 133 derived from pLNOH2 vector (Norderhaug et. al. J. Immunol. Methods 204, 1997; Neuberger EMBO J. 1983; 2 (8): 1373-8,1983) genes encoding the chimeric antibody chains were cloned into pCEP vector system for recombinant expression. Light chain genes were cloned NheI (5') and XhoI (3') into pCEP4 (Invitrogen) heavy chain genes KpnI (5') and XhoI (3') into pCEP-Pu (Kohfeld FEBS Vol 414; (3) 557ff, 1997).

HEK 293 cells seeded on 20x20 cm plates were co-transfected with 1µg/ml of each plasmid coding for light and heavy chain genes using standard CaPO4 transfection method for transient expression. Culture conditions were 37°C, 5% CO2 in DMEM/F12 high glucose medium containing 5% low IgG FCS, 1% pyrovate, 1% glutamine, 1% penicillin/streptomycin. 24h after transfection medium was exchanged by fresh medium. Supernatants were collected every 2-3 days for approximately 3 weeks. Chimeric antibodies were purified from approximatelly 600ml supernatant utilizing 1 ml Hitrap rProtein A columns (GE-Healthcare) under standard buffer conditions (loading: 50 mM Tris; pH=8.5, wash: 50mM PO4; pH= 8.5, elution: 100mM Glycin; pH 3,3) as described for rat antibody purification.

One of the obtained chimeric molecules derived from rat 11 B7 antibody consists of a human IgG1 heavy chain represented by the amino acid sequence of SEQ ID NO: 136 (Figure 14) and a human kappa light chain represented by the amino acid sequence of SEQ ID NO: 135 (Figure 14).

One of the obtained chimeric molecules derived from rat 11D5 antibody consists of a human IgG1 heavy chain represented by the amino acid sequence of SEQ ID NO: 138 (Figure 14) and a human kappa light chain represented by the amino acid sequence of SEQ ID NO: 137 (Figure 14).

These chimeric 11 B7 and 11D5 antibodies, designated as ch11B7-IgG1 and ch11D5-IgG1, respectively, were applied to Examples 13, 14 and 17 to 20.

### Example 5. Rat anti-AXL antibodies of the invention reduce human prostate carcinoma growth in nude mice

The anti-tumor efficacy of therapeutic antibodies is often evaluated in human xenograft tumor studies. In these model systems, human tumors grow as xenografts in immunocompromised mice and therapeutic efficacy is measured by the degree of tumor growth inhibition. The aim of this study was to evaluate whether the antagonistic rat anti-AXL antibody 11 B7 of the invention interferes with tumor growth of human prostate cancer cells in nude mice. In brief, on day 0, 7-8 weeks old male NMRI^{-nu/nu} mice (approximate weight: 30 g after acclimatization) were anesthesized with 1.5-2.0 volume percent isoflurane at an oxygen flow rate of 2 I/min, and 1x10⁶ PC-3-LN cells in 25 µl PBS were orthotopically implanted into the prostate. PC-3-LN cells are derived from the PC-3 prostate carcinoma cell line which was infected with a retrovirus coding for a luciferase-neomycin fusion protein. The onset of tumor growth and tumor growth progression was therefore measurable via *in vivo* bioluminescence imaging. For this purpose, luciferin was injected intraperitoneally (i.p.) into the mice and light emission was measured 10 min post injection using a NightOWL LB 981 bioluminescence imaging system (Berthold Technologies, Germany). Prior to first treatment, mice were randomized and statistical tests performed to assure uniformity in starting tumor volumes (mean, median and standard deviation) across the treatment groups of 10 animals each. On day 8, all treatments started and were continued until day 34, followed by necropsy on day 35. 25 mg/kg of the isotypic control antibody 1 D5 and the antagonistic rat anti-AXL antibody 11 B7 were intraperitoneally (i.p.) administered 3x a week (Mo, Wed, Fr) into animals of group 1 and 2, respectively. Animals of group 3 orally (p.o.) received 40 mg/kg of Sutent once a day. Animals of Group 4 received three intraveneous (i.v.) injections with 12.5 mg/kg of Taxotere 4 days apart of each other. An overview of the treatment groups is given below.

Figure 16 shows the results of this experiment. Compared to the isotypic control antibody 1D5, the antagonistic rat anti-AXL antibody 11 B7 of the invention reduced the overall growth of PC-3-LN prostate tumors in nude mice.

### Example 6. Rat anti-AXL antibodies of the invention inhibit metastasis of human prostate carcinoma

In the same experiment as described under "Rat anti-AXL antibodies of the invention reduce human prostate carcinoma growth in nude mice", relocalization of PC-3-LN tumor cells into other organs (metastasis) was analyzed post necropsy to evaluate anti-metastatic effects of the antagonistic rat anti-AXL antibody 11 B7 of the invention..For this purpose, selected organs (liver, spleen, lungs, femur, part of the lumbar spine) were collected post necropsy, homogenized, and supplemented with luciferin. Subsequently, light emission was measured using a NightOWL LB 981 bioluminescence imaging system (Berthold Technologies, Germany).

**Figure 17** shows the results of this experiment for the analysis of spleens. Compared to the isotypic control antibody 1 D5, the antagonistic rat anti-AXL antibody 11 B7 of the invention reduced the occurrence of spleen metastases. Noteworthy, the anti-metastatic effect of 11 B7 in this experiment was stronger than that of Sutent. Similar observations were obtained for liver, lung, femur, and lumbar spine metastasis.

### Example 7: Design of humanized antibody

### 7.1 Design of humanized version of #1187

### 7.1.1 Molecular modeling of #11B7 variable domains

The molecular modeling of #11B7 variable domains was practised according to the method generally known in the art as homology modeling (Methods in Enzymology, 203, 121-153, (1991)). The primary sequences (three-dimensional structures derived from X-ray crystal structures are available) of human immunoglobulin variable domains registered in Protein Data Bank (Nuc. Acid Res. 35, D301-D303 (2007)) were compared with the #11B7 variable domains thus determined. As a result, 1JPT was selected as having the highest sequence homology to the #11B7 light chain variable domain. Moreover, 1 F8T was selected as having the highest sequence homology to the #11B7 heavy chain variable domain. The three-dimensional structures of framework domains were prepared based on a "framework model" obtained by combining the coordinates of 1JPT and 1 F8T corresponding to the #11B7 light and heavy chains. For #11B7 CDRs, CDRL₁, CDRL₂, CDRL₃, CDRH₁, and CDRH₂ were assigned to clusters 11A, 7A, 9A, 10A, and 9A, respectively, according to the classification of Thornton et al. (J. Mol. Biol., 263, 800-815, (1996)). CDRH₃ was classified in k (3) - according to the H3-rules (FEBS letters 399, 1-8 (1996)). Subsequently, the typical conformation of each CDR was incorporated in the framework model.

Finally, to obtain possible molecular models of the #11B7 variable domains in terms of energy, energy calculation was conducted for excluding disadvantageous interatomic contact. These procedures were performed using a commercially available three-dimensional protein structure prediction program Prime and coordinate search program MacroModel (Schrödinger, LLC).

### 7.1.2 Design of amino acid sequence of humanized #11 B7

Humanized #11 B7 antibodies were constructed according to the method generally known in the art as CDR grafting (Proc. Natl. Acad. Sci. USA 86, 10029-10033 (1989)). Acceptor antibodies were selected in two ways based on amino acid homology within the framework domains.

The sequences of the #11 B7 framework domains were compared with those of all human frameworks registered in the Kabat Database (Nuc. Acid Res. 29, 205-206 (2001)) involving antibody amino acid sequences. As a result, a GM4672'CL antibody was selected as an acceptor due to 72% sequence homology between their framework domains. The amino acid residues of the framework domains in GM4672'CL were aligned with the corresponding amino acid residues in #11 B7 to identify positions where different amino acids there between were used. The positions of these residues were analysed using the three-dimensional model of #11 B7 thus constructed. Then, donor residues to be grafted on the acceptor were selected according to the criteria provided by Queen et al. (Proc. Natl. Acad. Sci. USA 86, 10029-10033 (1989)).

The sequences of the #11 B7 framework domains were compared with those of all human frameworks registered in IgBLAST (Nuc. Acid Res. 36, D25-D30 (2007)). As a result, BAC01582 was selected as an L chain acceptor due to 76% sequence homology between their framework domains. AAF80028 was selected as an H chain acceptor due to 66% sequence homology between their framework domains. The amino acid residues of the framework domains in the BAC01582 L chain and in the AAF80028 H chain were aligned with the corresponding amino acid residues in #11B7 to identify positions where different amino acids therebetween were used. The positions of these residues were analysed using the three-dimensional model of #11B7 thus constructed. Then, donor residues to be grafted on the acceptor were selected according to the criteria provided by Queen et al. (Proc. Natl. Acad. Sci. USA 86, 10029-10033 (1989)).

In all methods, humanized #11B7 sequences were constructed as described in Examples below by transferring some selected donor residues to the acceptor antibodies.

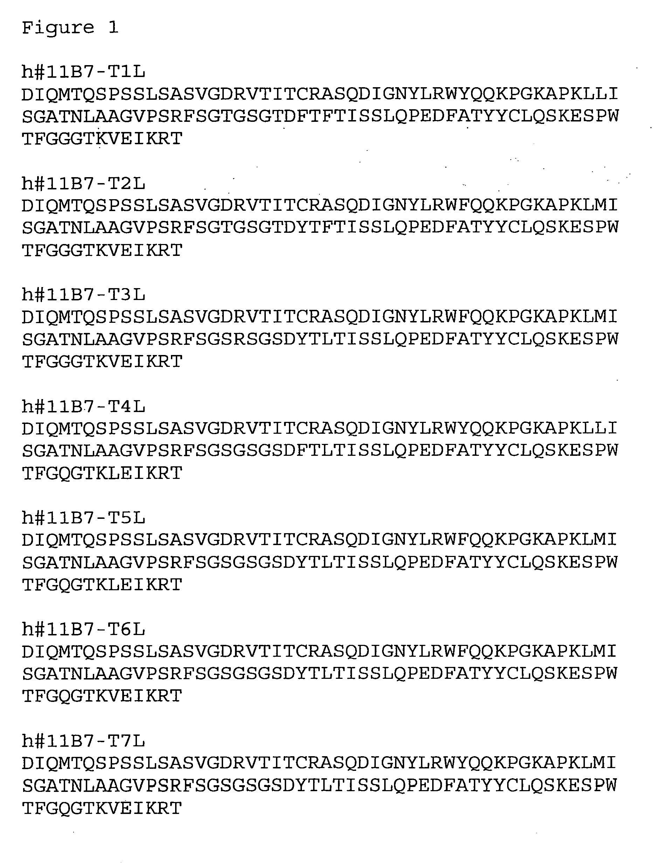

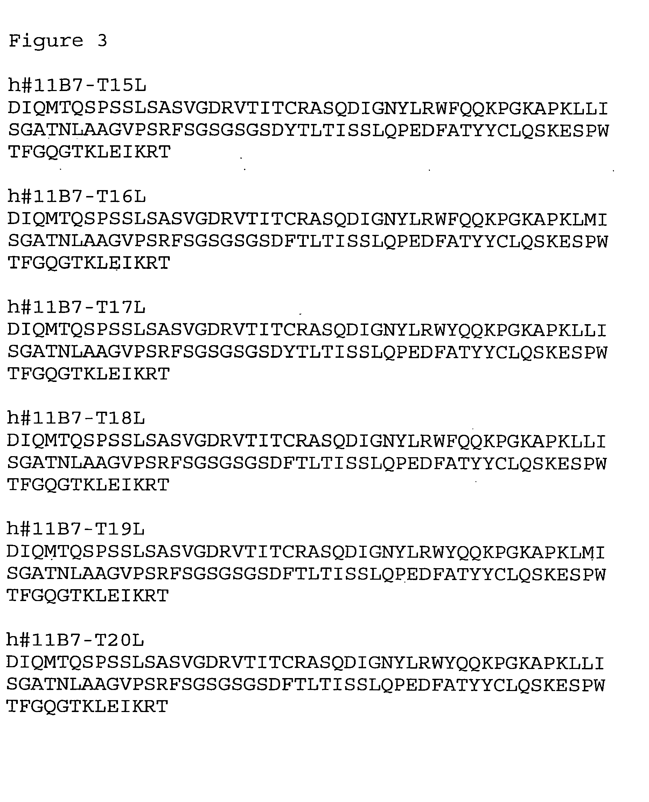

### 7.1.3 Humanization of #11B7 light chain (Figures 1, 2 and 3)

### 7.1.3.1 h#11 B7-T1 L-type light chain:

A humanized #11B7 light chain variable region designed by substituting amino acid Nos. 7 (alanine), 12 (proline), 15 (leucine), 36 (phenylalanine), 43 (serine), 45 (arginine), 47 (methionine), 65 (serine), 66 (arginine), 69 (serine), 71 (tyrosine), 72 (serine), 73 (leucine), 79 (glutamic acid), 80 (serine), 83 (methionine), 85 (aspartic acid), 104 (leucine), 106 (leucine), and 109 (proline) of #11B7 light chain variable region represented by SEQ ID NO: 140 of Sequence Listing (Figure 30B) by serine, serine, valine, tyrosine, alanine, lysine, leucine, threonine, glycine, threonine, phenylalanine, threonine, phenylalanine, glutamine, proline, phenylalanine, threonine, valine, isoleucine, and threonine, respectively, was designated as "h#11 B7-T1L"-type light chain variable region.

### 7.1.3.2 h#11B7-T2L-type light chain:

A humanized #11B7 light chain variable region designed by substituting amino acid Nos. 7 (alanine), 12 (proline), 15 (leucine), 43 (serine), 45 (arginine), 65 (serine), 66 (arginine), 69 (serine), 72 (serine), 73 (leucine), 79 (glutamic acid), 80 (serine), 83 (methionine), 85 (aspartic acid), 104 (leucine), 106 (leucine), and 109 (proline) of #11B7 light chain variable region represented by SEQ ID NO: 140 of Sequence Listing (Figure 30B) by serine, serine, valine, alanine, lysine, threonine, glycine, threonine, threonine, phenylalanine, glutamine, proline, phenylalanine, threonine, valine, isoleucine, and threonine, respectively, was designated as "h#11B7-T2L"-type light chain variable region.

### 7.1.3.3 h#11B7-T3L-type light chain:

A humanized #11B7 light chain variable region designed by substituting amino acid Nos. 7 (alanine), 12 (proline), 15 (leucine), 43 (serine), 45 (arginine), 72 (serine), 79 (glutamic acid), 80 (serine), 83 (methionine), 85 (aspartic acid), 104 (leucine), 106 (leucine), and 109 (proline) of #11B7 light chain variable region represented by SEQ ID NO: 140 of Sequence Listing (Figure 30B) by serine, serine, valine, alanine, lysine, threonine, glutamine, proline, phenylalanine, threonine, valine, isoleucine, and threonine, respectively, was designated as "h#11B7-T3L"-type light chain variable region.

### 7.1.3.4 h#11B7-T4L-type light chain:

A humanized #11 B7 light chain variable region designed by substituting amino acid Nos. 7 (alanine), 12 (proline), 15 (leucine), 36 (phenylalanine), 43 (serine), 45 (arginine), 47 (methionine), 66 (arginine), 71 (tyrosine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, tyrosine, alanine, lysine, leucine, glycine, phenylalanine, threonine, glutamine, proline, phenylalanine, threonine, glutamine, isoleucine, and threonine, respectively, was designated as "h#11B7-T4L"-type light chain variable region.

### 7.1.3.5 h#11B7-T5L-type light chain:

A humanized #11B7 light chain variable region designed by substituting amino acid Nos. 7 (alanine), 12 (proline), 15 (leucine), 43 (serine), 45 (arginine), 66 (arginine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, alanine, lysine, glycine, threonine, glutamine, proline, phenylalanine, threonine, glutamine, isoleucine, and threonine, respectively, was designated as "h#11B7-T5L"-type light chain variable region.

### 7.1.3.6 h#11B7-T6L-type light chain:

A humanized #11 B7 light chain variable region designed by substituting amino acid Nos. 7 (alanine), 12 (proline), 15 (leucine), 43 (serine), 45 (arginine), 66 (arginine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, alanine, lysine, glycine, threonine, glutamine, proline, phenylalanine, threonine, glutamine, valine, isoleucine, and threonine, respectively, was designated as "h#11B7-T6L"-type light chain variable region.

### 7.1.3.7 h#11B7-T7L-type light chain:

A humanized #11 B7 light chain variable region designed by substituting amino acid Nos. 7 (alanine), 12 (proline), 15 (leucine), 36 (phenylalanine), 43 (serine), 45 (arginine), 66 (arginine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, tyrosine, alanine, lysine, glycine, threonine, glutamine, proline, phenylalanine, threonine, glutamine, valine, isoleucine, and threonine, respectively, was designated as "h#11B7-T7L"-type light chain variable region.

### 7.1.3.8 h#11B7-T8L-type light chain:

A humanized #11 B7 light chain variable region designed by substituting amino acid Nos. 7 (alanine), 12 (proline), 15 (leucine), 43 (serine), 45 (arginine), 47 (methionine), 66 (arginine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, alanine, lysine, leucine, glycine, threonine, glutamine, proline, phenylalanine, threonine, glutamine, valine, isoleucine, and threonine, respectively, was designated as "h#11B7-T8L"-type light chain variable region.

### 7.1.3.9 h#11B7-T9L-type light chain:

A humanized #11B7 light chain variable region designed by substituting amino acid Nos. 7 (alanine), 12 (proline), 15 (leucine), 43 (serine), 45 (arginine), 66 (arginine), 71 (tyrosine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, alanine, lysine, glycine, phenylalanine, threonine, glutamine, proline, phenylalanine, threonine, glutamine, valine, isoleucine, and threonine, respectively, was designated as "h#11B7-T9L"-type light chain variable region.

### 7.1.3.10 h#11B7-T10L-type light chain:

A humanized #11 B7 light chain variable region designed by substituting amino acid Nos. 7 (alanine), 12 (proline), 15 (leucine), 36 (phenylalanine), 43 (serine), 45 (arginine), 47 (methionine), 66 (arginine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, tyrosine, alanine, lysine, leucine, glycine, threonine, glutamine, proline, phenylalanine, threonine, glutamine, valine, isoleucine, and threonine, respectively, was designated as "h#11B7-T10L"-type light chain variable region.

### 7.1.3.11 h#11B7-T11L-type light chain:

A humanized #11 B7 light chain variable region designed by substituting amino acid Nos. 7 (alanine), 12 (proline), 15 (leucine), 43 (serine), 45 (arginine), 47 (methionine), 66 (arginine), 71 (tyrosine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, alanine, lysine, leucine, glycine, phenylalanine, threonine, glutamine, proline, phenylalanine, threonine, glutamine, valine, isoleucine, and threonine, respectively, was designated as "h#11B7-T11 L"-type light chain variable region.

### 7.1.3.12 h#11B7-T12L-type light chain:

A humanized #11B7 light chain variable region designed by substituting amino acid Nos. 7 (alanine), 12 (proline), 15 (leucine), 36 (phenylalanine), 43 (serine), 45 (arginine), 66 (arginine), 71 (tyrosine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by in SEQ ID NO: 140 of Sequence Listing (Figure 30B) by serine, serine, valine, tyrosine, alanine, lysine, glycine, phenylalanine, threonine, glutamine, proline, phenylalanine, threonine, glutamine, valine, isoleucine, and threonine, respectively, was designated as "h#11B7-T12L"-type light chain variable region.

### 7.1.3.13 h#11B7-T13L-type light chain:

A humanized #11B7 light chain variable region designed by substituting amino acid Nos. 7 (alanine), 12 (proline), 15 (leucine), 36 (phenylalanine), 43 (serine), 45 (arginine), 47 (methionine), 66 (arginine), 71 (tyrosine), 72 (serine), 79 (glutamic acid), 80 (serine), 83 (methionine), 85 (aspartic acid), 100 (glycine), 104 (leucine), 106 (leucine), and 109 (proline) of #11B7 light chain variable region represented by SEQ ID NO: 140 of Sequence Listing (Figure 30B) by serine, serine, valine, tyrosine, alanine, lysine, leucine, glycine, phenylalanine, threonine, glutamine, proline, phenylalanine, threonine, glutamine, valine, isoleucine, and threonine, respectively, was designated as "h#11 B7-T13L"-type light chain variable region.

### 7.1.3.14 h#11B7-T14L-type light chain:

A humanized #11B7 light chain variable region designed by substituting amino acid Nos. 7 (alanine), 12 (proline), 15 (leucine), 36 (phenylalanine), 43 (serine), 45 (arginine), 66 (arginine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, tyrosine, alanine, lysine, glycine, threonine, glutamine, proline, phenylalanine, threonine, glutamine, isoleucine, and threonine, respectively, was designated as "h#11B7-T14L"-type light chain variable region.

### 7.1.3.15 h#11B7-T15L-type light chain:

A humanized #11B7 light chain variable region designed by substituting amino acid Nos. 7 (alanine), 12 (proline), 15 (leucine), 43 (serine), 45 (arginine), 47 (methionine), 66 (arginine), 72 (serine), 79 (glutamic acid), 80 (serine), 83 (methionine), 85 (aspartic acid), 100 (glycine), 106 (leucine), and 109 (proline) of #11 B7 light chain variable region represented by SEQ ID NO: 140 of Sequence Listing (Figure 30B) by serine, serine, valine, alanine, lysine, leucine, glycine, threonine, glutamine, proline, phenylalanine, threonine, glutamine, isoleucine, and threonine, respectively, was designated as "h#11B7-T15L"-type light chain variable region.

### 7.1.3.16 h#11B7-T16L-type light chain:

A humanized #11 B7 light chain variable region designed by substituting amino acid Nos. 7 (alanine), 12 (proline), 15 (leucine), 43 (serine), 45 (arginine), 66 (arginine), 71 (tyrosine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, alanine, lysine, glycine, phenylalanine, threonine, glutamine, proline, phenylalanine, threonine, glutamine, isoleucine, and threonine, respectively, was designated as "h#11B7-T16L"-type light chain variable region.

### 7.1.3.17 h#11B7-T17L-type light chain:

A humanized #11B7 light chain variable region designed by substituting amino acid Nos. 7 (alanine), 12 (proline), 15 (leucine), 36 (phenylalanine), 43 (serine), 45 (arginine), 47 (methionine), 66 (arginine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, tyrosine, alanine, lysine, leucine, glycine, threonine, glutamine, proline, phenylalanine, threonine, glutamine, isoleucine, and threonine, respectively, was designated as "h#11B7-T17L"-type light chain variable region.

### 7.1.3.18 h#11B7-T18L-type light chain:

A humanized #11B7 light chain variable region designed by substituting amino acid Nos. 7 (alanine), 12 (proline), 15 (leucine), 43 (serine), 45 (arginine), 47 (methionine), 66 (arginine), 71 (tyrosine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, alanine, lysine, leucine, glycine, phenylalanine, threonine, glutamine, proline, phenylalanine, threonine, glutamine, isoleucine, and threonine, respectively, was designated as "h#11B7-T18L"-type light chain variable region.

### 7.1.3.19 h#11B7-T19L-type light chain:

A humanized #11B7 light chain variable region designed by substituting amino acid Nos. 7 (alanine), 12 (proline), 15 (leucine), 36 (phenylalanine), 43 (serine), 45 (arginine), 66 (arginine), 71 (tyrosine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, tyrosine, alanine, lysine, glycine, phenylalanine, threonine, glutamine, proline, phenylalanine, threonine, glutamine, isoleucine, and threonine, respectively, was designated as "h#11B7-T19L"-type light chain variable region.

### 7.1.3.20 h#11B7-T20L-type light chain:

A humanized #11B7 light chain variable region designed by substituting amino acid Nos. 7 (alanine), 12 (proline), 15 (leucine), 36 (phenylalanine), 43 (serine), 45 (arginine), 47 (methionine), 66 (arginine), 71 (tyrosine), 72 (serine), 79 (glutamic acid), 80 (serine), 83 (methionine), 85 (aspartic acid), 100 (glycine), 106 (leucine), and 109 (proline) of #11B7 light chain variable region represented by SEQ ID NO: 140 of Sequence Listing (Figure 30B) by serine, serine, valine, tyrosine, alanine, lysine, leucine, glycine, phenylalanine, threonine, glutamine, proline, phenylalanine, threonine, glutamine, isoleucine, and threonine, respectively, was designated as "h#11B7-T20L"-type light chain variable region.

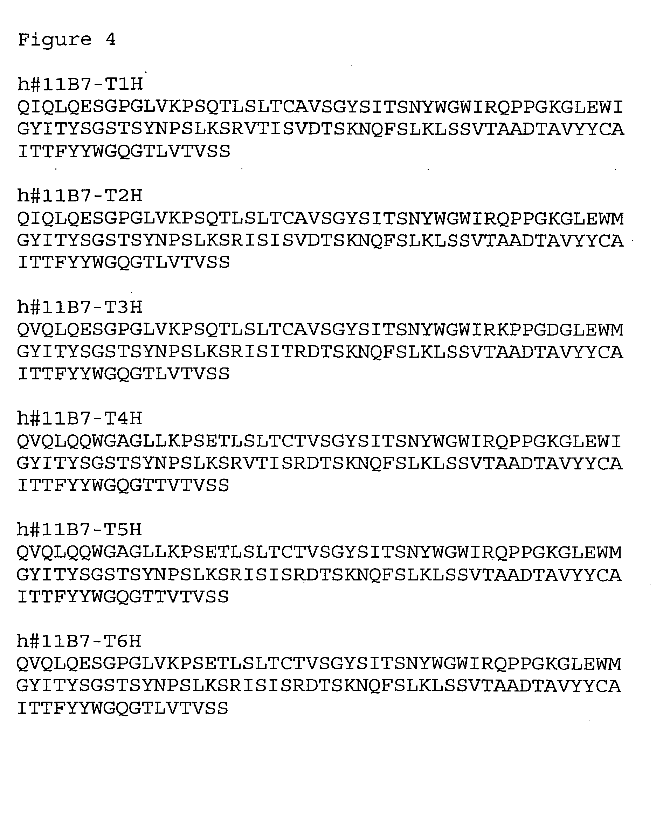

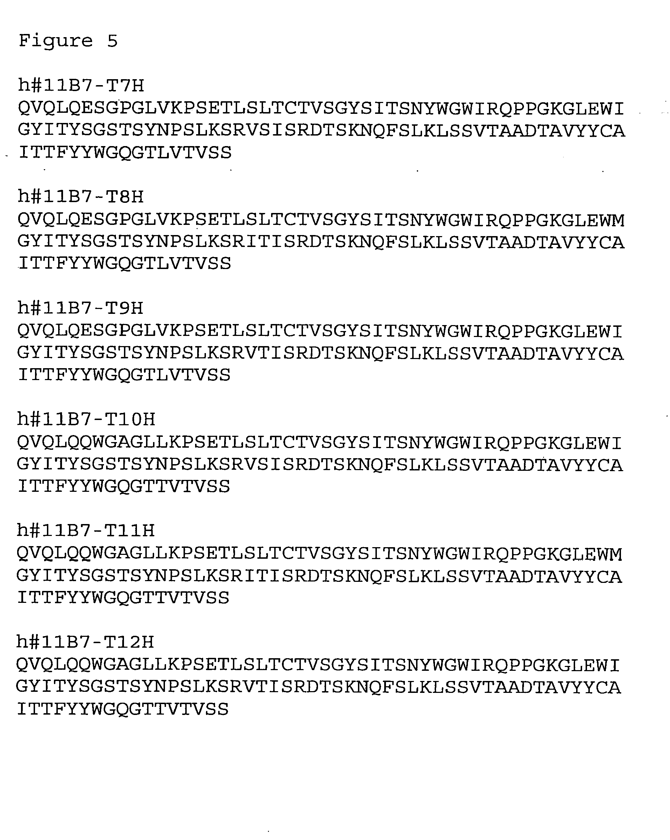

### 7.1.4 Humanization of #11B7 heavy chain (Figures 4 and 5)

### 7.1.4.1 h#11B7-T1H-type heavy chain:

A humanized #11B7 heavy chain variable region designed by substituting amino acid Nos. 1 (glutamic acid), 2 (valine), 17 (serine), 23 (serine), 25 (threonine), 39 (lysine), 40 (phenylalanine), 43 (aspartic acid), 44 (lysine), 45 (methionine), 48 (methionine), 67 (isoleucine), 68 (serine), 70 (threonine), 71 (arginine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, isoleucine, threonine, alanine, serine, glutamine, proline, lysine, glycine, leucine, isoleucine, valine, threonine, serine, valine, serine, lysine, serine, alanine, alanine, valine, threonine, and leucine, respectively, was designated as "h#11B7-T1H"-type heavy chain variable region.

### 7.1.4.2 h#11B7-T2H-type heavy chain:

A humanized #11B7 heavy chain variable region designed by substituting amino acid Nos. 1 (glutamic acid), 2 (valine), 17 (serine), 23 (serine), 25 (threonine), 39 (lysine), 40 (phenylalanine), 43 (aspartic acid), 44 (lysine), 45 (methionine), 70 (threonine), 71 (arginine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, isoleucine, threonine, alanine, serine, glutamine, proline, lysine, glycine, leucine, serine, valine, serine, lysine, serine, alanine, alanine, valine, threonine, and leucine, respectively, was designated as "h#11B7-T2H"-type heavy chain variable region.

### 7.1.4.3 h#11B7-T3H-type heavy chain:

A humanized #11B7 heavy chain variable region designed by substituting amino acid Nos. 1 (glutamic acid), 17 (serine), 23 (serine), 25 (threonine), 40 (phenylalanine), 44 (lysine), 45 (methionine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, threonine, alanine, serine, proline, glycine, leucine, serine, lysine, serine, alanine, alanine, valine, threonine, and leucine, respectively, was designated as "h#11B7-T3H"-type heavy chain variable region.

### 7.1.4.4 h#11B7-T4H-type heavy chain:

A humanized #11B7 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (aspartic acid), 44 (lysine), 45 (methionine), 48 (methionine), 67 (isoleucine), 68 (serine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamine, tryptophan, alanine, leucine, glutamic acid, threonine, threonine, serine, glutamine, proline, lysine, glycine, leucine, isoleucine, valine, threonine, serine, serine, lysine, serine, alanine, alanine, valine, threonine, and threonine, respectively, was designated as "h#11B7-T4H"-type heavy chain variable region.

### 7.1.4.5 h#11B7-T5H-type heavy chain:

A humanized #11B7 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (aspartic acid), 44 (lysine), 45 (methionine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamine, tryptophan, alanine, leucine, glutamic acid, threonine, threonine, serine, glutamine, proline, lysine, glycine, leucine, serine, serine, lysine, serine, alanine, alanine, valine, threonine, and threonine, respectively, was designated as "h#11B7-T5H"-type heavy chain variable region.

### 7.1.4.6 h#11B7-T6H-type heavy chain:

A humanized #11B7 heavy chain variable region designed by substituting amino acid Nos. 1 (glutamic acid), 16 (glutamine), 17 (serine), 23 (serine), 25 (threonine), 39 (lysine), 40 (phenylalanine), 43 (aspartic acid), 44 (lysine), 45 (methionine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamic acid, threonine, threonine, serine, glutamine, proline, lysine, glycine, leucine, serine, serine, lysine, serine, alanine, alanine, valine, threonine, and leucine, respectively, was designated as "h#11B7-T6H"-type heavy chain variable region.

### 7.1.4.7 h#11B7-T7H-type heavy chain:

A humanized #11B7 heavy chain variable region designed by substituting amino acid Nos. 1 (glutamic acid), 16 (glutamine), 17 (serine), 23 (serine), 25 (threonine), 39 (lysine), 40 (phenylalanine), 43 (aspartic acid), 44 (lysine), 45 (methionine), 48 (methionine), 67 (isoleucine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamic acid, threonine, threonine, serine, glutamine, proline, lysine, glycine, leucine, isoleucine, valine, serine, serine, lysine, serine, alanine, alanine, valine, threonine, and leucine, respectively, was designated as "h#11B7-T7H"-type heavy chain variable region.

### 7.1.4.8 h#11B7-T8H-type heavy chain:

A humanized #11B7 heavy chain variable region designed by substituting amino acid Nos. 1 (glutamic acid), 16 (glutamine), 17 (serine), 23 (serine), 25 (threonine), 39 (lysine), 40 (phenylalanine), 43 (aspartic acid), 44 (lysine), 45 (methionine), 68 (serine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamic acid, threonine, threonine, serine, glutamine, proline, lysine, glycine, leucine, threonine, serine, serine, lysine, serine, alanine, alanine, valine, threonine, and leucine, respectively, was designated as "h#11B7-T8H"-type heavy chain variable region.

### 7.1.4.9 h#11B7-T9H-type heavy chain:

A humanized #11B7 heavy chain variable region designed by substituting amino acid Nos. 1 (glutamic acid), 16 (glutamine), 17 (serine), 23 (serine), 25 (threonine), 39 (lysine), 40 (phenylalanine), 43 (aspartic acid), 44 (lysine), 45 (methionine), 48 (methionine), 67 (isoleucine), 68 (serine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamic acid, threonine, threonine, serine, glutamine, proline, lysine, glycine, leucine, isoleucine, valine, threonine, serine, serine, lysine, serine, alanine, alanine, valine, threonine, and leucine, respectively, was designated as "h#11B7-T9H"-type heavy chain variable region.

### 7.1.4.10 h#11B7-T10H-type heavy chain:

A humanized #11B7 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (aspartic acid), 44 (lysine), 45 (methionine), 48 (methionine), 67 (isoleucine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamine, tryptophan, alanine, leucine, glutamic acid, threonine, threonine, serine, glutamine, proline, lysine, glycine, leucine, isoleucine, valine, serine, serine, lysine, serine, alanine, alanine, valine, threonine, and threonine, respectively, was designated as "h#11B7-T10H"-type heavy chain variable region.

### 7.1.4.11 h#11B7-T11H-type heavy chain:

A humanized #11B7 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (aspartic acid), 44 (lysine), 45 (methionine), 68 (serine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamine, tryptophan, alanine, leucine, glutamic acid, threonine, threonine, serine, glutamine, proline, lysine, glycine, leucine, threonine, serine, serine, lysine, serine, alanine, alanine, valine, threonine, and threonine, respectively, was designated as "h#11B7-T11H"-type heavy chain variable region.

### 7.1.4.12 h#11B7-T12H-type heavy chain:

A humanized #11B7 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (aspartic acid), 44 (lysine), 45 (methionine), 48 (methionine), 67 (isoleucine), 68 (serine), 70 (threonine), 79 (phenylalanine), 81 (glutamine), 83 (asparagine), 87 (serine), 88 (glutamic acid), 92 (threonine), 107 (valine), and 108 (methionine) of #11B7 heavy chain variable region represented by SEQ ID NO: 141 of Sequence Listing (Figure 30C) by glutamine, glutamine, tryptophan, alanine, leucine, glutamic acid, threonine, threonine, serine, glutamine, proline, lysine, glycine, leucine, isoleucine, valine, threonine, serine, serine, lysine, serine, alanine, alanine, valine, threonine, and threonine, respectively, was designated as "h#11B7-T12H"-type heavy chain variable region.

### 7.2 Design of humanized version of #11D5

### 7.2.1 Molecular modeling of #11D5 variable domains

The molecular modeling of #11D5 variable domains was practiced according to the method generally known in the art as homology modeling (Methods in Enzymology, 203, 121-153, (1991)). The primary sequences (three-dimensional structures derived from X-ray crystal structures are available) of human immunoglobulin variable domains registered in Protein Data Bank (Nuc. Acid Res. 35, D301-D303 (2007)) were compared with the #11D5 variable domains thus determined. As a result, 1D5I was selected as having the highest sequence homology to the #11D5 light chain variable domain. Moreover, 1ORS was selected as having the highest sequence homology to the #11D5 heavy chain variable domain. The three-dimensional structures of framework domains were prepared based on a "framework model" obtained by combining the coordinates of 1D5I and 1ORS corresponding to the #11D5 light and heavy chains. For #11D5 CDRs, CDRL₁, CDRL₂, CDRL₃, CDRH₁, and CDRH₂ were assigned to clusters 11A, 7A, 9A, 10A, and 9A, respectively, according to the classification of Thornton et al. (J. Mol. Biol., 263, 800-815, (1996)). CDRH₃ was classified in k (3) - according to the H3-rules (FEBS letters 399, 1-8 (1996)). Subsequently, the typical conformation of each CDR was incorporated in the framework model.

Finally, to obtain possible molecular models of the #11D5 variable domains in terms of energy, energy calculation was conducted for excluding disadvantageous interatomic contact. These procedures were performed using a commercially available three-dimensional protein structure prediction program Prime and coordinate search program MacroModel (Schrödinger, LLC).

### 7.2.2 Design of amino acid sequence of humanized #11D5

Humanized #11D5 antibodies were constructed according to the method generally known in the art as CDR grafting (Proc. Natl. Acad. Sci. BSA 86, 10029-10033 (1989)). Acceptor antibodies were selected in two ways based on amino acid homology within the framework domains.

The sequences of the #11D5 framework domains were compared with those of all human frameworks registered in the Kabat Database (Nuc. Acid Res. 29, 205-206 (2001)) involving antibody amino acid sequences. As a result, a T33-4'CL antibody was selected as an acceptor due to 70% sequence homology between their framework domains. The amino acid residues of the framework domains in T33-4'CL were aligned with the corresponding amino acid residues in #11D5 to identify positions where different amino acids there between were used. The positions of these residues were analysed using the three-dimensional model of #11D5 thus constructed. Then, donor residues to be grafted on the acceptor were selected according to the criteria provided by Queen et al. (Proc. Natl. Acad. Sci. USA 86, 10029-10033 (1989)).

The sequences of the #11D5 framework domains were compared with those of all human frameworks registered in IgBLAST (Nuc. Acid Res. 36, D25-D30 (2007)). As a result, 1603260B was selected as an L chain acceptor due to 74% sequence homology between their framework domains. AAF80028 was selected as an H chain acceptor due to 66% sequence homology between their framework domains. The amino acid residues of the framework domains in the 1603260B L chain and in the AAF80028 H chain were aligned with the corresponding amino acid residues in #11D5 to identify positions where different amino acids therebetween were used. The positions of these residues were analysed using the three-dimensional model of #11D5 thus constructed. Then, donor residues to be grafted on the acceptor were selected according to the criteria provided by Queen et al. (Proc. Natl. Acad. Sci. USA 86, 10029-10033 (1989)).

In all methods, humanized #11D5 sequences were constructed as described in Examples below by transferring some selected donor residues to the acceptor antibodies.

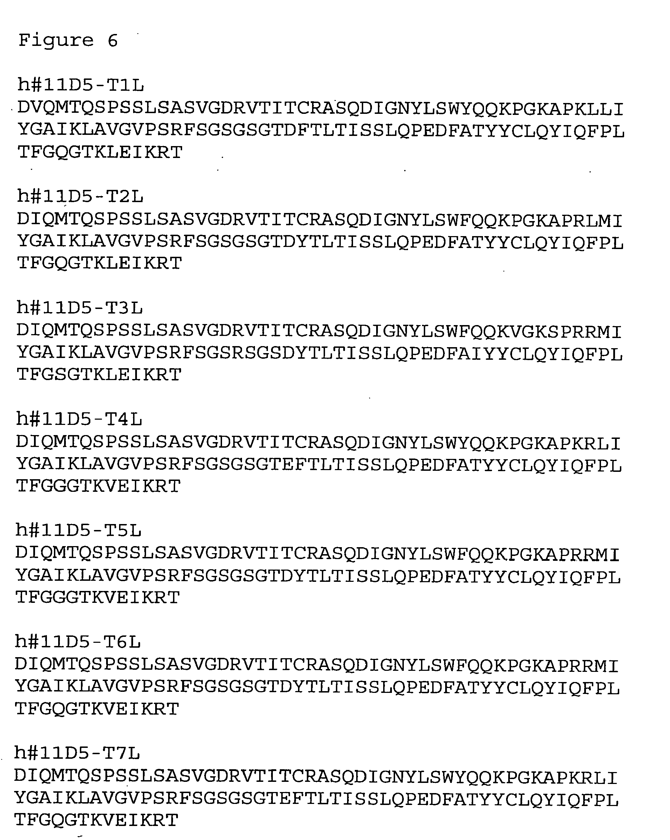

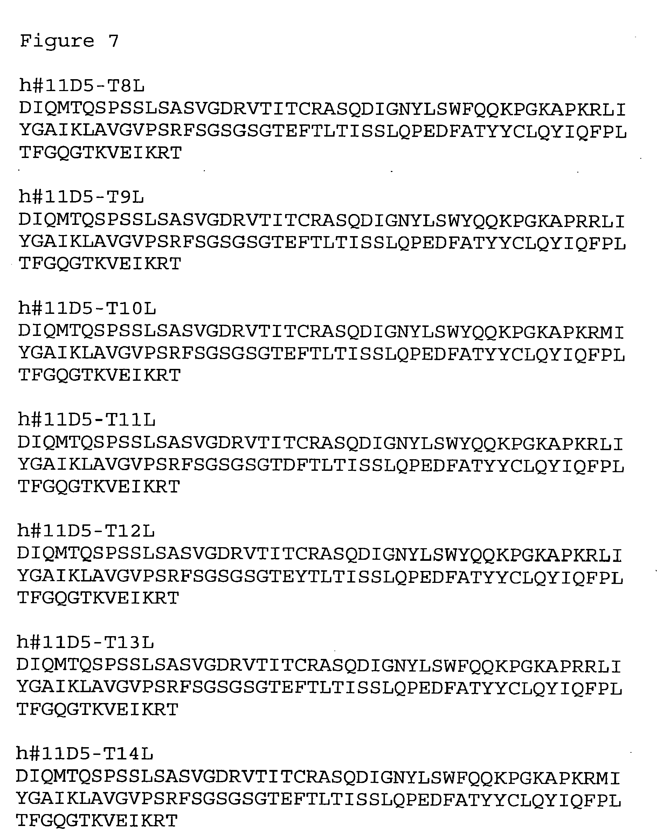

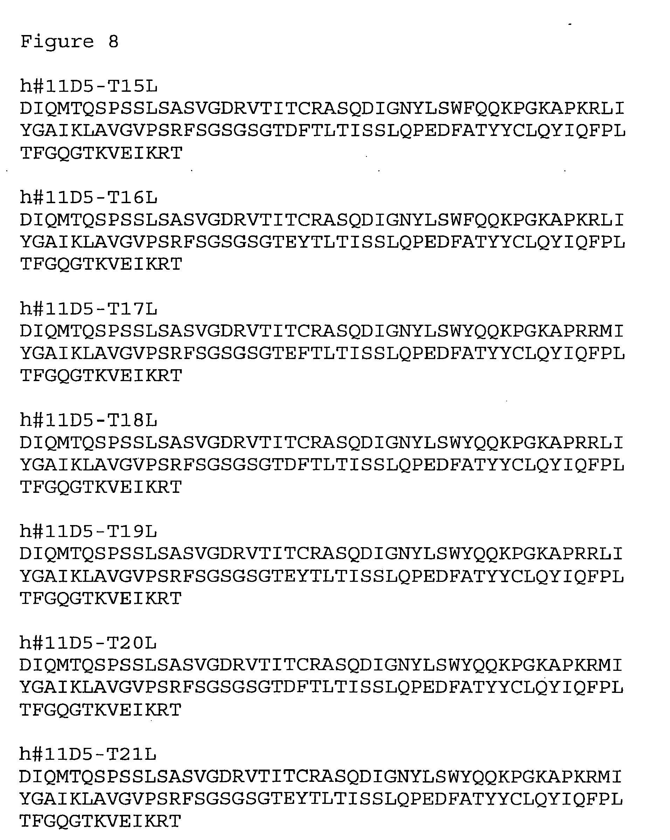

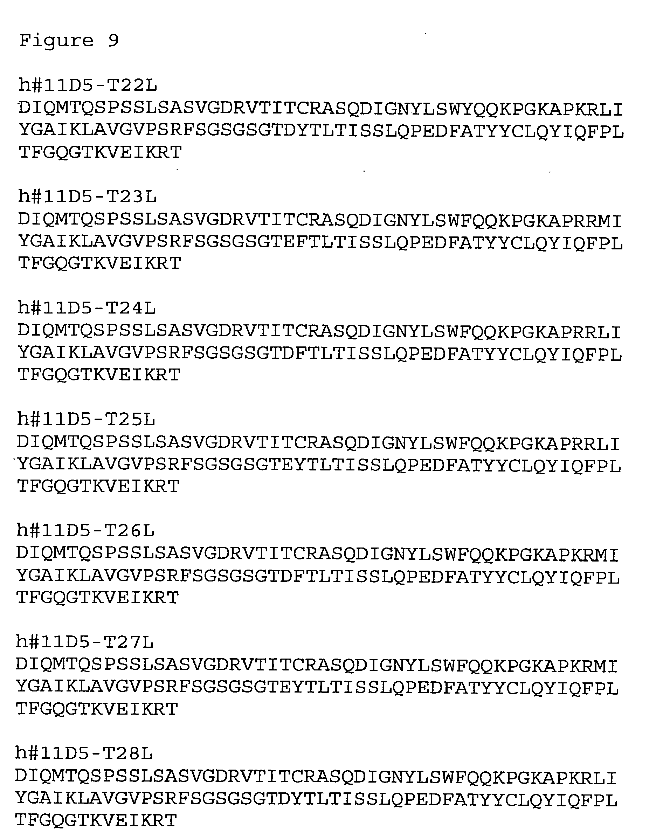

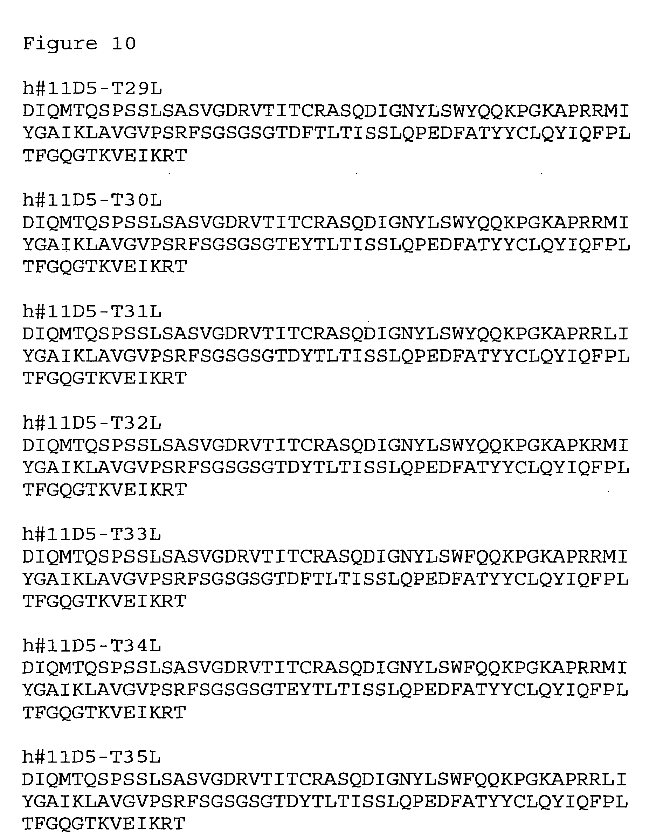

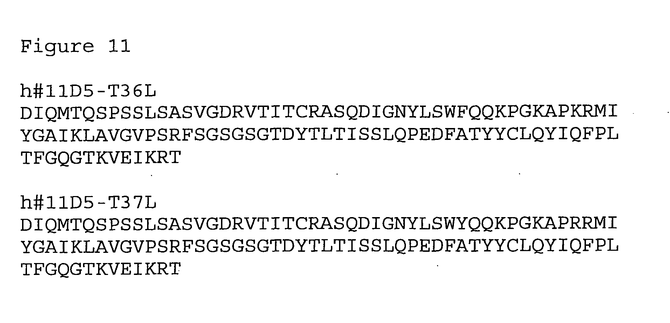

### 7.2.3 Humanization of #11D5 light chain (Figures 6, 7, 8, 9,10 and 11)

### 7.2.3.1 h#11D5-T1L-type light chain:

A humanized #11D5 light chain variable region designed by substituting amino acid Nos. 2 (isoleucine), 11 (methionine), 13 (threonine), 15 (leucine), 36 (phenylalanine), 40 (valine), 43 (serine), 45 (arginine), 46 (arginine), 47 (methionine), 66 (arginine), 69 (serine), 71 (tyrosine), 72 (serine), 79 (glutamic acid), 80 (serine), 83 (methionine), 85 (isoleucine), 100 (serine), 106 (leucine), and 109 (proline) of #11D5 light chain variable region represented by SEQ ID NO: 142 of Sequence Listing (Figure 30D) by valine, leucine, alanine, valine, tyrosine, proline, alanine, lysine, leucine, leucine, glycine, threonine, phenylalanine, threonine, glutamine, proline, phenylalanine, threonine, glutamine, isoleucine, and threonine, respectively, was designated as "h#11D5-T1L"-type light chain variable region.

### 7.2.3.2 h#11D5-T2L-type light chain:

A humanized #11D5 light chain variable region designed by substituting amino acid Nos. 11 (methionine), 13 (threonine), 15 (leucine), 40 (valine), 43 (serine), 46 (arginine), 66 (arginine), 69 (serine), 72 (serine), 79 (glutamic acid), 80 (serine), 83 (methionine), 85 (isoleucine), 100 (serine), 106 (leucine), and 109 (proline) of #11D5 light chain variable region represented by SEQ ID NO: 142 of Sequence Listing (Figure 30D) by leucine, alanine, valine, proline, alanine, leucine, glycine, threonine, threonine, glutamine, proline, phenylalanine, threonine, glutamine, isoleucine, and threonine, respectively, was designated as "h#11D5-T2L"-type light chain variable region.

### 7.2.3.3 h#11D5-T3L-type light chain:

A humanized #11D5 light chain variable region designed by substituting amino acid Nos. 11 (methionine), 13 (threonine), 15 (leucine), 72 (serine), 79 (glutamic acid), 80 (serine), 83 (methionine), 106 (leucine), and 109 (proline) of #11D5 light chain variable region represented by SEQ ID NO: 142 of Sequence Listing (Figure 30D) by leucine, alanine, valine, threonine, glutamine, proline, phenylalanine, isoleucine, and threonine, respectively, was designated as "h#11D5-T3L"-type light chain variable region.

### 7.2.3.4 h#11D5-T4L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 47 (methionine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, leucine, glycine, threonine, glutamic acid, phenylalanine, threonine, glutamine, proline, phenylalanine, threonine, glycine, valine, isoleucine, and threonine, respectively, was designated as "h#11D5-T4L"-type light chain variable region.

### 7.2.3.5 h#11D5-T5L-type light chain:

A humanized #11D5 light chain variable region designed by substituting amino acid Nos. 11 (methionine), 13 (threonine), 15 (leucine), 40 (valine), 43 (serine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, glycine, threonine, threonine, glutamine, proline, phenylalanine, threonine, glycine, valine, isoleucine, and threonine, respectively, was designated as "h#11D5-T5L"-type light chain variable region.

### 7.2.3.6 h#11D5-T6L-type light chain:

A humanized #11D5 light chain variable region designed by substituting amino acid Nos. 11 (methionine), 13 (threonine), 15 (leucine), 40 (valine), 43 (serine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, glycine, threonine, threonine, glutamine, proline, phenylalanine, threonine, glutamine, valine, isoleucine, and threonine, respectively, was designated as "h#11D5-T6L"-type light chain variable region.

### 7.2.3.7 h#11D5-T7L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 47 (methionine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, leucine, glycine, threonine, glutamic acid, phenylalanine, threonine, glutamine, proline, phenylalanine, threonine, glutamine, valine, isoleucine, and threonine, respectively, was designated as "h#11D5-T7L"-type light chain variable region.

### 7.2.3.8 h#11D5-T8L-type light chain:

A humanized #11D5 light chain variable region designed by substituting amino acid Nos. 11 (methionine), 13 (threonine), 15 (leucine), 40 (valine), 43 (serine), 45 (arginine), 47 (methionine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, leucine, glycine, threonine, glutamic acid, phenylalanine, threonine, glutamine, proline, phenylalanine, threonine, glutamine, valine, isoleucine, and threonine, respectively, was designated as "h#11D5-T8L"-type light chain variable region.

### 7.2.3.9 h#11D5-T9L-type light chain:

A humanized #11D5 light chain variable region designed by substituting amino acid Nos. 11 (methionine), 13 (threonine), 15 (leucine), 36 (phenylalanine), 40 (valine), 43 (serine), 47 (methionine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, leucine, glycine, threonine, glutamic acid, phenylalanine, threonine, glutamine, proline, phenylalanine, threonine, glutamine, valine, isoleucine, and threonine, respectively, was designated as "h#11D5-T9L"-type light chain variable region.

### 7.2.3.10 h#11D5-T10L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, glycine, threonine, glutamic acid, phenylalanine, threonine, glutamine, proline, phenylalanine, threonine, glutamine, valine, isoleucine, and threonine, respectively, was designated as "h#11D5-T10L"-type light chain variable region.

### 7.2.3.11 h#11D5-T11L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 47 (methionine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, leucine, glycine, threonine, phenylalanine, threonine, glutamine, proline, phenylalanine, threonine, glutamine, valine, isoleucine, and threonine, respectively, was designated as "h#11D5-T11 L"-type light chain variable region.

### 7.2.3.12 h#11D5-T12L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 47 (methionine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, leucine, glycine, threonine, glutamic acid, threonine, glutamine, proline, phenylalanine, threonine, glutamine, valine, isoleucine, and threonine, respectively, was designated as "h#11D5-T12L"-type light chain variable region.

### 7.2.3.13 h#11D5-T13L-type light chain:

A humanized #11D5 light chain variable region designed by substituting amino acid Nos. 11 (methionine), 13 (threonine), 15 (leucine), 40 (valine), 43 (serine), 47 (methionine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, leucine, glycine, threonine, glutamic acid, phenylalanine, threonine, glutamine, proline, phenylalanine, threonine, glutamine, valine, isoleucine, and threonine, respectively, was designated as "h#11D5-T13L"-type light chain variable region.

### 7.2.3.14 h#11D5-T14L-type light chain:

A humanized #11D5 light chain variable region designed by substituting amino acid Nos. 11 (methionine), 13 (threonine), 15 (leucine), 40 (valine), 43 (serine), 45 (arginine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, glycine, threonine, glutamic acid, phenylalanine, threonine, glutamine, proline, phenylalanine, threonine, glutamine, valine, isoleucine, and threonine, respectively, was designated as "h#11D5-T14L"-type light chain variable region.

### 7.2.3.15 h#11D5-T15L-type light chain:

A humanized #11D5 light chain variable region designed by substituting amino acid Nos. 11 (methionine), 13 (threonine), 15 (leucine), 40 (valine), 43 (serine), 45 (arginine), 47 (methionine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, leucine, glycine, threonine, phenylalanine, threonine, glutamine, proline, phenylalanine, threonine, glutamine, valine, isoleucine, and threonine, respectively, was designated as "h#11D5-T15L"-type light chain variable region.

### 7.2.3.16 h#11D5-T16L-type light chain:

A humanized #11D5 light chain variable region designed by substituting amino acid Nos. 11 (methionine), 13 (threonine), 15 (leucine), 40 (valine), 43 (serine), 45 (arginine), 47 (methionine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, leucine, glycine, threonine, glutamic acid, threonine, glutamine, proline, phenylalanine, threonine, glutamine, valine, isoleucine, and threonine, respectively, was designated as "h#11D5-T16L"-type light chain variable region.

### 7.2.3.17 h#11D5-T17L-type light chain:

A humanized #11D5 light chain variable region designed by substituting amino acid Nos. 11 (methionine), 13 (threonine), 15 (leucine), 36 (phenylalanine), 40 (valine), 43 (serine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, glycine, threonine, glutamic acid, phenylalanine, threonine, glutamine, proline, phenylalanine, threonine, glutamine, valine, isoleucine, and threonine, respectively, was designated as "h#11D5-T17L"-type light chain variable region.

### 7.2.3.18 h#11D5-T18L-type light chain:

A humanized #11D5 light chain variable region designed by substituting amino acid Nos. 11 (methionine), 13 (threonine), 15 (leucine), 36 (phenylalanine), 40 (valine), 43 (serine), 47 (methionine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, leucine, glycine, threonine, phenylalanine, threonine, glutamine, proline, phenylalanine, threonine, glutamine, valine, isoleucine, and threonine, respectively, was designated as "h#11D5-T18L"-type light chain variable region.

### 7.2.3.19 h#11D5-T19L-type light chain:

A humanized #11D5 light chain variable region designed by substituting amino acid Nos. 11 (methionine), 13 (threonine), 15 (leucine), 36 (phenylalanine), 40 (valine), 43 (serine), 47 (methionine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, leucine, glycine, threonine, glutamic acid, threonine, glutamine, proline, phenylalanine, threonine, glutamine, valine, isoleucine, and threonine, respectively, was designated as "h#11D5-T19L"-type light chain variable region.

### 7.2.3.20 h#11D5-T20L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, glycine, threonine, phenylalanine, threonine, glutamine, proline, phenylalanine, threonine, glutamine, valine, isoleucine, and threonine, respectively, was designated as "h#11D5-T20L"-type light chain variable region.

### 7.2.3.21 h#11D5-T21 L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, glycine, threonine, glutamic acid, threonine, glutamine, proline, phenylalanine, threonine, glutamine, valine, isoleucine, and threonine, respectively, was designated as "h#11D5-T21L"-type light chain variable region.

### 7.2.3.22 h#11D5-T22L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 47 (methionine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, leucine, glycine, threonine, threonine, glutamine, proline, phenylalanine, threonine, glutamine, valine, isoleucine, and threonine, respectively, was designated as "h#11D5-T22L"-type light chain variable region.

### 7.2.3.23 h#11D5-T23L-type light chain:

A humanized #11D5 light chain variable region designed by substituting amino acid Nos. 11 (methionine), 13 (threonine), 15 (leucine), 40 (valine), 43 (serine), 66 (arginine), 69 (serine), 70 (aspartic acid), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, glycine, threonine, glutamic acid, phenylalanine, threonine, glutamine, proline, phenylalanine, threonine, glutamine, valine, isoleucine, and threonine, respectively, was designated as "h#11D5-T23L"-type light chain variable region.

### 7.2.3.24 h#11D5-T24L-type light chain:

A humanized #11D5 light chain variable region designed by substituting amino acid Nos. 11 (methionine), 13 (threonine), 15 (leucine), 40 (valine), 43 (serine), 47 (methionine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, leucine, glycine, threonine, phenylalanine, threonine, glutamine, proline, phenylalanine, threonine, glutamine, valine, isoleucine, and threonine, respectively, was designated as "h#11D5-T24L"-type light chain variable region.

### 7.2.3.25 h#11D5-T25L-type light chain:

A humanized #11D5 light chain variable region designed by substituting amino acid Nos. 11 (methionine), 13 (threonine), 15 (leucine), 40 (valine), 43 (serine), 47 (methionine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, leucine, glycine, threonine, glutamic acid, threonine, glutamine, proline, phenylalanine, threonine, glutamine, valine, isoleucine, and threonine, respectively, was designated as "h#11D5-T25L"-type light chain variable region.

### 7.2.3.26 h#11D5-T26L-type light chain:

A humanized #11D5 light chain variable region designed by substituting amino acid Nos. 11 (methionine), 13 (threonine), 15 (leucine), 40 (valine), 43 (serine), 45 (arginine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, glycine, threonine, phenylalanine, threonine, glutamine, proline, phenylalanine, threonine, glutamine, valine, isoleucine, and threonine, respectively, was designated as "h#11D5-T26L"-type light chain variable region.

### 7.2.3.27 h#11D5-T27L-type light chain:

A humanized #11D5 light chain variable region designed by substituting amino acid Nos. 11 (methionine), 13 (threonine), 15 (leucine), 40 (valine), 43 (serine), 45 (arginine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, glycine, threonine, glutamic acid, threonine, glutamine, proline, phenylalanine, threonine, glutamine, valine, isoleucine, and threonine, respectively, was designated as "h#11D5-T27L"-type light chain variable region.

### 7.2.3.28 h#11D5-T28L-type light chain:

A humanized #11D5 light chain variable region designed by substituting amino acid Nos. 11 (methionine), 13 (threonine), 15 (leucine), 40 (valine), 43 (serine), 45 (arginine), 47 (methionine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, leucine, glycine, threonine, threonine, glutamine, proline, phenylalanine, threonine, glutamine, valine, isoleucine, and threonine, respectively, was designated as "h#11D5-T28L"-type light chain variable region.

### 7.2.3.29 h#11D5-T29L-type light chain:

A humanized #11D5 light chain variable region designed by substituting amino acid Nos. 11 (methionine), 13 (threonine), 15 (leucine), 36 (phenylalanine), 40 (valine), 43 (serine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, glycine, threonine, phenylalanine, threonine, glutamine, proline, phenylalanine, threonine, glutamine, valine, isoleucine, and threonine, respectively, was designated as "h#11D5-T29L"-type light chain variable region.

### 7.2.3.30 h#11D5-T30L-type light chain:

A humanized #11D5 light chain variable region designed by substituting amino acid Nos. 11 (methionine), 13 (threonine), 15 (leucine), 36 (phenylalanine), 40 (valine), 43 (serine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, glycine, threonine, glutamic acid, threonine, glutamine, proline, phenylalanine, threonine, glutamine, valine, isoleucine, and threonine, respectively, was designated as "h#11D5-T30L"-type light chain variable region.

### 7.2.3.31 h#11D5-T31 L-type light chain:

A humanized #11D5 light chain variable region designed by substituting amino acid Nos. 11 (methionine), 13 (threonine), 15 (leucine), 36 (phenylalanine), 40 (valine), 43 (serine), 47 (methionine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, leucine, glycine, threonine, threonine, glutamine, proline, phenylalanine, threonine, glutamine, valine, isoleucine, and threonine, respectively, was designated as "h#11D5-T31L"-type light chain variable region.

### 7.2.3.32 h#11D5-T32L-type light chain:

A humanized #11D5 light chain variable region designed by substituting amino acid Nos. 11 (methionine), 13 (threonine), 15 (leucine), 36 (phenylalanine), 40 (valine), 43 (serine), 45 (arginine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, lysine, glycine, threonine, threonine, glutamine, proline, phenylalanine, threonine, glutamine, valine, isoleucine, and threonine, respectively, was designated as "h#11D5-T32L"-type light chain variable region.

### 7.2.3.33 h#11D5-T33L-type light chain:

A humanized #11D5 light chain variable region designed by substituting amino acid Nos. 11 (methionine), 13 (threonine), 15 (leucine), 40 (valine), 43 (serine), 66 (arginine), 69 (serine), 71 (tyrosine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, glycine, threonine, phenylalanine, threonine, glutamine, proline, phenylalanine, threonine, glutamine, valine, isoleucine, and threonine, respectively, was designated as "h#11D5-T33L"-type light chain variable region.

### 7.2.3.34 h#11D5-T34L-type light chain:

A humanized #11D5 light chain variable region designed by substituting amino acid Nos. 11 (methionine), 13 (threonine), 15 (leucine), 40 (valine), 43 (serine), 66 (arginine), 69 (serine), 70 (aspartic acid), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, glycine, threonine, glutamic acid, threonine, glutamine, proline, phenylalanine, threonine, glutamine, valine, isoleucine, and threonine, respectively, was designated as "h#11D5-T34L"-type light chain variable region.

### 7.2.3.35 h#11D5-T35L-type light chain:

A humanized #11D5 light chain variable region designed by substituting amino acid Nos. 11 (methionine), 13 (threonine), 15 (leucine), 40 (valine), 43 (serine), 47 (methionine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, leucine, glycine, threonine, threonine, glutamine, proline, phenylalanine, threonine, glutamine, valine, isoleucine, and threonine, respectively, was designated as "h#11D5-T35L"-type light chain variable region.

### 7.2.3.36 h#11D5-T36L-type light chain:

A humanized #11D5 light chain variable region designed by substituting amino acid Nos. 11 (methionine), 13 (threonine), 15 (leucine), 40 (valine), 43 (serine), 45 (arginine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, proline, alanine, lysine, glycine, threonine, threonine, glutamine, proline, phenylalanine, threonine, glutamine, valine, isoleucine, and threonine, respectively, was designated as "h#11D5-T36L"-type light chain variable region.

### 7.2.3.37 h#11D5-T37L-type light chain:

A humanized #11D5 light chain variable region designed by substituting amino acid Nos. 11 (methionine), 13 (threonine), 15 (leucine), 36 (phenylalanine), 40 (valine), 43 (serine), 66 (arginine), 69 (serine), 72 (serine), 79 (glutamic acid), 80 (serine), 83 (methionine), 85 (isoleucine), 100 (serine), 104 (leucine), 106 (leucine), and 109 (proline) of #11D5 light chain variable region represented by SEQ ID NO: 142 of Sequence Listing (Figure 30D) by leucine, alanine, valine, tyrosine, proline, alanine, glycine, threonine, threonine, glutamine, proline, phenylalanine, threonine, glutamine, valine, isoleucine, and threonine, respectively, was designated as "h#11D5-T37L"-type light chain variable region.

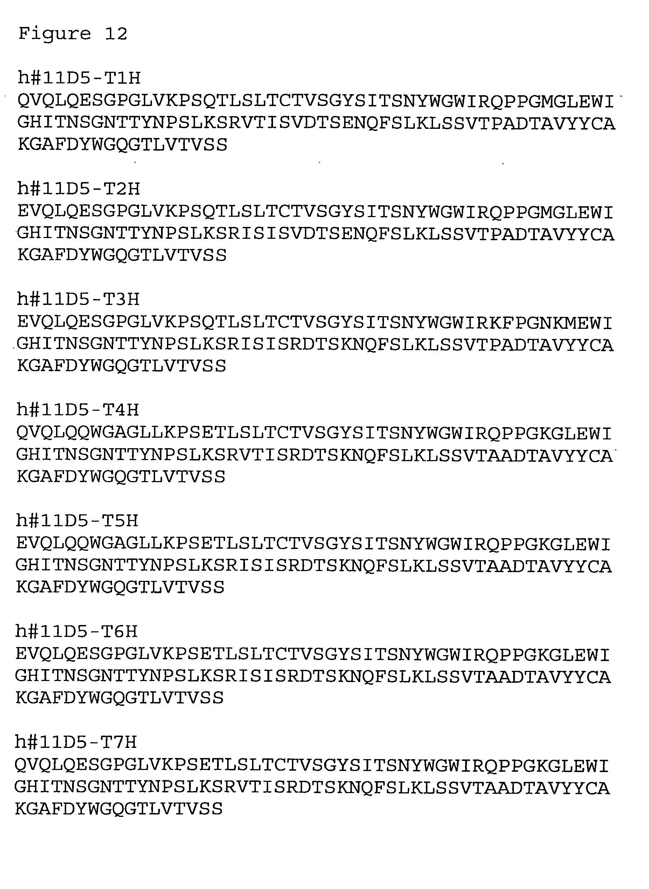

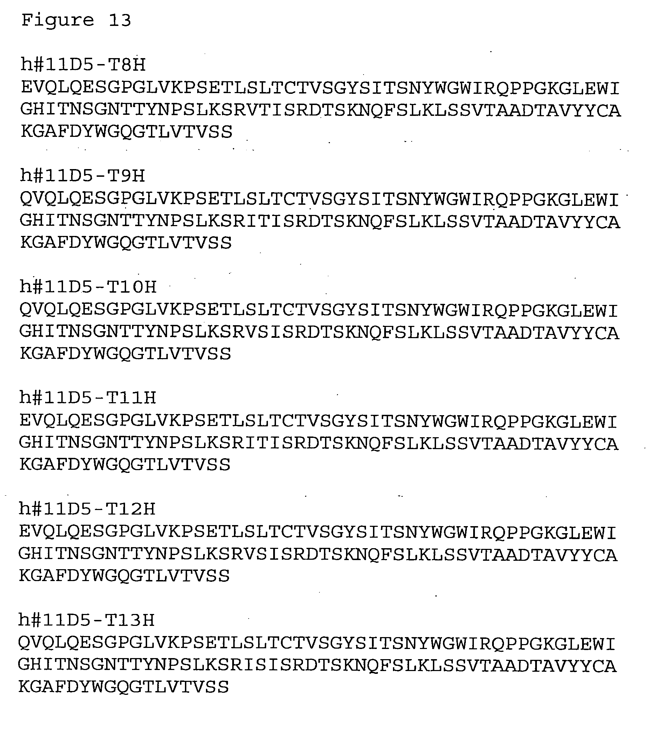

### 7.2.4 Humanization of #11D5 heavy chain (Figures 12 and 13)

### 7.2.4.1 h#11D5-T1H-type heavy chain:

A humanized #11D5 heavy chain variable region designed by substituting amino acid Nos. 1 (glutamic acid), 17 (serine), 23 (serine), 25 (threonine), 39 (lysine), 40 (phenylalanine), 43 (asparagine), 44 (lysine), 45 (methionine), 67 (isoleucine), 68 (serine), 71 (arginine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, threonine, threonine, serine, glutamine, proline, methionine, glycine, leucine, valine, threonine, valine, glutamic acid, serine, lysine, serine, proline, alanine, valine, threonine, and leucine, respectively, was designated as "h#11D5-T1H"-type heavy chain variable region.

### 7.2.4.2 h#11D5-T2H-type heavy chain:

A humanized #11D5 heavy chain variable region designed by substituting amino acid Nos. 17 (serine), 23 (serine), 25 (threonine), 39 (lysine), 40 (phenylalanine), 43 (asparagine), 44 (lysine), 45 (methionine), 71 (arginine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by threonine, threonine, serine, glutamine, proline, methionine, glycine, leucine, valine, glutamic acid, serine, lysine, serine, proline, alanine, valine, threonine, and leucine, respectively, was designated as "h#11D5-T2H"-type heavy chain variable region.

### 7.2.4.3 h#11D5-T3H-type heavy chain:

A humanized #11D5 heavy chain variable region designed by substituting amino acid Nos. 17 (serine), 23 (serine), 25 (threonine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by threonine, threonine, serine, lysine, serine, lysine, serine, proline, alanine, valine, threonine, and leucine, respectively, was designated as "h#11D5-T3H"-type heavy chain variable region.

### 7.2.4.4 h#11D5-T4H-type heavy chain:

A humanized #11D5 heavy chain variable region designed by substituting amino acid Nos. 1 (glutamic acid), 6 (glutamic acid), 7 (serine), 9 (proline), 12 (valine), 16 (glutamine), 17 (serine), 23 (serine), 25 (threonine), 39 (lysine), 40 (phenylalanine), 43 (asparagine), 44 (lysine), 45 (methionine), 67 (isoleucine), 68 (serine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, glutamine, tryptophan, alanine, leucine, glutamic acid, threonine, threonine, serine, glutamine, proline, lysine, glycine, leucine, valine, threonine, lysine, serine, lysine, serine, alanine, alanine, valine, threonine, and leucine, respectively, was designated as "h#11D5-T4H"-type heavy chain variable region.

### 7.2.4.5 h#11D5-T5H-type heavy chain:

A humanized #11D5 heavy chain variable region designed by substituting amino acid Nos. 6 (glutamic acid), 7 (serine), 9 (proline), 12 (valine), 16 (glutamine), 17 (serine), 23 (serine), 25 (threonine), 39 (lysine), 40 (phenylalanine), 43 (asparagine), 44 (lysine), 45 (methionine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, tryptophan, alanine, leucine, glutamic acid, threonine, threonine, serine, glutamine, proline, lysine, glycine, leucine, lysine, serine, lysine, serine, alanine, alanine, valine, threonine, and leucine, respectively, was designated as "h#11D5-T5H"-type heavy chain variable region.

### 7.2.4.6 h#11D5-T6H-type heavy chain:

A humanized #11D5 heavy chain variable region designed by substituting amino acid Nos. 16 (glutamine), 17 (serine), 23 (serine), 25 (threonine), 39 (lysine), 40 (phenylalanine), 43 (asparagine), 44 (lysine), 45 (methionine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamic acid, threonine, threonine, serine, glutamine, proline, lysine, glycine, leucine, lysine, serine, lysine, serine, alanine, alanine, valine, threonine, and leucine, respectively, was designated as "h#11D5-T6H"-type heavy chain variable region.

### 7.2.4.7 h#11D5-T7H-type heavy chain:

A humanized #11D5 heavy chain variable region designed by substituting amino acid Nos. 1 (glutamic acid), 16 (glutamine), 17 (serine), 23 (serine), 25 (threonine), 39 (lysine), 40 (phenylalanine), 43 (asparagine), 44 (lysine), 45 (methionine), 67 (isoleucine), 68 (serine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID, NO: 143 of Sequence Listing (Figure 30E) by glutamine, glutamic acid, threonine, threonine, serine, glutamine, proline, lysine, glycine, leucine, valine, threonine, lysine, serine, lysine, serine, alanine, alanine, valine, threonine, and leucine, respectively, was designated as "h#11D5-T7H"-type heavy chain variable region.

### 7.2.4.8 h#11D5-T8H-type heavy chain:

A humanized #11D5 heavy chain variable region designed by substituting amino acid Nos. 16 (glutamine), 17 (serine), 23 (serine), 25 (threonine), 39 (lysine), 40 (phenylalanine), 43 (asparagine), 44 (lysine), 45 (methionine), 67 (isoleucine), 68 (serine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamic acid, threonine, threonine, serine, glutamine, proline, lysine, glycine, leucine, valine, threonine, lysine, serine, lysine, serine, alanine, alanine, valine, threonine, and leucine, respectively, was designated as "h#11D5-T8H"-type heavy chain variable region.

### 7.2.4.9 h#11D5-T9H-type heavy chain:

A humanized #11D5 heavy chain variable region designed by substituting amino acid Nos. 1 (glutamic acid), 16 (glutamine), 17 (serine), 23 (serine), 25 (threonine), 39 (lysine), 40 (phenylalanine), 43 (asparagine), 44 (lysine), 45 (methionine), 68 (serine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, glutamic acid, threonine, threonine, serine, glutamine, proline, lysine, glycine, leucine, threonine, lysine, serine, lysine, serine, alanine, alanine, valine, threonine, and leucine, respectively, was designated as "h#11D5-T9H"-type heavy chain variable region.

### 7.2.4.10 h#11D5-T10H-type heavy chain:

A humanized #11D5 heavy chain variable region designed by substituting amino acid Nos. 1 (glutamic acid), 16 (glutamine), 17 (serine), 23 (serine), 25 (threonine), 39 (lysine), 40 (phenylalanine), 43 (asparagine), 44 (lysine), 45 (methionine), 67 (isoleucine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, glutamic acid, threonine, threonine, serine, glutamine, proline, lysine, glycine, leucine, valine, lysine, serine, lysine, serine, alanine, alanine, valine, threonine, and leucine, respectively, was designated as "h#11D5-T10H"-type heavy chain variable region.

### 7.2.4.11 h#11D5-T11H-type heavy chain:

A humanized #11D5 heavy chain variable region designed by substituting amino acid Nos. 16 (glutamine), 17 (serine), 23 (serine), 25 (threonine), 39 (lysine), 40 (phenylalanine), 43 (asparagine), 44 (lysine), 45 (methionine), 68 (serine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamic acid, threonine, threonine, serine, glutamine, proline, lysine, glycine, leucine, threonine, lysine, serine, lysine, serine, alanine, alanine, valine, threonine, and leucine, respectively, was designated as "h#11D5-T11H"-type heavy chain variable region.

### 7.2.4.12 h#11D5-T12H-type heavy chain:

A humanized #11D5 heavy chain variable region designed by substituting amino acid Nos. 16 (glutamine), 17 (serine), 23 (serine), 25 (threonine), 39 (lysine), 40 (phenylalanine), 43 (asparagine), 44 (lysine), 45 (methionine), 67 (isoleucine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamic acid, threonine, threonine, serine, glutamine, proline, lysine, glycine, leucine, valine, lysine, serine, lysine, serine, alanine, alanine, valine, threonine, and leucine, respectively, was designated as "h#11D5-T12H"-type heavy chain variable region.

### 7.2.4.13 h#11D5-T13H-type heavy chain:

A humanized #11D5 heavy chain variable region designed by substituting amino acid Nos. 1 (glutamic acid), 16 (glutamine), 17 (serine), 23 (serine), 25 (threonine), 39 (lysine), 40 (phenylalanine), 43 (asparagine), 44 (lysine), 45 (methionine), 75 (arginine), 79 (phenylalanine), 81 (glutamine), 83 (asparagine), 87 (threonine), 88 (glutamic acid), 92 (threonine), 107 (valine), and 108 (methionine) of #11D5 heavy chain variable region represented by SEQ ID NO: 143 of Sequence Listing (Figure 30E) by glutamine, glutamic acid, threonine, threonine, serine, glutamine, proline, lysine, glycine, leucine, lysine, serine, lysine, serine, alanine, alanine, valine, threonine, and leucine, respectively, was designated as "h#11D5-T13H"-type heavy chain variable region.

### Example 8: Construction of general-purpose vectors for humanized antibody expression

### 8.1 Construction of a vector, pEF6KCL, for humanized antibody light chain expression.

PCR was performed using a plasmid pEF6/V5-His B (Invitrogen Corp.) as a template and the following primers to obtain a DNA fragment from immediately following BGHpA (2174) to Smal (2958) (DNA fragment containing the f1 origin of replication, and the SV40 promotor and origin; hereinafter, referred to as a "fragment A"):
5'-ccacgcgccctgtagcggcgcattaagc-3' (primer EFF1: SEQ ID NO:1), and
5'-aaacccgggagctttttgcaaaagcctagg-3' (primer EFsmaR: SEQ ID NO:2).

The obtained fragment A was ligated by overlap PCR to a DNA fragment comprising a DNA sequence encoding a human K-chain secretion signal, a human K-chain constant region, and a human poly(A) addition signal (SEQ ID NO:3; hereinafter, referred to as a "fragment B"). The obtained DNA fragment in which the fragment A is ligated to fragment B (hereinafter, referred to as a "fragment A+B") was digested with restriction enzymes KpnI and Smal and ligated to a plasmid pEF6/V5-His B (Invitrogen Corp.) digested in advance with restriction enzymes KpnI and SmaI to construct a human L chain expression plasmid "pEF6KCL" (Fig. 19) having the signal sequence, the cloning site, the human K-chain constant region-encoding sequence, and the human poly(A) addition signal sequence downstream of the EF1 promoter.

### 8.2 Construction of a vector, pEF1/FCCU-1, for humanized antibody heavy chain expression

### 8.2.1 Construction of pEF1/KCL

A plasmid pEF6KCL obtained by the method described above was digested with restriction enzymes KpnI and SmaI. The resulting DNA fragment was ligated to pEF1/myc-His B (Invitrogen Corp.) digested in advance with Kpnl and Smal to construct a plasmid pEF1/KCL.

### 8.2.2 Construction of pEF1/FCCU-1

A DNA fragment comprising a DNA sequence encoding the amino acid sequence of the signal sequence and constant region of human IgG1 (SEQ ID NO: 4) was digested with restriction enzymes NheI and PmeI and ligated to the plasmid pEF1/KCL digested in advance with NheI and PmeI to construct a human heavy chain expression plasmid pEF1/FCCU-1 having the signal sequence, the cloning site, the human heavy chain constant region-encoding sequence, and the human poly(A) addition signal sequence downstream of the EF1 promoter.

### Example 9: Production of humanized antibody gene of rat anti-human AXL monoclonal antibody #11B7

### a) Construction of h#11B7-T1L, h#11B7-T2L, h#11B7-T3L, h#11B7-T4L, h#11B7-T5L, h#11B7-T6L, h#11B7-T7L, h#11B7-T8L, h#11B7-T9L, h#11B7-T10L, h#11B7-T11L, h#11B7-T12L, and h#11B7-T13L, light chain expression vectors

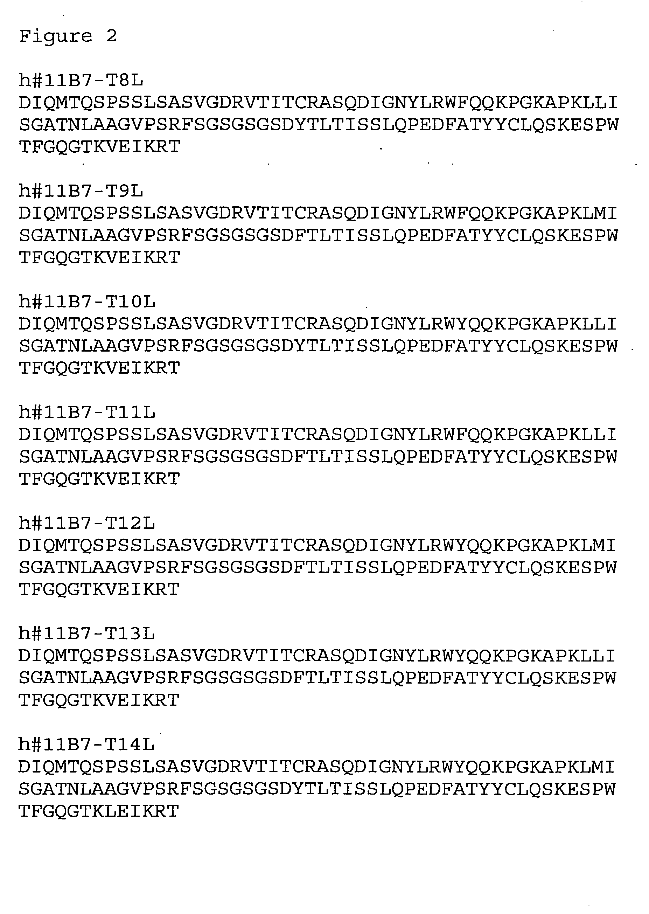

Each DNA containing a gene encoding a h#11B7-T1L, h#11B7-T2L, h#11B7-T3L, h#11B7-T4L, h#11B7-T5L, h#11B7-T6L, h#11B7-T7L, h#11B7-T8L, h#11B7-T9L, h#11B7-T10L, h#11B7-T11L, h#11B7-T12L,or h#11B7-T13L light chain variable region (Figures 1 and 2) represented by amino acid Nos. 21 to 129 of SEQ ID NO:18; 19, 20, 21, 22, 23, 24, 25, 26, 27, 28, 29, or 30, respectively, of the Sequence Listing, fused with a secretion signal was synthesized (MBL Medical & Biological Laboratories Co., Ltd, Artificial Gene Synthesis Service) and digested with restriction enzymes Nhel and BsiWI. The resulting DNA fragments were separately inserted into sites of general-purpose vectors for humanized antibody light chain expression (pEF6KCL) digested in advance with restriction enzymes NheI and BsiWI to thereby construct h#11B7-T1L, h#11B7-T2L, h#11B7-T3L, h#11B7-T4L, h#11B7-T5L, h#11B7-T6L, h#11B7-T7L, h#11B7-TBL, h#11B7-T9L, h#11B7-T10L, h#11B7-T11L, h#11B7-T12L,and h#11B7-T13L light chain expression vectors. The obtained expression vectors were designated as "pEF6KCL/h#11B7-T1L", "pEF6KCL/h#11B7-T2L", "pEF6KCL/h#11B7-T3L", "pEF6KCL/h#11B7-T4L", "pEF6KCL/h#11B7-T5L", "pEF6KCL/h#11B7-T6L", "pEF6KCL/h#11B7-T7L", "pEF6KCL/h#11B7-T8L", "pEF6KCL/h#11B7-T9L", "pEF6KCL/h#11B7-T10L", "pEF6KCL/h#11B7-T11 L", "pEF6KCL/h#11B7-T12L", or "pEF6KCL/h#11B7-T13L", and the insert of each expression vector is represented by the nucleotide sequence of SEQ ID NO: 5, 6, 7, 8, 9, 10, 11, 12, 13, 14, 15, 16, or 17 of the Sequence Listing, respectively.

### b) Construction of h#11B7-T1H, h#11B7-T2H, h#11B7-T3H, h#11B7-T4H, h#11B7-T5H, h#11B7-T6H, h#11B7-T7H, h#11B7-T8H,and h#11B7-T9H heavy chain expression vectors

Each DNA containing a gene encoding a h#11B7-T1H, h#11B7-T2H, h#11B7-T3H, h#11B7-T4H, h#11B7-T5H, h#11B7-T6H, h#11B7-T7H, h#11B7-T8H, or h#11B7-T9H heavy chain variable region (Figures 4 and 5) represented by amino acid Nos. 20 to 132 of SEQ ID NO:40, 41, 42, 43, 44, 45, 46, 47, or 48, respectively, of the Sequence Listing was synthesized (MBL Medical & Biological Laboratories Co., Ltd, Artificial Gene Synthesis Service) and digested with a restriction enzyme Blpl. The resulting DNA fragments were separately inserted into sites of general-purpose vectors for humanized antibody heavy chain expression (pEF1/FCCU-1) digested in advance with a restriction enzyme Blpl to thereby construct h#11B7-T1H, h#11B7-T2H, h#11B7-T3H, h#11B7-T4H, h#11B7-T5H, h#11B7-T6H, h#11B7-T7H, h#11B7-T8H,and h#11B7-T9H heavy chain expression vectors. The obtained expression vectors were designated as "pEF1/FCCU/h#11B7-T1H", "pEF1/FCCU/h#11B7-T2H", "pEF1/FCCU/h#11B7-T3H", "pEF1/FCCU/h#11B7-T4H", "pEF1/FCCU/h#11B7-T5H", "pEF1/FCCU/h#11B7-T6H", "pEF1/FCCU/h#11B7-T7H", "pEF1/FCCU/h#11B7-T8H", or "pEF1/FCCU/h#11B7-T9H", and the insert of each expression vector is represented by the nucleotide sequence of SEQ ID NO:31, 32, 33, 34, 35, 36, 37, 38, or 39 of the Sequence Listing, respectively.

### Example 10: Preparation of humanized antibody of rat anti-human AXL monoclonal antibody #11B7

### a) Production of humanized antibody

1.5x10⁸ FreeStyle 293-F cells at the log growth phase were seeded onto 100 mL of fresh FreeStyle 293 Expression Medium (Invitrogen Corp.) and shake-cultured (125 rpm) in an incubator at 37°C in 8% CO₂. 1 mg of Polyethyleneimine (Polyscience #24765) was dissolved in 4 mL of an Opti-Pro SFM medium ( Invitrogen Corp.) and left at room temperature for 5 minutes. A heavy chain expression plasmid (0.05 mg) and a light chain expression plasmid (0.15 mg) prepared using a PureLink HiPure Plasmid kit (Invitrogen Corp.) were suspended in 4 mL of an Opti-Pro SFM medium (Invitrogen Corp.). 4 mL of the expression plasmid/Opti-Pro SFM mixed solution was added to 4 mL of the Polyethyleneimine/Opti-Pro SFM mixed solution thus left at room temperature for 5 minutes and further left at room temperature for 5 minutes. Next, 8 mL of the Polyethyleneimine/expression plasmid/Opti-Pro SFM mixed solution was added to the FreeStyle 293-F cell suspension, and shake-culture was continued. After 7-day culture at 37°C in 8% CO₂, the culture supernatant was collected.

### b) Purification of humanized antibody

The culture supernatant obtained in the preceding paragraph a) was purified by Protein A affinity column chromatography. 100 mL of the culture supernatant was placed in a 500-mL flask with a cap, to which 1 mL of a MabSelect SuRe (GE Healthcare Bio-science Ltd.) suspension (50% slurry) equilibrated with PBS was in turn added. The mixture was stirred overnight at 100 rpm in an incubator at 10°C. The FreeStyle 293-F cell culture supernatant/MabSelect SuRe suspension was applied to 5 mL of Zeba Spin Column, empty (PIERCE). The whole resin was placed in the column and then washed with 10 mL of 1 M NaCl. Next, 1 mL of a 1 M arginine solution (pH 4.0) was applied thereto to collect antibody-containing fractions. The fractions were added to Centrifugal Filter Device (Amicon Ultra-4, molecular weight cut off: 50 K, Millipore Corp.), followed by solution replacement by a citrate buffer and concentration was adjusted to 200 µL to prepare purified samples.

A humanized antibody #11B7 obtained by the combination between pEF6KCL/h#11B7-T1L and pEF1/FCCU/h#11B7-T1H was designated as "h#11B7-T1"; a humanized antibody #11B7 obtained by the combination between pEF6KCL/h#11B7-T2L and pEF1/FCCU/h#11B7-T2H was designated as "h#11B7-T2"; a humanized antibody #11B7 obtained by the combination between pEF6KCL/h#11B7-T3L and pEF1/FCCU/h#11B7-T3H was designated as "h#11B7-T3"; a humanized antibody #11B7 obtained by the combination between pEF6KCL/h#11B7-T4L and pEF1/FCCU/h#11B7-T4H was designated as "h#11B7-T4"; a humanized antibody #11B7 obtained by the combination between pEF6KCL/h#11B7-T5L and pEF1/FCCU/h#11B7-T5H was designated as "h#11B7-T5"; a humanized antibody #11B7 obtained by the combination between pEF6KCL/h#11B7-T6L and pEF1/FCCU/h#11B7-T6H was designated as "h#11B7-T6"; a humanized antibody #11B7 obtained by the combination between pEF6KCL/h#11B7-T7L and pEF1/FCCU/h#11B7-T7H was designated as "h#11B7-T7"; a humanized antibody #11B7 obtained by the combination between pEF6KCL/h#11B7-T8L and pEF1/FCCU/h#11B7-T7H was designated as "h#11B7-T8"; a humanized antibody #11B7 obtained by the combination between pEF6KCL/h#11B7-T9L and pEF1/FCCU/h#11B7-T7H was designated as "h#11B7-T9"; a humanized antibody #11B7 obtained by the combination between pEF6KCL/h#11B7-T10L and pEF1/FCCU/h#11B7-T7H was designated as "h#11B7-T10"; a humanized antibody #11B7 obtained by the combination between pEF6KCL/h#11B7-T11L and pEF1/FCCU/h#11B7-T7H was designated as "h#11B7-T11"; a humanized antibody #11B7 obtained by the combination between pEF6KCL/h#11B7-T12L and pEF1/FCCU/h#11B7-T7H was designated as "h#11B7-T12"; a humanized antibody #11B7 obtained by the combination between pEF6KCL/h#11B7-T13L and pEF1/FCCU/h#11B7-T7H was designated as "h#11B7-T13"; a humanized antibody #11B7 obtained by the combination between pEF6KCL/h#11B7-T7L and pEF1/FCCU/h#11B7-T8H was designated as "h#11B7-T14"; a humanized antibody #11B7 obtained by the combination between pEF6KCL/h#11B7-T8L and pEF1/FCCU/h#11B7-T8H was designated as "h#11B7-T15"; a humanized antibody #11B7 obtained by the combination between pEF6KCL/h#11B7-T9L and pEF1/FCCU/h#11B7-T8H was designated as "h#11B7-T16"; a humanized antibody #11B7 obtained by the combination between pEF6KCL/h#11B7-T10L and pEF1/FCCU/h#11B7-T8H was designated as "h#11B7-T17"; a humanized antibody #11B7 obtained by the combination between pEF6KCL/h#11B7-T11L and pEF1/FCCU/h#11B7-T8H was designated as "h#11B7-T18"; a humanized antibody #11B7 obtained by the combination between pEF6KCL/h#11B7-T12L and pEF1/FCCU/h#11B7-T8H was designated as "h#11B7-T19"; a humanized antibody #11B7 obtained by the combination between pEF6KCL/h#11B7-T13L and pEF1/FCCU/h#11B7-T8H was designated as "h#11B7-T20"; a humanized antibody #11B7 obtained by the combination between pEF6KCL/h#11B7-T7L and pEF1/FCCU/h#11B7-T9H was designated as "h#11B7-T21"; a humanized antibody #11B7 obtained by the combination between pEF6KCL/h#11B7-T8L and pEF1/FCCU/h#11B7-T9H was designated as "h#11B7-T22"; a humanized antibody #11B7 obtained by the combination between pEF6KCL/h#11B7-T9L and pEF1/FCCU/h#11B7-T9H was designated as "h#11B7-T23"; a humanized antibody #11B7 obtained by the combination between pEF6KCL/h#11B7-T10L and pEF1/FCCU/h#11B7-T9H was designated as "h#11B7-T24"; a humanized antibody #11B7 obtained by the combination between pEF6KCL/h#11B7-T11L and pEF1/FCCU/h#11B7-T9H was designated as "h#11B7-T25"; a humanized antibody #11B7 obtained by the combination between pEF6KCUh#11B7-T12L and pEF1/FCCU/h#11B7-T9H was designated as "h#11B7-T26";and a humanized antibody #11B7 obtained by the combination between pEF6KCL/h#11B7-T13L and pEF1/FCCU/h#11B7-T9H was designated as "h#11B7-T27".

**Table 1**

| h#11B7 | H plasmid | L plasmid |
|---|---|---|
| h#11B7-T□ | pEF1/FCCU/h#11B7-T1H | pEF6KCL/h#11B7-T1L |
| h#11B7-T2 | pEF1/FCCU/h#11B7-T2H | pEF6KCL/h#11B7-T2L |
| h#11B7-T3 | pEF1/FCCU/h#11B7-T3H | pEF6KCL/h#11B7-T3L |
| h#11B7-T4 | pEF1/FCCU/h#11B7-T4H | pEF6KCL/h#11B7-T4L |
| h#11B7-T5 | pEF1/FCCU/h#11B7-T5H | pEF6KCL/h#11B7-T5L |
| h#11B7-T6 | pEF1/FCCU/h#11B7-T6H | pEF6KCL/h#11B7-T6L |
| h#11B7-T7 | pEF1/FCCU/h#11B7-T7H | pEF6KCL/h#11B7-T7L |
| h#11B7-T8 | pEF1/FCCU/h#11B7-T7H | pEF6KCL/h#11B7-T8L |
| h#11B7-T9 | pEF1/FCCU/h#11B7-T7H | pEF6KCL/h#11B7-T9L |
| h#11B7-T10 | pEF1/FCCU/h#11B7-T7H | pEF6KCL/h#11B7-T10L |
| h#11B7-T11 | pEF1/FCCU/h#11B7-T7H | pEF6KCL/h#11B7-T11L |
| h#11B7-T12 | pEF1/FCCU/h#11B7-T7H | pEF6KCL/h#11B7-T12L |
| h#11B7-T13 | pEF1/FCCU/h#11B7-T7H | pEF6KCL/h#11B7-T13L |
| h#11B7-T14 | pEF1/FCCU/h#11B7-T8H | pEF6KCL/h#11B7-T7L |
| h#11B7-T15 | pEF1/FCCU/h#11B7-T8H | pEF6KCL/h#11B7-T8L |
| h#11B7-T16 | pEF1/FCCU/h#11B7-T8H | pEF6KCL/h#11B7-T9L |
| h#11B7-T17 | pEF1/FCCU/h#11B7-T8H | pEF6KCL/h#11B7-T10L |
| h#11B7-T18 | pEF1/FCCU/h#11B7-T8H | pEF6KCL/h#11B7-T11L |
| h#11B7-T19 | pEF1/FCCU/h#11B7-T8H | pEF6KCL/h#11B7-T12L |
| h#11B7-T20 | pEF1/FCCU/h#11B7-T8H | pEF6KCL/h#11B7-T13L |
| h#11B7-T21 | pEF1/FCCU/h#11B7-T9H | pEF6KCL/h#11B7-T7L |
| h#11B7-T22 | pEF1/FCCU/h#11B7-T9H | pEF6KCL/h#11B7-T8L |
| h#11B7-T23 | pEF1/FCCU/h#11B7-T9H | pEF6KCL/h#11B7-T9L |
| h#11B7-T24 | pEF1/FCCU/h#11B7-T9H | pEF6KCL/h#11B7-T10L |
| h#11B7-T25 | pEF1/FCCU/h#11B7-T9H | pEF6KCL/h#11B7-T11L |
| h#11B7-T26 | pEF1/FCCU/h#11B7-T9H | pEF6KCL/h#11B7-T12L |
| h#11B7-T27 | pEF1/FCCU/h#11B7-T9H | pEF6KCL/h#11B7-T13L |

### Example 11: Production of humanized antibody gene of rat anti-human AXL monoclonal antibody #11D5

### a) Construction of h#11D5-T1L, h#11D5-T2L, h#11D5-T3L, h#11D5-T4L, h#11D5-T5L, and h#11D5-T6L light chain expression vectors

Each DNA containing a gene encoding a h#11D5-T1L, h#11D5-T2L, h#11D5-T3L, h#11D5-T4L, h#11D5-T5L, and h#11D5-T6L light chain variable region (Figure 6) represented by amino acid Nos. 21 to 129 of SEQ ID NO:55, 56, 57, 58, 59, or 60, respectively, of the Sequence Listing, fused with a secretion signal was synthesized (MBL Medical & Biological Laboratories Co., Ltd, Artificial Gene Synthesis Service) and digested with restriction enzymes Nhel and BsiWI. The resulting DNA fragments were separately inserted into sites of general-purpose vectors for humanized antibody light chain expression (pEF6KCL) digested in advance with restriction enzymes NheI and BsiWI to thereby construct h#11D5-T1L, h#11D5-T2L, h#11D5-T3L, h#11D5-T4L, h#11D5-T5L, and h#11D5-T6L light chain expression vectors. The obtained expression vectors were designated as "pEF6KCL/h#11D5-T1L", "pEF6KCL/h#11D5-T2L", "pEF6KCL/h#11D5-T3L", "pEF6KCL/h#11D5-T4L", "pEF6KCL/h#11D5-T5L", or "pEF6KCL/h#11B7-T6L", and the insert of each expression vector is represented by the nucleotide sequence of SEQ ID NO:49, 50, 51, 52, 53, or 54 of the Sequence Listing, respectively.

### b) Construction of h#11D5-T1H, h#11D5-T2H, h#11D5-T3H, h#11D5-T4H, h#11D5-T5H, and h#11D5-T6H heavy chain expression vectors

Each DNA containing a gene encoding a h#11D5-T1H, h#11D5-T2H, h#11D5-T3H, h#11D5-T4H, h#11D5-T5H, or h#11D5-T6H heavy chain variable region (Figure 12) represented by amino acid Nos. 20 to 132 of SEQ ID NO:67, 68, 69, 70, 71, or 72, respectively, of the Sequence Listing was synthesized (MBL Medical & Biological Laboratories Co., Ltd, Artificial Gene Synthesis Service) and digested with a restriction enzyme Blpl. The resulting DNA fragments were separately inserted into sites of general-purpose vectors for humanized antibody heavy chain expression (pEF1/FCCU-1) digested in advance with a restriction enzyme BlpI to thereby construct h#11D5-T1H, h#11D5-T2H, h#11D5-T3H, h#11D5-T4H, h#11D5-T5H, and h#11D5-T6H heavy chain expression vectors. The obtained expression vectors were designated as "pEF1/FCCU/h#11D5-T1H", "pEF1/FCCU/h#11D5-T2H", "pEF1/FCCU/h#11D5-T3H", "pEF1/FCCU/h#11D5-T4H", "pEF1/FCCU/h#11D5-T5H", or "pEF1/FCCU/h#11D5-T6H", and the insert of each expression vector is represented by the nucleotide sequence of SEQ ID NO:61, 62, 63, 64, 65, or 66 of the Sequence Listing, respectively.

### Example 12: Preparation of humanized antibody of rat anti-human AXL monoclonal antibody #11D5

### a) Production of humanized antibody

1.5x10⁸ FreeStyle 293-F cells at the log growth phase were seeded onto 100 mL of fresh FreeStyle 293 Expression Medium (Invitrogen Corp.) and shake-cultured (125 rpm) in an incubator at 37°C in 8% CO₂. 1 mg of Polyethyleneimine (Polyscience #24765) was dissolved in 4 mL of an Opti-Pro SFM medium ( Invitrogen Corp.) and left at room temperature for 5 minutes. A heavy chain expression plasmid (0.05 mg) and a light chain expression plasmid (0.15 mg) prepared using a PureLink HiPure Plasmid kit (Invitrogen Corp.) were suspended in 4 mL of an Opti-Pro SFM medium (Invitrogen Corp.). 4 mL of the expression plasmid/Opti-Pro SFM mixed solution was added to 4 mL of the Polyethyleneimine/Opti-Pro SFM mixed solution thus left at room temperature for 5 minutes and further left at room temperature for 5 minutes. Next, 8 mL of the Polyethyleneimine/expression plasmid/Opti-Pro SFM mixed solution was added to the FreeStyle 293-F cell suspension, and shake-culture was continued. After 7-day culture at 37°C in 8% CO₂, the culture supernatant was collected.

### b) Purification of humanized antibody

The culture supernatant obtained in the preceding paragraph a) was purified by Protein A affinity column chromatography. 100 mL of the culture supernatant was placed in a 500-mL flask with a cap, to which 1 mL of a MabSelect SuRe (GE Healthcare Bio-science Ltd.) suspension (50% slurry) equilibrated with PBS was in turn added. The mixture was stirred overnight at 100 rpm in an incubator at 10°C. The FreeStyle 293-F cell culture supernatant/MabSelect SuRe suspension was applied to 5 mL of Zeba Spin Column, empty (PIERCE). The whole resin was placed in the column and then washed with 10 mL of 1 M NaCl. Next, 1 mL of a 1 M arginine solution (pH 4.0) was applied thereto to collect antibody-containing fractions. The fractions were added to Centrifugal Filter Device (Amicon Ultra-4, molecular weight cut off: 50 K, Millipore Corp.), followed by solution replacement by a citrate buffer and concentration. The final capacity was adjusted to 200 µl to prepare purified samples.

A humanized antibody #11D5 obtained by the combination between pEF6KCL/h#11D5-T1L and pEF1/FCCU/h#11D5-T1H was designated as "h#11D5-T1"; a humanized antibody #11D5 obtained by the combination between pEF6KCL/h#11D5-T2L and pEF1/FCCU/h#11D5-T2H was designated as "h#11D5-T2"; a humanized antibody #11D5 obtained by the combination between pEF6KCL/h#11D5-T3L and pEF1/FCCU/h#11D5-T3H was designated as "h#11D5-T3"; a humanized antibody #11D5 obtained by the combination between pEF6KCL/h#11D5-T4L and pEF1/FCCU/h#11D5-T4H was designated as "h#11D5-T4"; a humanized antibody #11D5 obtained by the combination between pEF6KCUh#11D5-T5L and pEF1/FCCU/h#11D5-T5H was designated as "h#11D5-T5"; and a humanized antibody #11D5 obtained by the combination between pEF6KCL/h#11D5-T6L and pEF1/FCCU/h#11D5-T6H was designated as "h#11D5-T6".

### Example 13: Evaluation of affinity of humanized antibody of rat anti-human AXL monoclonal antibody #11B7 for human AXL-Fc fusion protein

The humanized antibodies h#11B7-T1 to h#11B7-T27 derived from rat anti-human AXL monoclonal antibody #11 B7 were evaluated for their affinity for human AXL-Fc by method shown below. Human AXL-Fc (manufactured by R&D Systems, #154-AL) was diluted with PBS to 1 µg/ml. Then, the solution was dispensed in an amount of 100 µl/well to Immuno Plate (manufactured by Nalge Nunc International K.K., #437111) and left standing overnight at 4°C to adsorb the protein onto the plate. The next day, the wells were washed 5 times with a PBS-T solution (PBS and 0.05% (v/v) Tween 20). Then, a solution containing skim milk (manufactured by Morinaga Milk Industry Co., Ltd.) diluted with PBS to 5% was dispensed in an amount of 100 µl/well and left standing at room temperature for 2 hours. The solutions in the wells were removed and the wells were washed 5 times with a PBS-T solution. Then, the purified humanized antibodies T1 to T27 of rat anti-human AXL monoclonal antibody #11B7 prepared in Example 10 were separately diluted at a final concentration of 1 µg/ml to 0.00256 ng/ml (5-fold dilution series) with a PBS solution containing 0.5% skim milk. Then, the solution was dispensed in an amount of 100 µl/well and left standing at room temperature for 2 hours. In this procedure, each antibody concentration was determined according to Example 15. The wells were washed 5 times with a PBS-T solution. Then, Alkaline Phosphatase-conjugated AffiniPure Goat Anti-Human IgG (manufactured by Jackson ImmunoResearch Laboratories, Inc., #109-055-097) diluted 2500 times with a TBS-T solution (TBS and 0.05% (v/v) Tween 20) was added in an amount of 100 µl/well and left standing at room temperature for 1 hour. The solutions in the wells were removed and the wells were washed 5 times with a TBS-T solution. Then, a fluorescent substrate solution (manufactured by Roche Diagnostics K.K., #11681982001) was added in an amount of 100 µl/well to perform fluorescence reaction. The fluorescence intensity on the human AXL-Fc-adsorbed plate was measured 15 minutes after the addition of the fluorescent substrate solution using SpectraMax M5 (manufactured by Molecular Devices Corp.). As a result, of the examined 27 samples of humanized antibodies of rat anti-human AXL monoclonal antibody #11 B7, #11B7-T1 and h#11B7-T4 were confirmed to have lower binding activity than that of human chimeric antibody. However, the other 25 samples were confirmed to have binding activity almost equal to that of human chimeric antibody for human AXL-Fc protein in an antibody concentration-dependent manner (Figures 19, 20, 21 and 22).

### Example 14: Evaluation of affinity of humanized antibody of rat anti-human AXL monoclonal antibody #11D5 for human AXL-Fc fusion protein

The humanized antibodies h#11D5-T1 to h#11D5-T6 of rat anti-human AXL monoclonal antibody #11 D5 were evaluated for their affinity for human AXL-Fc by method shown below. Human AXL-Fc (manufactured by R&D Systems, #154-AL) was diluted with PBS to 1 µg/ml. Then, the solution was dispensed in an amount of 100 µl/well to Immuno Plate (manufactured by Nalge Nunc International K.K., #437111) and left standing overnight at 4°C to adsorb the protein onto the plate. The next day, the wells were washed 5 times with a PBS-T solution (PBS and 0.05% (v/v) Tween 20). Then, a solution containing skim milk (manufactured by Morinaga Milk Industry Co., Ltd.) diluted with PBS to 5% was dispensed in an amount of 100 µl/well and left standing at room temperature for 2 hours. The solutions in the wells were removed and the wells were washed 5 times with a PBS-T solution. Then, the purified humanized antibodies T1 to T6 of rat anti-human AXL monoclonal antibody #11 D5 prepared in Example 12 were separately diluted at a final concentration of 1 µg/ml to 0.00256 ng/ml (5-fold dilution series) with a PBS solution containing 0.5% skim milk. Then, the solution was dispensed in an amount of 100 µl/well and left standing at room temperature for 2 hours. In this procedure, each antibody concentration was determined according to Example 15. The wells were washed 5 times with a PBS-T solution. Then, Alkaline Phosphatase-conjugated AffiniPure Goat Anti-Human IgG (manufactured by Jackson ImmunoResearch Laboratories, Inc., #109-055-097) diluted 2500 times with a TBS-T solution (TBS and 0.05% (v/v) Tween 20) was added in an amount of 100 µl/well and left standing at room temperature for 1 hour. The solutions in the wells were removed and the wells were washed 5 times with a TBS-T solution. Then, a fluorescent substrate solution (manufactured by Roche Diagnostics K.K., #11681982001) was added in an amount of 100 µl/well to perform fluorescence reaction. The fluorescence intensity on human AXL-Fc-adsorbed plate was measured 15 minutes after the addition of the fluorescent substrate solution using SpectraMax M5 (manufactured by Molecular Devices Corp.). As a result, of examined 6 samples of humanized antibodies of rat anti-human AXL monoclonal antibody #11 D5, h#11D5-T4, h#11D5-T5, and h#11D5-T6 were confirmed to have binding activity almost equal to that of human chimeric antibody for human AXL-Fc protein in an antibody concentration-dependent manner (Figure 23).

### Example 15: The absorption coefficient calculation and direct protein absorbance assay of humanized antibody of rat anti-human AXL monoclonal antibodies #11B7 and #11D5

The protein concentration of humanized antibodies h#11B7-T1 to h#11B7-T27 derived from the rat anti-human AXL monoclonal antibody #11 B7 and h#11D5-T1 to h#11D5-T6 derived from the rat anti-human AXL monoclonal antibody #11D5 was determined by the protocol shown below. Each humanized antibody of rat anti-human AXL monoclonal antibody #11B7 or #11D5 prepared in Example 10 or 12, respectively, was diluted by its mass using an MX5 Automated-S microbalance (METTLER TOLEDO). The absorbance at 280 nm (A280) was measured for each antibody with a DU-7400 UV-vis Spectrophotometer (Beckman Coulter, Inc.) using quartz cells at room temperature. The protein concentration of each antibody was determined by the absorption coefficient. The absorption coefficient of each antibody was determined using the software Sednterp, which can be downloaded from the website of the National Institute of Health (http://iphilo.mailway.com/download.htm). The results are described in Figures 24 (upper panel for h#11B7-T1 to h#11B7-T14; lower panel for h#11B7-T15 to h#11B7-T27) and 25 (for h#11D5-T1 to h#11D5-T6).

### Example 16: Determination of binding site (epitope) for the rat anti-human AXL monoclonal antibody #11B7

### a) Expression and purification of human AXL tandem IG domain

DNA encoding a protein comprising human AXL IG domains (amino acid Nos. 26 to 220 in NCBI protein database ACCESSION No. P_30530: SEQ ID NO: 139) linked to an N-terminal His-tag and a Thrombin recognition sequence was incorporated in a vector pDEST14 (Invitrogen Corp., catalog No.: 11801-016). E. coli Rosetta-gami B(DE3) (Novagen, catalog No.: 71136-4) was transformed with this plasmid and cultured in a TB medium (Invitrogen Corp., catalog No.: 22711-022). The bacterial cells thus cultured were ultrasonically disrupted and centrifuged, and the supernatant was purified using a HisTrap HP column (GE Healthcare, catalog No.: 17-5247-01). The elution was pooled and desalted by PD-10 column (GE Healthcare, catalog No.: 17-0851-01) then His-tag was cleaved by Thrombin (Sigma-Aldrich, catalog No.: T-7009). Cleaved protein was then subjected to MONO Q 5/50 GL(GE Healthcare, catalog No.: 17-5166-01) and Superdex 75 10/300 column (GE Healthcare, catalog No.: 17-5174-01) until a single band with a molecular weight of 21 kDa was obtained.

### b) Expression and purification of human AXL single IG domain

DNA encoding a protein comprising human AXL IG domains (amino acid Nos. 26 to 131 and 129 to 220 in NCBI protein database ACCESSION No. P_30530) linked to an N-terminal His-tag and a Factor Xa recognition sequence was incorporated in a vector pDEST14 (Invitrogen Corp., catalog No.: 11801-016). E. coli Rosetta-gami B(DE3) (Novagen, catalog No.: 71136-4) was transformed with this plasmid and cultured in a TB medium (Invitrogen Corp., catalog No.: 22711-022). The bacterial cells thus cultured were ultrasonically disrupted and centrifuged, and the supernatant was purified using a HisTrap HP column (GE Healthcare, catalog No.: 17-5247-01). Then, the human AXL IG domains were purified by electrophoresis using a Superdex 75 10/300 column (GE Healthcare, catalog No.: 17-5174-01) until a single band with a molecular weight of 11 kDa was obtained.

### c) Determination of the binding site (epitope) on the human AXL-IG domain for rat anti-human AXL monoclonal antibody #11B7

The binding site (epitope) on the human AXL-IG domain for the rat anti-human AXL monoclonal antibody #11 B7 was evaluated by the following method: Peptides containing either of the two IG domains in human AXL (NHis-hAXL26-131 and NHis-hAXL129-220), and a peptide containing both of the two IG domains in human AXL (hAXL26-220) were separately diluted with PBS to 1 µg/ml. Then, each solution was dispensed in the amount of 100 µl/well to an Immuno Plate (manufactured by Nalge Nunc International K.K., #437111) and left standing overnight at 4°C to adsorb the peptide onto the plate. On the next day, the wells were washed 5 times with a PBS-T solution (PBS and 0.05% (v/v) Tween 20). Then, a solution containing skim milk (manufactured by Morinaga Milk Industry Co., Ltd.) diluted with PBS to 5% was dispensed in an amount of 100 µl/well and left standing at room temperature for 2 hours. The solutions in the wells were removed, and the wells were washed 5 times with a PBS-T solution. The rat anti-human AXL monoclonal antibody #11B7 was diluted to 0.04 µg/ml with a PBS solution containing 0.5% skim milk. Then, the solution was dispensed in amount of 100 µl/well and left standing at room temperature for 2 hours. The wells were washed 5 times with a PBS-T solution. Then, Alkaline Phosphatase-conjugated AffiniPure Goat Anti-Human IgG (manufactured by Jackson ImmunoResearch Laboratories, Inc., #109-055-097) diluted 2500 times with a TBS-T solution (TBS and 0.05% (v/v) Tween 20) was added in an amount of 100 µl/well and left standing at room temperature for 1 hour. The solutions in the wells were removed, and the wells were washed 5 times with a TBS-T solution. Then, a fluorescent substrate solution (manufactured by Roche Diagnostics K.K., #11681982001) was added in the amount of 100 µl/well to perform fluorescence reaction. The fluorescence intensity on the human AXL-IG domain-adsorbed plate was measured 15 minutes after the addition of the fluorescent substrate solution using a SpectraMax M5 (manufactured by Molecular Devices Corp.). As a result of the ELISA, only hAXL26-220 and NHis-hAXL129-220 were confirmed to have binding activity. Therefore, it was demonstrated that the epitope for the rat anti-human AXL monoclonal antibody #11 B7 is, of the two IG domains in human AXL, the domain closer to the carboxy terminus (the domain comprising amino acid residues of amino acid Nos. 129-220 in NCBI protein database ACCESSION No. P_30530: SEQ ID NO: 139; Figure 30A) (Figure 26).

### Example 17: Humanized anti-AXL antibodies of the invention inhibit ligand-induced AXL phosphorylation in vitro

The protein encoded by the growth arrest specific gene 6, Gas6, represents a natural ligand of the receptor tyrosine kinase AXL. Binding of Gas6 to AXL results in receptor activation which is reflected by increased receptor tyrosine kinase phosphorylation levels. The experiments described in example 17 were set out to address the potential of the humanized anti-AXL antibodies h#11B7-T2, h#11B7-T3, h#11B7-T4, h#11B7-T5, and h#11B7-T6 of the invention to interfere with Gas6-mediated activation of the receptor tyrosine kinase AXL.

### Example 17.1: Humanized anti-AXL antibodies of the invention inhibit ligand-induced AXL phosphorylation in vitro as determined by ELISA

ELISA experiments were performed in order to investigate whether the humanized anti-AXL antibodies of the invention are able to block ligand Gas6-induced phosphorylation of AXL. In brief, on day 1, 3x10⁴ cells per well were seeded in normal growth medium in flat-bottom 96 well plates. The next day, growth medium was replaced by serum-free medium to starve cells over night for 24 h. Also over night, black Maxi-Sorp 96 well plates (Nunc) were coated with mouse anti-phospho-tyrosine antibody 4G10 at 2 µg/ml PBS and 4°C. On day 3, the 4G10 antibody solution was removed and Maxi-Sorp wells were blocked with PBS, 0.5% BSA for at least 4 h at room temperature. In parallel, cells were pre-incubated with 10 µg/ml of Gammagard control antibody (Sigma), the chimeric anti-AXL mAb #11B7 as well as the humanized anti-AXL antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Medium was then flicked out and cells were lysed in lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) for 30 min on ice. Meanwhile, blocking buffer was removed and Maxi-Sorp plates were washed 6x with wash buffer (PBS, 0.05% Tween 20), before lysates were transferred and incubated over night at 4°C. After plates were washed 6x with wash buffer on day 4, wells were incubated with biotinylated rat anti-AXL antibody 12B7 at 0.5 µg/ml PBS for 2 h at room temperature. Plates were washed 6x with wash buffer and AP-conjugated streptavidin (Chemicon #SA110) diluted 1:4.000 in PBS was added to each well and incubated for 30 min at room temperature. Afterwards, wells were washed 6x with wash buffer and AttoPhos substrate solution (Roche #11681982) was added. Using a Victor plate reader (Perkin Elmer), the fluorescence of each well was collected at an excitation wavelength of 430 nm and an emission wavelength of 580 nm.

**Figure 27** shows representative results of this experiment for Hs578T breast cancer cells. Compared with Gammagard control antibody, the chimeric anti-AXL antibody #11B7 as well as the humanized anti-AXL antibodies h#11B7-T2, h#11B7-T3, h#11B7-T4, h#11B7-T5, and h#11B7-T6 of the invention were able to block or significantly reduce Gas6-mediated AXL activation in Hs578T breast cancer cells as indicated by decreased AXL tyrosine phosphorylation levels. Similar effects with the same panel of antibodies were observed in AXL over-expressing NIH3T3-AXL cl. 7 fibroblasts, the lung cancer cell line NCI-H292, the melanoma cell line C-8161, as well as the prostate cancer cell lines PC-3 and DU-145.

### Example 17.2: Humanized anti-AXL antibodies of the invention inhibit ligand-induced AXL phosphorylation in vitro as determined by Western-Blot analysis

Moreover, Western Blot analyses were conducted in order to confirm the humanized antibodies' ability to interfere with ligand Gas6-induced activation of AXL. For this purpose, 1.5 x 10⁶ Hs578T breast cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Sigma), the chimeric anti-AXL mAb #11 B7 as well as the humanized anti-AXL antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 1 ml of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. For immunoprecipitation, the protein concentrations of supernatants were determined, and 500 µg of whole cell lysates were incubated with 30 µl of a protein A-sepharose suspension and 1 µg of the rat anti-AXL monoclonal antibody 12B7 on a rotation wheel for 3 h at 4°C. The precipitates were washed three times with 0.7 ml of chilled HNTG buffer (20 mM HEPES pH 7.5, 150 mM NaCl, 0.1% Triton-X-100, 10% glycerol, and 10 mM Na₄P₂O₇), suspended and boiled in 40 µl of 3xSDS Laemmli sample buffer, and subjected to SDS PAGE. For Western Blot analysis, proteins were transferred to nitrocellulose membrane. Afterwards, the membrane was blocked with NET-Gelatine and incubated with mouse monoclonal anti-phospho-tyrosine primary antibody 4G10 (Upstate) over night. After 3 washes with NET-Gelatine the next day, the membrane was incubated with HRP-conjugated anti-mouse secondary antibody for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-AXL antibody.

**Figure 27** shows representative results of this experiment. Compared with Gammagard control antibody, the chimeric anti-AXL antibody #11B7 as well as the humanized anti-AXL antibodies h#11B7-T2, h#11B7-T3, h#11B7-T4, h#11B7-T5, and h#11B7-T6 of the invention were able to block or significantly reduce Gas6-induced AXL activation in Hs578T breast cancer cells as indicated by decreased AXL tyrosine phosphorylation levels.

### Example 18: Humanized anti-AXL antibodies of the invention inhibit ligand-induced phosphorylation of AXL downstream signaling molecules in vitro

Gas6-induced activation of the receptor tyrosine kinase AxI is not only reflected by increased tyrosine phosphorylation levels of AxI itself, but is also associated with the induction of downstream signaling cascades, including the ERK1/2 and the PKB/AKT pathways. We therefore continued with experiments to address the potential of the humanized anti-Axl antibodies h#1187-T2, h#11B7-T3, h#11B7-T4, h#11B7-T5, and h#11B7-T6 of the invention to interfere with Gas6-mediated activation of ERK1 and ERK2 as well as AKT. Moreover, we found the phosphorylation status of the signaling molecules GSK-3β, TSC2, mTOR, and S6K1 to be modulated upon stimulation with Gas6 as well, and thus investigated the inhibitory effect of the humanized anti-Axl antibodies h#11B7-T2, h#11B7-T3, h#11B7-T4, h#11B7-T5, and h#11B7-T6 of the invention on those molecules as well.

### Example 18.1: Humanized 11 B7 anti-Axl antibodies of the invention inhibit ligand-induced phosphorylation of ERK1/2 in vitro

Western Blot analyses were conducted in order to investigate whether the humanized antibodies of the invention have the potential to interfere with Gas6-induced activation of ERK1/2. Gas6-mediated activation of ERK1/2 is reflected by increased phosphorylation levels at its positions Thr202 and Tyr204. For this purpose, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3 x SDS Laemmli sample buffer, and subjected to SDS PAGE. Upon transfer of the proteins to nitrocellulose membrane, the membrane was blocked with NET-Gelatine and incubated with rabbit polyclonal anti-phospho-p44/42 MAP kinase (Thr202/Tyr204) primary antibody (Cell Signaling Technologies, #9101) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-p44/42 MAP kinase antibody (Santa Cruz K-23, #sc-153).

Figure 28A shows representative results of this experiment. Compared with Gammagard control antibody, the chimeric anti-Axl antibody chm11 B7 as well as the humanized anti-Axl antibodies h#11B7-T2, h#11B7-T3, h#11B7-T4, h#11B7-T5, and h#11B7-T6 of the invention interfered with the weak, Gas6-induced increase of ERK1/2 activation in Hs578T breast cancer cells. Due to the high basal activity of ERK1/2 in this cell line, however, these effects which are indicated by decreased ERK1/2 Thr202/Tyr204 phosphorylation levels in Gas6-stimulated versus corresponding non-stimulated cells appear relatively moderate only (top). In contrast, the inhibitory effects of the chimeric anti-Axl antibody chm11B7 as well as the humanized anti-Axl antibodies h#11B7-T2, h#11B7-T3, h#11B7-T4, h#11B7-T5, or h#11B7-T6 on ERK1/2 Thr202/Tyr204 phosphorylation are much clearer reflected in NCI-H292 lung cancer cells (bottom).

### Example 18.2: Humanized 11B7 anti-Axl antibodies of the invention inhibit ligand-induced phosphorylation of AKT in vitro

Western Blot analyses were conducted in order to investigate whether the humanized antibodies of the invention have the potential to interfere with Gas6-induced activation of AKT. Gas6-mediated AKT activation is thereby reflected by increased phosphorylation levels at its position Ser473. For this purpose, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3xSDS Laemmli sample buffer, and subjected to SDS PAGE. Upon transfer of the proteins to nitrocellulose membrane, the membrane was blocked with NET-Gelatine and incubated with rabbit polyclonal anti-AKT1/2/3 primary antibody (Cell Signaling Technologies, #9272) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-phospho-AKT (Ser473) antibody (Cell Signaling Technologies, #9271).

Figure 28B shows representative results of this experiment. Compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention were able to significantly reduce Gas6-induced AKT activation in Hs578T breast cancer cells (top) and NCl-H292 lung cancer cells (bottom) as indicated by decreased AKT Ser473 phosphorylation levels in Gas6-stimulated versus corresponding non-stimulated cells.

### Example 18.3: Humanized 11B7 anti-Axl antibodies of the invention inhibit ligand-induced phosphorylation of GSK-3β in vitro

Western Blot analyses were conducted in order to investigate whether the humanized antibodies of the invention have the potential to interfere with Gas6-induced activation of GSK-3β which is reflected by increased phosphorylation levels at its position Ser9. For this purpose, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3xSDS Laemmli sample buffer, and subjected to SDS PAGE. Upon transfer of the proteins to nitrocellulose membrane, the membrane was blocked with NET-Gelatine and incubated with rabbit polyclonal anti-phospho- GSK-3β (Ser9) primary antibody (Cell Signaling Technologies, #9336) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti- GSK-3β antibody (Becton Dickinson, #610201) and HRP-conjugated anti-mouse secondary antibody (Dianova, #315-036-045).

**Figure 28C** shows representative results of this experiment. Compared with Gammagard control antibody, the chimeric anti-Axl antibody chm11 B7 as well as the humanized anti-Axl antibodies h#11B7-T2, h#11B7-T3, h#11B7-T4, h#11B7-T5, and h#11B7-T6 of the invention were able to significantly reduce Gas6-induced GSK-3β activation in Hs578T breast cancer cells (top) and NCI-H292 lung cancer cells (bottom) as indicated by decreased GSK-3β Ser9 phosphorylation levels in Gas6-stimulated versus corresponding non-stimulated cells.

### Example 18.4: Humanized 11B7 anti-Axl antibodies of the invention inhibit ligand-induced phosphorylation of TSC2 in vitro

Western Blot analyses were performed in order to investigate whether the humanized antibodies of the invention have the potential to interfere with Gas6-induced and PI3K/AKT-mediated phosphorylation of TSC2 at its amino acid residue Thr1462. The phosphorylation of TSC2 on Thr1462 is generally associated with an inhibition of the tumor suppressor function of the TSC complex and, as a consequence, the activation of the downstream mTOR pathway. In brief, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3 x SDS Laemmli sample buffer, and subjected to SDS PAGE. Upon transfer of the proteins to nitrocellulose membrane, the membrane was blocked with NET-Gelatine and incubated with rabbit polyclonal anti-TSC2 primary antibody (Cell Signaling Technologies, #3612) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-phospho-TSC2 (Thr1462) antibody (Cell Signaling Technologies, #3617).

**Figure 28D** shows representative results of this experiment. Compared with Gammagard control antibody, the chimeric anti-Axl antibody chm11 B7 as well as the humanized anti-Axl antibodies h#11B7-T2, h#11B7-T3, h#11B7-T4, h#11B7-T5, and h#11B7-T6 of the invention were able to significantly reduce Gas6-induced phosphorylation of TCS2 on Thr1462 in Hs578T breast cancer cells (top) and NCI-H292 lung cancer cells (bottom) as indicated by decreased phosphorylation levels of this amino acid residue in Gas6-stimulated versus corresponding non-stimulated cells.

### Example 18.5: Humanized 11B7 anti-Axl antibodies of the invention inhibit ligand-induced phosphorylation of mTOR in vitro

Given the effects on TSC2 phosphorylation, Western Blot analyses were conducted in order to investigate whether the humanized antibodies of the invention do also have the potential to interfere with Gas6-induced activation of mTOR. Gas6-mediated mTOR activation is reflected by increased phosphorylation levels at its position Ser2448. For this purpose, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11 B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3 x SDS Laemmli sample buffer, and subjected to SDS PAGE. Upon transfer of the proteins to nitrocellulose membrane, the membrane was blocked with NET-Gelatine and incubated with rabbit polyclonal anti-phospho-mTOR (Ser2448) primary antibody (Cell Signaling Technologies, #2971) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-mTOR kinase antibody (Cell Signaling Technologies, #2972).

**Figure 28E** shows representative results of this experiment. The inhibitory effects of the anti-Axl antibodies were relatively weak in Hs578T breast cancer cells (top). However, compared with Gammagard control antibody, the chimeric anti-Axl antibody chm11B7 as well as the humanized anti-Axl antibodies h#11B7-T2, h#11B7-T3, h#11B7-T4, h#11B7-T5, and h#11B7-T6 of the invention were able to interfere with the Gas6-induced mTOR activation in NCI-H292 lung cancer cells as indicated by decreased mTOR Ser2448 phosphorylation levels in Gas6-stimulated versus corresponding non-stimulated cells (bottom).

### Example 18.6: Humanized 11B7 anti-Axl antibodies of the invention inhibit ligand-induced phosphorylation of S6K1 in vitro

Finally, Western Blot analyses were performed in order to investigate whether the humanized antibodies of the invention have the potential to interfere with Gas6-induced activation of S6K1. Gas6-mediated S6K1 activation is reflected by increased phosphorylation levels at its positions Thr421 and Ser424. For this purpose, 5 x 10⁶ Hs578T breast cancer cells or 3 x 10⁶ NCI-H292 lung cancer cells were seeded on 10cm culture dishes on day 1. The day after, growth medium was replaced by serum-free medium in order to starve cells over night for 24 h. On day 3, cells were pre-incubated with 10 µg/ml of Gammagard control antibody (Baxter), the chimeric anti-Axl mAb chm11 B7 as well as the humanized anti-Axl antibodies h#11B7-T2, h#11B7-T3, h#11B7-T4, h#11B7-T5, and h#11B7-T6 for 3 h at 37°C, and then treated with or without 400 ng/ml Gas6 (R&D Systems) for 15 min at 37°C. Afterwards, medium was removed, cells were lysed in 800 µl of lysis buffer (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) on ice for 30 min, and cell debris were removed by centrifugation for 10 minutes at 10,000 x g and 4°C. The protein concentrations of supernatants were determined, 50 µg of whole cell lysates were suspended and boiled in 3xSDS Laemmli sample buffer, and subjected to SDS PAGE. Upon transfer of the proteins to nitrocellulose membrane, the membrane was blocked with NET-Gelatine and incubated with rabbit polyclonal anti-phospho-p70 S6 Kinase 1 (Thr421/Ser424) primary antibody (Cell Signaling Technologies, #9204) over night. After 3 washes with NET-Gelatine the next day, the membrane was incubated with HRP-conjugated anti-rabbit secondary antibody (Dianova, #111-036-045) for 1 h at room temperature and then washed 3 times with NET-Gelatine again. Applying the chemiluminescence detection kit according to the manufacturer's instructions (GE Healthcare), filters were finally exposed to films (Kodak). Afterwards, the same filters were re-probed with anti-β-actin antibody (Cell Signaling Technologies, #4967).

**Figure 28F** shows representative results of this experiment. Compared with Gammagard control antibody, the chimeric anti-Axl antibody chm11 B7 as well as the humanized anti-Axl antibodies h#11B7-T2, h#11B7-T3, h#11B7-T4, h#11B7-T5, and h#11B7-T6 of the invention showed some inhibitory effects on Gas6-induced S6K1 activation in Hs578T breast cancer cells as indicated by decreased S6K1 Thr421/Ser424 phosphorylation levels in Gas6-stimulated versus corresponding non-stimulated cells (top). However, a much stronger decrease of Gas6-induced S6K1 Thr421/Ser424 phosphorylation and thus activation upon pre-treatment with the chimeric anti-Axl antibody chm11B7 as well as the humanized anti-Axl antibodies h#11B7-T2, h#11B7-T3, h#11B7-T4, h#11B7-T5, or h#11B7-T6 of the invention could be observed in NCI-H292 lung cancer cells (bottom).

### Example 19: Humanized 11D5 anti-AxI antibodies of the invention inhibit ligand-induced AxI phosphorylation in vitro as determined by ELISA

In addition to the characterization of humanized h#11B7-T2, h#11B7-T3, h#11B7-T4, h#11B7-T5 and h#11B7-T6 anti-Axl mAb series, ELISA experiments were performed in order to investigate the potential of the humanized anti-Axl antibodies h#11 D5-T2, h#11D5-T3, h#11D5-T4, h#11D5-T5, and h#11D5-T6 of the invention to interfere with Gas6-induced phosphorylation and thus activation of the receptor tyrosine kinase Axl.
In brief, on day one 3 x 10⁴ Hs578T breast cancer cells per well were seeded in normal growth medium in flat-bottom 96 well plates. The next day, growth medium was replaced by serum-free medium to starve cells over night for 24 h. Also over night, black Maxi-Sorp 96 well plates (Nunc) were coated with mouse anti-phospho-tyrosine antibody 4G10 at 2 µg/ml PBS at 4°C. On day 3, the 4G10 antibody solution was removed and Maxi-Sorp wells were blocked with blocking buffer (PBS, 0.5% BSA) for at lest 4 h at room temperature. In parallel, cells were pre-incubated with 10 µg/ml of Gammagard control antibody (Baxter), the chimeric anti-Axl mAb chm11D5 as well as the humanized anti-Axl antibodies h#11D5-T2, h#11D5-T3, h#11D5-T4, h#11D5-T5, and h#11D5-T6 for 3 h at 37°C, and then treated with or without 400 ng/ml Gas6 (R&D Systems) for 15 min at 37°C. Medium was then flicked out and cells were lysed in 110 µl of lysis buffer per well (50 mM HEPES, pH 7.5, 150 mM NaCl, 1 mM EDTA, 10% glycerine, and 1% Triton X-100) supplemented with phosphatase and protease inhibitors (10 mM Na₄P₂O₇, 1 mM phenylmethylsulfonyl fluoride, 1 mM orthovanadate, 1 mM NaF, and 0,5% aprotinin) for 30 min on ice. Meanwhile, blocking buffer was removed and Maxi-Sorp plates were washed 6x with wash buffer (PBS, 0.05% Tween 20), before 100 µl lysate of each well were transferred and incubated over night at 4°C. After plates were washed 6x with wash buffer on day 4, wells were incubated with biotinylated rat anti-Axl antibody 12B7 at 0.125 µg/ml dilution buffer (20mM Tris, 50mM NaCl, pH7.3, 0.05% Tween 20, 0.1% BSA) for 2 h at room temperature. Plates were washed 6x with wash buffer and AP-conjugated streptavidin (Chemicon #SA110) diluted 1:20000 in dilution buffer was added to each well and incubated for 30 min at room temperature. Afterwards, wells were washed 6x with wash buffer and AttoPhos substrate solution (Roche #11681982) was added. Using a SpectraMax-GeminiEM plate reader (Molecular Devices), the fluorescence of each well was collected at an excitation wavelength of 430 nm and an emission wavelength of 580 nm.

**Figure 29** shows representative results of this experiment. Compared with Gammagard control antibody, the chimeric anti-Axl antibody chm11D5 as well as the humanized anti-Axl antibodies h#11D5-T2, h#11D5-T3, h#11D5-T4, h#11D5-T5, and h#11D5-T6 of the invention were able to block or significantly reduce Gas6-mediated AxI activation as indicated by decreased Axl tyrosine phosphorylation levels in Gas6-stimulated versus corresponding non-stimulated cells.

### Example 20: Rat and chimeric anti-AxI antibodies of the invention inhibit ligand-induced AxI phosphorylation in vitro to similar extent

Chimeric derivatives of the rat anti-Axl antibodies 11 B7 and 11D5 were generated as part of this invention (see above), In order to investigate whether the rat anti-Axl antibodies of the invention and the corresponding chimeric anti-AxI antibodies of the invention were able to inhibit ligand Gas6mediated AxI activation in vitro to similar extent, ELISA experiments on CaSki cervical cancer cells were performed. Gas6-mediated AxI activation was thereby detected by increased receptor tyrosine phosphorylation, In brief, on day 1, 3x104cells per well were seeded in normal growth medium in flat-bottom 96 well plates. The next day, growth medium was replaced by serum-free medium to starve cells over night for 24 h. Also over night, black Maxi-Sorp 96 well plates (Nunc) were coated with mouse anti-phosphotyrosine antibody 4G10 at 2 IJg/ml PBS and 4°C, On day 3, the 4G10 antibody solution was removed and Maxi-Sorp wells were blocked with PBS, 0.5% BSA for at lest 4 h at room temperature. In parallel, cells were preincubated with 50 ng/ml, 100 ng/ml, 300 ng/ml, 750 ng/ml, 1 IJg/ml, and 10 IJg/ml of rat anti-Axl antibody 11 B7 or chimeric anti-Axl antibody ch11B7 for 1 h at 37"C and subsequently treated with or without 400 ng/ml Gas6 (R&D Systems) for 10 min at 37"C. Medium was then flicked out and cells were lysed in lysis buffer (50 mM HEPES, pH 7,5, 150 mM NaCl, 1 mM EDTA, 10% glycerine, and 1% Triton X-100) supplemented with phosphatase and protease inhibitors (10 mM Na4P207, 1 mM phenylmethylsulfonyl fluoride, 1 mM orlhovanadate, 1 mM NaF, and 0,5% aprotinin) for 30 min on ice. Meanwhile, blocking buffer was removed and Maxl-Sorp plates were washed 6x with wash buffer (PBS, 0.05% Tween 20), before lysates were transferred and incubated over night at 4°C. After plates were washed 6x with wash buffer on day 4, wells were incubated with biotinylated rat anti-Axl antibody 12B7 at 0.5 IJg/ml PBS for 2 h at room temperature. Plates were washed 6x with wash buffer and AP-conjugated streptavidin (Chemicon#SA110) diluted 1:4,000 in PBS was added to each well and incubated for 30 min at room temperature. Afterwards, wells were washed 6x with wash buffer and AttoPhos substrate solution (Roche #11681982) was added. Using a Victor plate reader (Perkin Elmer), the fluorescence of each well was collected at an excitation wavelength of 430 nm and an emission wavelength of 580nm. **Figure 31** shows representative results of this experiment for the cervical cancer cell line CaSki. As demonstrated by concentration-dependent decrease of the relative AxI phosphorylation, the rat anti-Axl antibody 11 B7 (A) and the chimeric anti-Axl antibody ch11 B7 (B) of the invention were able to block ligand-induced activation of the receptor tyrosine kinase Axl to similar extent. Comparable effects applying the same experimental settings were observed with the melanoma cell line C-8161.

SEQUENCE LISTING

(SEQ ID NO:1) Primer EFF1
ccacgcgccctgtagcggcgcattaagc

(SEQ ID NO:2) Primer EFsmaR
aaacccgggagcttttgcaaaagcctagg

(SEQ ID NO:3) Fragment B
ggtaccacccaagctggctaggtaagcttgctagcgccaccatggtgctgcagacccaggtg
ttcatctccctgctgctgtggatctccggcgcatatggcgatatcgtgatgattaaacgtac
ggtggccgcccccctccgtgttcatcttccccccctccgacgagcagctgaagtccggcaccg
cctccgtggtgtgcctgctgaataacttctaccccagagaggccaaggtgcagtggaaggtg
gacaacgccctgcagtccgggaactcccaggagagcgtgaccgagcaggacagcaaggacag
cacctacagcctgagcagcaccctgaccctgagcaaagccgactacgagaagcacaaggtgt
acgcctgcgaggtgacccaccagggcctgagctcccccgtcaccaagagcttcaacagggggg
gagtgttaggggcccgtttaaacgggtggcatccctgtgacccctccccagtgcctctcctg
gccctggaagttgccactccagtgcccaccagccttgtcctaataaaattaagttgcatcat
tttgtctgactaggtgtccttctataatattatggggtggaggggggtggtatggagcaagg
ggcaagttgggaagacaacctgtagggcctgcggggtctattgggaaccaagctggagtgca
gtggcacaatcttggctcactgcaatctccgcctcctgggttcaagcgattctcctgcctca
gcctcccgagttgttgggattccaggcatgcatgaccaggctcacctaatttttgttttttt
ggtagagacggggtttcaccatattggccaggctggtctccaactcctaatctcaggtgatc
tacccaccttggcctcccaaattgctgggattacaggcgtgaaccactgctccacgcgccct
gtagcggcgcattaagcgcggcgggtgtggtggttacgcgcagcgtgaccgctacacttgcc
agcgccctagcgcccgctcctttcgctttcttcccttcctttctcgccacgttcgccggctt
tccccgtcaagctctaaatcgggggctccctttagggttccgatttagtgctttacggcacc
tcgaccccaaaaaacttgattagggtgatggttcacgtagtgggccatcgccctgatagacg
gtttttcgccctttgacgttggagtccacgttctttaatagtggactcttgttccaaactgg
aacaacactcaaccctatctcggtctattcttttgatttataagggattttgccgatttcgg
cctattggttaaaaaatgagctgatttaacaaaaatttaacgcgaattaattctgtggaatg
tgtgtcagttagggtgtggaaagtccccaggctccccagcaggcagaagtatgcaaagcatg
catctcaattagtcagcaaccaggtgtggaaagtccccaggctccccagcaggcagaagtat
gcaaagcatgcatctcaattagtcagcaaccatagtcccgcccctaactccgcccatcccgc
ccctaactccgcccagttccgcccattctccgccccatggctgactaattttttttatttat
gcagaggccgaggccgcctctgcctctgagctattccagaagtagtgaggaggcttttttgg
aggcctaggcttttgcaaaagctcccggg

(SEQ ID NO:4) human IgG1 signal + human IgG1 constant
tgctagcgccaccatgaaacacctgtggttcttcctcctgctggtggcagctcccagatggg
tgctgagccaggtgcaattgtgcaggcggttagctcagcctccaccaagggcccaagcgtct
tccccctggcaccctcctccaagagcacctctggcggcacagccgccctgggctgcctggtc
aaggactacttccccgaacccgtgaccgtgagctggaactcaggcgccctgaccagcggcgt
gcacaccttccccgctgtcctgcagtcctcaggactctactccctcagcagcgtggtgaccg
tgccctccagcagcttgggcacccagacctacatctgcaacgtgaatcacaagcccagcaac
accaaggtggacaagagagttgagcccaaatcttgtgacaaaactcacacatgcccaccctg
cccagcacctgaactcctggggggaccctcagtcttcctcttccccccaaaacccaaggaca
ccctcatgatctcccggacccctgaggtcacatgcgtggtggtggacgtgagccacgaagac
cctgaggtcaagttcaactggtacgtggacggcgtggaggtgcataatgccaagacaaagcc
ccgggaggagcagtacaacagcacgtaccgggtggtcagcgtcctcaccgtcctgcaccagg
actggctgaatggcaaggagtacaagtgcaaggtctccaacaaagccctcccagcccccatc
gagaaaaccatctccaaagccaaaggccagccccgggaaccacaggtgtacaccctgccccc
atcccgggaggagatgaccaagaaccaggtcagcctgacctgcctggtcaaaggcttctatc
ccagcgacatcgccgtggagtgggagagcaatggccagcccgagaacaactacaagaccacc
cctcccgtgctggactccgacggctccttcttcctctacagcaagctcaccgtggacaagag
caggtggcagcagggcaacgtcttctcatgctccgtgatgcatgaggctctgcacaaccact
acacccagaagagcctctccctgtctcccggcaaatgagatatcgggcccgtttaaacgggt
ggca

(SEQ ID NO:5) h#11B7-T1L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGCTGATCAGCGGCGCCACCAACCTGGCCGCCGGCGTGCCTAGCCGGTTCTC
TGGAACCGGATCTGGCACCGACTTCACCTTCACCATCAGCTCTCTGCAGCCTGAGGACTTCG
CCACCTACTACTGTCTGCAGAGCAAGGAGTCTCCTTGGACCTTCGGCGGAGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:6) h#11B7-T2L light chain(DNA)
ATGGTGCTGCAGACCCAGGTGTTCATCTCCCTGCTGCTGTGGATCTCCGGCGCATATGGCGA

CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGATGATCAGCGGCGCCACCAACCTGGCCGCCGGCGTGCCTAGCCGGTTCTC
TGGAACCGGATCTGGCACCGACTACACCTTCACCATCAGCTCTCTGCAGCCTGAGGACTTCG
CCACCTACTACTGTCTGCAGAGCAAGGAGTCTCCTTGGACCTTCGGCGGAGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:7) h#11B7-T3L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGATGATCAGCGGCGCCACCAACCTGGCCGCCGGCGTGCCTAGCCGGTTCTC
TGGAAGCCGGTCTGGCAGCGACTACACCCTGACCATCAGCTCTCTGCAGCCTGAGGACTTCG
CCACCTACTACTGTCTGCAGAGCAAGGAGTCTCCTTGGACCTTCGGCGGAGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:8) h#11B7-T4L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGCTGATCAGCGGCGCCACCAACCTGGCCGCCGGCGTGCCTAGCCGGTTCTC
TGGAAGCGGATCTGGCAGCGACTTCACCCTGACCATCAGCTCTCTGCAGCCTGAGGACTTCG
CCACCTACTACTGTCTGCAGAGCAAGGAGTCTCCTTGGACCTTCGGCCAGGGCACCAAGCTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG

TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:9) h#11B7-T5L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGATGATCAGCGGCGCCACCAACCTGGCCGCCGGCGTGCCTAGCCGGTTCTC
TGGAAGCGGATCTGGCAGCGACTACACCCTGACCATCAGCTCTCTGCAGCCTGAGGACTTCG
CCACCTACTACTGTCTGCAGAGCAAGGAGTCTCCTTGGACCTTCGGCCAGGGCACCAAGCTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:10) h#11B7-T6L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGATGATCAGCGGCGCCACCAACCTGGCCGCCGGCGTGCCTAGCCGGTTCTC
TGGAAGCGGATCTGGCAGCGACTACACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:11) h#11B7-T7L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGATGATCAGCGGCGCCACCAACCTGGCCGCCGGCGTGCCTAGCCGGTTCTC
TGGAAGCGGATCTGGCAGCGACTACACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:12) h#11B7-T8L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGCTGATCAGCGGCGCCACCAACCTGGCCGCCGGCGTGCCTAGCCGGTTCTC
TGGAAGCGGATCTGGCAGCGACTACACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:13) h#11B7-T9L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGATGATCAGCGGCGCCACCAACCTGGCCGCCGGCGTGCCTAGCCGGTTCTC
TGGAAGCGGATCTGGCAGCGACTTCACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG

GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:14) h#11B7-T10L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGCTGATCAGCGGCGCCACCAACCTGGCCGCCGGCGTGCCTAGCCGGTTCTC
TGGAAGCGGATCTGGCAGCGACTACACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:15) h#11B7-T11L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTTCCAGCAGAAGCCTGGAAAG
GCCCCTAAGCTGCTGATCAGCGGCGCCACCAACCTGGCCGCCGGCGTGCCTAGCCGGTTCTC
TGGAAGCGGATCTGGCAGCGACTTCACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant

region(388-702)

(SEQ ID NO:16) h#11B7-T12L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGATGATCAGCGGCGCCACCAACCTGGCCGCCGGCGTGCCTAGCCGGTTCTC
TGGAAGCGGATCTGGCAGCGACTTCACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:17) h#11B7-T13L light chain(DNA)
ATGGTGCTGCAGACCCAGGTGTTCATCTCCCTGCTGCTGTGGATCTCCGGCGCATATGGCGA
CATCCAGATGACCCAGAGCCCTAGCTCTCTGAGCGCCTCTGTGGGCGACCGGGTGACCATCA
CCTGTCGGGCCAGCCAGGACATCGGCAACTACCTGAGATGGTACCAGCAGAAGCCTGGAAAG
GCCCCTAAGCTGCTGATCAGCGGCGCCACCAACCTGGCCGCCGGCGTGCCTAGCCGGTTCTC
TGGAAGCGGATCTGGCAGCGACTTCACCCTGACCATCAGCTCTCTGCAGCCTGAGGACTTCG
CCACCTACTACTGTCTGCAGAGCAAGGAGTCTCCTTGGACCTTCGGCCAGGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:18) h#11B7-T1L light chain(AA)
MVLQTQVFISLLLWISGAYGDIQMTQSPSSLSASVGDRVTITCRASQDIGNYLRWYQQKPGK
APKLLISGATNLAAGVPSRFSGTGSGTDFTFTISSLQPEDFATYYCLQSKESPWTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC

signal sequence(1-20), variable region(21-129), constant
region(130-234)

(SEQ ID NO:19) h#11B7-T2L light chain(AA)
MVLQTQVFISLLLWISGAYGDIQMTQSPSSLSASVGDRVTITCRASQDIGNYLRWFQQKPGK
APKLMISGATNLAAGVPSRFSGTGSGTDYTFTISSLQPEDFATYYCLQSKESPWTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:20) h#11B7-T3L light chain(AA)
MVLQTQVFISLLLWISGAYGDIQMTQSPSSLSASVGDRVTITCRASQDIGNYLRWFQQKPGK
APKLMISGATNLAAGVPSRFSGSRSGSDYTLTISSLQPEDFATYYCLQSKESPWTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:21) h#11B7-T4L light chain(AA)
MVLQTQVFISLLLWISGAYGDIQMTQSPSSLSASVGDRVTITCRASQDIGNYLRWYQQKPGK
APKLLISGATNLAAGVPSRFSGSGSGSDFTLTISSLQPEDFATYYCLQSKESPWTFGQGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:22) h#11B7-T5L light chain(AA)
MVLQTQVFISLLLWISGAYGDIQMTQSPSSLSASVGDRVTITCRASQDIGNYLRWFQQKPGK
APKLMISGATNLAAGVPSRFSGSGSGSDYTLTISSLQPEDFATYYCLQSKESPWTFGQGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:23) h#11B7-T6L light chain(AA)
MVLQTQVFISLLLWISGAYGDIQMTQSPSSLSASVGDRVTITCRASQDIGNYLRWFQQKPGK
APKLMISGATNLAAGVPSRFSGSGSGSDYTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ

DSKDSTYSLSSTLTLSKADYEKHKVYACEVTHQGLSSPVTKSFNRGEC
signal sequence(1-20), variable region(21-129), constant
region(130-234)

(SEQ ID NO:24) h#11B7-T7L light chain(AA)
MVLQTQVFISLLLWISGAYGDIQMTQSPSSLSASVGDRVTITCRASQDIGNYLRWYQQKPGK
APKLMISGATNLAAGVPSRFSGSGSGSDYTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:25) h#11B7-T8L light chain(AA)
MVLQTQVFISLLLWISGAYGDIQMTQSPSSLSASVGDRVTITCRASQDIGNYLRWFQQKPGK
APKLLISGATNLAAGVPSRFSGSGSGSDYTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:26) h#11B7-T9L light chain(AA)
MVLQTQVFISLLLWISGAYGDIQMTQSPSSLSASVGDRVTITCRASQDIGNYLRWFQQKPGK
APKLMISGATNLAAGVPSRFSGSGSGSDFTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:27) h#11B7-T10L light chain(AA)
MVLQTQVFISLLLWISGAYGDIQMTQSPSSLSASVGDRVTITCRASQDIGNYLRWYQQKPGK
APKLLISGATNLAAGVPSRFSGSGSGSDYTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:28) h#11B7-T11L light chain(AA)
MVLQTQVFISLLLWISGAYGDIQMTQSPSSLSASVGDRVTITCRASQDIGNYLRWFQQKPGK
APKLLISGATNLAAGVPSRFSGSGSGSDFTLTISSLQPEDFATYYCLQSKESPWTFGQGTKV

EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:29) h#11B7-T12L light chain(AA)
MVLQTQVFISLLLWISGAYGDIQMTQSPSSLSASVGDRVTITCRASQDIGNYLRWYQQKPGK
APKLMISGATNLAAGVPSRFSGSGSGSDFTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:30) h#11B7-T13L light chain(AA)
MVLQTQVFISLLLWISGAYGDIQMTQSPSSLSASVGDRVTITCRASQDIGNYLRWYQQKPGK
APKLLISGATNLAAGVPSRFSGSGSGSDFTLTISSLQPEDFATYYCLQSKESPWTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

SEQ ID NO:31) h#11B7-T1H heavy chain(DNA)
ATGAAACACCTGTGGTTCTTCCTCCTGCTGGTGGCAGCTCCCAGATGGGTGCTGAGCCAGAT
CCAGCTGCAGGAGAGCGGCCCTGGCCTGGTGAAGCCTAGCCAGACCCTGAGCCTGACCTGTG
CCGTGTCTGGATACAGCATCACCAGCAACTACTGGGGCTGGATCCGGCAGCCTCCTGGCAAG
GGACTGGAGTGGATCGGCTACATCACCTACAGCGGCTCTACCAGCTACAACCCTAGCCTGAA
GTCTAGAGTGACCATCAGCGTGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT

CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:32) h#11B7-T2H heavy chain(DNA)
ATGAAACACCTGTGGTTCTTCCTCCTGCTGGTGGCAGCTCCCAGATGGGTGCTGAGCCAGAT
CCAGCTGCAGGAGAGCGGCCCTGGCCTGGTGAAGCCTAGCCAGACCCTGAGCCTGACCTGTG
CCGTGTCTGGATACAGCATCACCAGCAACTACTGGGGCTGGATCCGGCAGCCTCCTGGCAAG
GGACTGGAGTGGATGGGCTACATCACCTACAGCGGCTCTACCAGCTACAACCCTAGCCTGAA
GTCTAGAATCAGCATCAGCGTGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:33) h#11B7-T3H heavy chain(DNA)
ATGAAACACCTGTGGTTCTTCCTCCTGCTGGTGGCAGCTCCCAGATGGGTGCTGAGCCAGGT
GCAGCTGCAGGAGAGCGGCCCTGGCCTGGTGAAGCCTAGCCAGACCCTGAGCCTGACCTGTG

CCGTGTCTGGATACAGCATCACCAGCAACTACTGGGGCTGGATCCGGAAGCCTCCTGGCGAC
GGACTGGAGTGGATGGGCTACATCACCTACAGCGGCTCTACCAGCTACAACCCTAGCCTGAA
GTCTAGAATCAGCATCACCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:34) h#11B7-T4H heavy chain(DNA)
ATGAAACACCTGTGGTTCTTCCTCCTGCTGGTGGCAGCTCCCAGATGGGTGCTGAGCCAGGT
GCAGCTGCAGCAGTGGGGCGCCGGCCTGCTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATCGGCTACATCACCTACAGCGGCTCTACCAGCTACAACCCTAGCCTGAA
GTCTAGAGTGACCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCACCGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT

TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:35) h#11B7-T5H heavy chain(DNA)
ATGAAACACCTGTGGTTCTTCCTCCTGCTGGTGGCAGCTCCCAGATGGGTGCTGAGCCAGGT
GCAGCTGCAGCAGTGGGGCGCCGGCCTGCTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATGGGCTACATCACCTACAGCGGCTCTACCAGCTACAACCCTAGCCTGAA
GTCTAGAATCAGCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCACCGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:36) h#11B7-T6H heavy chain(DNA)
ATGAAACACCTGTGGTTCTTCCTCCTGCTGGTGGCAGCTCCCAGATGGGTGCTGAGCCAGGT
GCAGCTGCAGGAGAGCGGCCCTGGCCTGGTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATGGGCTACATCACCTACAGCGGCTCTACCAGCTACAACCCTAGCCTGAA
GTCTAGAATCAGCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:37) h#11B7-T7H heavy chain(DNA)
ATGAAACACCTGTGGTTCTTCCTCCTGCTGGTGGCAGCTCCCAGATGGGTGCTGAGCCAGGT
GCAGCTGCAGGAGAGCGGCCCTGGCCTGGTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATCGGCTACATCACCTACAGCGGCTCTACCAGCTACAACCCTAGCCTGAA
GTCTAGAGTGAGCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA

AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:38) h#11B7-T8H heavy chain(DNA)
ATGAAACACCTGTGGTTCTTCCTCCTGCTGGTGGCAGCTCCCAGATGGGTGCTGAGCCAGGT
GCAGCTGCAGGAGAGCGGCCCTGGCCTGGTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATGGGCTACATCACCTACAGCGGCTCTACCAGCTACAACCCTAGCCTGAA
GTCTAGAATCACCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA

signal sequence(1-57), variable region(58-396), constant
region(397-1386)

(SEQ ID NO:39) h#11B7-T9H heavy chain(DNA)
ATGAAACACCTGTGGTTCTTCCTCCTGCTGGTGGCAGCTCCCAGATGGGTGCTGAGCCAGGT
GCAGCTGCAGGAGAGCGGCCCTGGCCTGGTGAAGCCTAGCGAGACCCTGAGCCTGACCTGTA
CCGTGTCTGGATACAGCATCACCAGCAACTACTGGGGCTGGATCCGGCAGCCTCCTGGCAAG
GGACTGGAGTGGATCGGCTACATCACCTACAGCGGCTCTACCAGCTACAACCCTAGCCTGAA
GTCTAGAGTGACCATCAGCCGGGACACCTCTAAGAACCAGTTCAGCCTGAAGCTGTCTTCTG
TGACCGCCGCCGACACCGCCGTGTACTACTGTGCCATCACCACCTTCTACTACTGGGGACAG
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(397-1386)

(SEQ ID NO:40) h#11B7-T1H heavy chain(AA)
MKHLWFFLLLVAAPRWVLSQIQLQESGPGLVKPSQTLSLTCAVSGYSITSNYWGWIRQPPGK
GLEWIGYITYSGSTSYNPSLKSRVTISVDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK

signal sequence(1-19), variable region(20-132), constant
region(133-462)

(SEQ ID NO:41) h#11B7-T2H heavy chain(AA)
MKHLWFFLLLVAAPRWVLSQIQLQESGPGLVKPSQTLSLTCAVSGYSITSNYWGWIRQPPGK
GLEWMGYITYSGSTSYNPSLKSRISISVDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:42) h#11B7-T3H heavy chain(AA)
MKHLWFFLLLVAAPRWVLSQVQLQESGPGLVKPSQTLSLTCAVSGYSITSNYWGWIRKPPGD
GLEWMGYITYSGSTSYNPSLKSRISITRDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:43) h#11B7-T4H heavy chain(AA)
MKHLWFFLLLVAAPRWVLSQVQLQQWGAGLLKPSETLSLTCTVSGYSITSNYWGWIRQPPGK
GLEWIGYITYSGSTSYNPSLKSRVTISRDTSKNQFSLKLSSVTAADTAVYYCAITTFYYWGQ
GTTVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:44) h#11B7-T5H heavy chain(AA)
MKHLWFFLLLVAAPRWVLSQVQLQQWGAGLLKPSETLSLTCTVSGYSITSNYWGWIRQPPGK
GLEWMGYITYSGSTSYNPSLKSRISISRDTSKNQFSLKLSSVTAADTAVYYCAITTFYYWGQ
GTTVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:45) h#11B7-T6H heavy chain(AA)
MKHLWFFLLLVAAPRWVLSQVQLQESGPGLVKPSETLSLTCTVSGYSITSNYWGWIRQPPGK
GLEWMGYITYSGSTSYNPSLKSRISISRDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:46) h#11B7-T7H heavy chain(AA)
MKHLWFFLLLVAAPRWVLSQVQLQESGPGLVKPSETLSLTCTVSGYSITSNYWGWIRQPPGK
GLEWIGYITYSGSTSYNPSLKSRVSISRDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:47) h#11B7-T8H heavy chain(AA)
MKHLWFFLLLVAAPRWVLSQVQLQESGPGLVKPSETLSLTCTVSGYSITSNYWGWIRQPPGK
GLEWMGYITYSGSTSYNPSLKSRITISRDTSKNQFSLKLSSVTAADTAVYYCAITTFYYWGQ

GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:48) h#11B7-T9H heavy chain(AA)
MKHLWFFLLLVAAPRWVLSQVQLQESGPGLVKPSETLSLTCTVSGYSITSNYWGWIRQPPGK
GLEWIGYITYSGSTSYNPSLKSRVTISRDTSKNQFSLKLSSVTAADTAVYYCAITTFYYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:49) h#11D5-T1L light chain(DNA)
ATGGTGCTGCAGACCCAGGTGTTCATCTCCCTGCTGCTGTGGATCTCCGGCGCATATGGCGA
CGTGCAAATGACCCAGAGCCCTTCCAGCCTGTCCGCCAGCGTGGGCGACAGAGTGACCATCA
CCTGTCGGGCCTCCCAAGACATCGGCAACTACCTGAGCTGGTACCAGCAAAAGCCTGGCAAG
GCCCCTAAGCTTCTGATCTACGGCGCCATCAAGCTGGCCGTGGGAGTGCCTTCCCGGTTTAG
CGGCTCCGGCAGCGGCACCGACTTTACCCTTACCATCTCCAGCCTGCAGCCTGAGGACTTTG
CCACCTACTACTGTCTGCAATACATCCAGTTTCCTCTTACCTTTGGCCAAGGAACCAAGCTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:50) h#11D5-T2L light chain(DNA)
ATGGTGCTGCAGACCCAGGTGTTCATCTCCCTGCTGCTGTGGATCTCCGGCGCATATGGCGA

CATCCAAATGACCCAGAGCCCTTCCAGCCTGTCCGCCAGCGTGGGCGACAGAGTGACCATCA
CCTGTCGGGCCTCCCAAGACATCGGCAACTACCTGAGCTGGTTTCAGCAAAAGCCTGGCAAG
GCCCCTAGACTTATGATCTACGGCGCCATCAAGCTGGCCGTGGGAGTGCCTTCCCGGTTTAG
CGGCTCCGGCAGCGGCACCGACTACACCCTGACCATCTCCAGCCTTCAGCCTGAGGACTTTG
CCACCTACTACTGTCTGCAATACATCCAGTTTCCTCTGACCTTTGGCCAAGGAACCAAGCTT
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:51) h#11D5-T3L light chain(DNA)
ATGGTGCTGCAGACCCAGGTGTTCATCTCCCTGCTGCTGTGGATCTCCGGCGCATATGGCGA
CATCCAAATGACCCAGAGCCCCTCCAGCCTGTCCGCCAGCGTGGGCGACAGAGTGACCATCA
CCTGTCGGGCCTCCCAAGACATCGGCAACTACCTGAGCTGGTTTCAACAGAAGGTGGGCAAG
TCCCCTAGACGGATGATCTACGGCGCCATCAAGCTTGCCGTGGGAGTGCCTAGCAGATTTTC
CGGCAGCCGGTCCGGCAGCGACTACACCCTGACCATCTCCAGCCTGCAACCTGAGGACTTTG
CCATCTACTACTGTCTTCAGTACATCCAATTTCCTCTGACCTTTGGCTCCGGAACCAAGCTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:52) h#11D5-T4L light chain(DNA)
ATGGTGCTGCAGACCCAGGTGTTCATCTCCCTGCTGCTGTGGATCTCCGGCGCATATGGCGA
CATCCAAATGACCCAGAGCCCTTCCAGCCTGTCCGCCAGCGTGGGCGACAGAGTGACCATCA
CCTGTCGGGCCTCCCAAGACATCGGCAACTACCTGAGCTGGTACCAGCAAAAGCCTGGCAAG
GCCCCTAAGAGACTTATCTACGGCGCCATCAAGCTGGCCGTGGGAGTGCCTTCCCGGTTTAG
CGGCTCCGGCAGCGGCACCGAGTTTACCCTGACCATCTCCAGCCTTCAGCCTGAGGACTTTG
CCACCTACTACTGTCTGCAATACATCCAGTTTCCTCTGACCTTTGGCGGAGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG

TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:53) h#11D5-T5L light chain(DNA)
ATGGTGCTGCAGACCCAGGTGTTCATCTCCCTGCTGCTGTGGATCTCCGGCGCATATGGCGA
CATCCAAATGACCCAGAGCCCTTCCAGCCTGTCCGCCAGCGTGGGCGACAGAGTGACCATCA
CCTGTCGGGCCTCCCAAGACATCGGCAACTACCTGAGCTGGTTTCAGCAAAAGCCTGGCAAG
GCCCCTAGACGGATGATCTACGGCGCCATCAAGCTTGCCGTGGGAGTGCCTTCCAGATTTAG
CGGCTCCGGCAGCGGCACCGACTACACCCTGACCATCTCCAGCCTGCAGCCTGAGGACTTTG
CCACCTACTACTGTCTTCAATACATCCAGTTTCCTCTGACCTTTGGCGGAGGCACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:54) h#11D5-T6L light chain(DNA)
ATGGTGCTGCAGACCCAGGTGTTCATCTCCCTGCTGCTGTGGATCTCCGGCGCATATGGCGA
CATCCAAATGACCCAGAGCCCTTCCAGCCTGTCCGCCAGCGTGGGCGACAGAGTGACCATCA
CCTGTCGGGCCTCCCAAGACATCGGCAACTACCTGAGCTGGTTTCAGCAAAAGCCTGGCAAG
GCCCCTAGACGGATGATCTACGGCGCCATCAAGCTTGCCGTGGGAGTGCCTTCCAGATTTAG
CGGCTCCGGCAGCGGCACCGACTACACCCTGACCATCTCCAGCCTGCAGCCTGAGGACTTTG
CCACCTACTACTGTCTTCAATACATCCAGTTTCCTCTGACCTTTGGCCAAGGAACCAAGGTG
GAGATCAAGCGTACGGTGGCCGCCCCCTCCGTGTTCATCTTCCCCCCCTCCGACGAGCAGCT
GAAGTCCGGCACCGCCTCCGTGGTGTGCCTGCTGAATAACTTCTACCCCAGAGAGGCCAAGG
TGCAGTGGAAGGTGGACAACGCCCTGCAGTCCGGGAACTCCCAGGAGAGCGTGACCGAGCAG
GACAGCAAGGACAGCACCTACAGCCTGAGCAGCACCCTGACCCTGAGCAAAGCCGACTACGA
GAAGCACAAGGTGTACGCCTGCGAGGTGACCCACCAGGGCCTGAGCTCCCCCGTCACCAAGA
GCTTCAACAGGGGGGAGTGT
signal sequence(1-60), variable region(61-387), constant
region(388-702)

(SEQ ID NO:55) h#11D5-T1L light chain(AA)
MVLQTQVFISLLLWISGAYGDVQMTQSPSSLSASVGDRVTITCRASQDIGNYLSWYQQKPGK
APKLLIYGAIKLAVGVPSRFSGSGSGTDFTLTISSLQPEDFATYYCLQYIQFPLTFGQGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:56) h#11D5-T2L light chain(AA)
MVLQTQVFISLLLWISGAYGDIQMTQSPSSLSASVGDRVTITCRASQDIGNYLSWFQQKPGK
APRLMIYGAIKLAVGVPSRFSGSGSGTDYTLTISSLQPEDFATYYCLQYIQFPLTFGQGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:57) h#11D5-T3L light chain(AA)
MVLQTQVFISLLLWISGAYGDIQMTQSPSSLSASVGDRVTITCRASQDIGNYLSWFQQKVGK
SPRRMIYGAIKLAVGVPSRFSGSRSGSDYTLTISSLQPEDFAIYYCLQYIQFPLTFGSGTKL
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:58) h#11D5-T4L light chain(AA)
MVLQTQVFISLLLWISGAYGDIQMTQSPSSLSASVGDRVTITCRASQDIGNYLSWYQQKPGK
APKRLIYGAIKLAVGVPSRFSGSGSGTEFTLTISSLQPEDFATYYCLQYIQFPLTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:59) h#11D5-T5L light chain(AA)
MVLQTQVFISLLLWISGAYGDIQMTQSPSSLSASVGDRVTITCRASQDIGNYLSWFQQKPGK
APRRMIYGAIKLAVGVPSRFSGSGSGTDYTLTISSLQPEDFATYYCLQYIQFPLTFGGGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:60) h#11D5-T6L light chain(AA)
MVLQTQVFISLLLWISGAYGDIQMTQSPSSLSASVGDRVTITCRASQDIGNYLSWFQQKPGK
APRRMIYGAIKLAVGVPSRFSGSGSGTDYTLTISSLQPEDFATYYCLQYIQFPLTFGQGTKV
EIKRTVAAPSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
signal sequence(1-20), variable region(21-129), constant
region(130-234)

(SEQ ID NO:61) h#11D5-T1H heavy chain(DNA)
ATGAAACACCTGTGGTTCTTCCTCCTGCTGGTGGCAGCTCCCAGATGGGTGCTGAGCCAAGT
GCAACTGCAAGAGAGCGGCCCTGGCCTGGTGAAGCCTTCCCAAACCCTTAGCCTGACCTGTA
CCGTGAGCGGCTACTCCATCACCAGCAACTACTGGGGCTGGATCAGACAACCTCCTGGCATG
GGCCTGGAGTGGATCGGACACATCACCAATAGCGGCAACACCACCTACAATCCCTCCCTTAA
GAGCCGGGTGACCATCTCCGTGGACACCAGCGAGAACCAATTTTCCCTGAAGCTGTCTTCCG
TGACCCCTGCCGACACCGCCGTGTACTACTGTGCCAAGGGCGCCTTTGACTACTGGGGCCAA
GGAACCCTTGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:62) h#11D5-T2H heavy chain(DNA)
ATGAAACACCTGTGGTTCTTCCTCCTGCTGGTGGCAGCTCCCAGATGGGTGCTGAGCGAGGT
GCAACTGCAAGAGAGCGGCCCTGGCCTGGTGAAGCCTTCCCAAACCCTTAGCCTGACCTGTA

CCGTGTCCGGCTACAGCATCACCTCCAACTACTGGGGCTGGATCAGACAACCTCCTGGCATG
GGCCTGGAGTGGATCGGACACATCACCAATAGCGGCAACACCACCTACAATCCCTCCCTTAA
GAGCCGGATCTCCATCAGCGTGGACACCTCCGAGAACCAATTTAGCCTGAAGCTGTCCAGCG
TGACCCCTGCCGACACCGCCGTGTACTACTGTGCCAAGGGCGCCTTTGACTACTGGGGCCAA
GGAACCCTTGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:63) h#11D5-T3H heavy chain(DNA)
ATGAAACACCTGTGGTTCTTCCTCCTGCTGGTGGCAGCTCCCAGATGGGTGCTGAGCGAGGT
GCAACTGCAAGAGAGCGGCCCTGGCCTGGTGAAGCCTTCCCAAACCCTTAGCCTGACCTGTA
CCGTGTCCGGCTACAGCATCACCTCCAACTACTGGGGCTGGATCAGAAAGTTTCCTGGCAAC
AAGATGGAGTGGATCGGACACATCACCAATAGCGGCAACACCACCTACAATCCCTCCCTGAA
GAGCCGGATCTCCATCAGCCGGGACACCTCCAAGAACCAATTTAGCCTTAAGCTGTCCAGCG
TGACCCCTGCCGACACCGCCGTGTACTACTGTGCCAAGGGCGCCTTTGACTACTGGGGCCAA
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT

TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:64) h#11D5-T4H heavy chain(DNA)
ATGAAACACCTGTGGTTCTTCCTCCTGCTGGTGGCAGCTCCCAGATGGGTGCTGAGCCAAGT
GCAACTGCAACAATGGGGCGCCGGCCTGCTTAAGCCTAGCGAGACCCTGTCCCTGACCTGTA
CCGTGAGCGGCTACTCCATCACCAGCAACTACTGGGGCTGGATCAGACAACCTCCTGGCAAG
GGCCTTGAGTGGATCGGACACATCACCAATTCCGGCAACACCACCTACAATCCCAGCCTGAA
GTCCCGGGTGACCATCAGCAGAGACACCTCCAAGAACCAATTTAGCCTGAAGCTTTCCAGCG
TGACCGCCGCCGACACCGCCGTGTACTACTGTGCCAAGGGCGCCTTTGACTACTGGGGCCAA
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:65) h#11D5-T5H heavy chain(DNA)
ATGAAACACCTGTGGTTCTTCCTCCTGCTGGTGGCAGCTCCCAGATGGGTGCTGAGCGAGGT
GCAACTGCAACAATGGGGCGCCGGCCTGCTTAAGCCTAGCGAGACCCTGTCCCTGACCTGTA
CCGTGAGCGGCTACTCCATCACCAGCAACTACTGGGGCTGGATCAGACAACCTCCTGGCAAG
GGCCTTGAGTGGATCGGACACATCACCAATTCCGGCAACACCACCTACAATCCCAGCCTGAA
GTCCCGGATCAGCATCTCCAGAGACACCAGCAAGAACCAATTTTCCCTGAAGCTTTCCAGCG
TGACCGCCGCCGACACCGCCGTGTACTACTGTGCCAAGGGCGCCTTTGACTACTGGGGCCAA
GGAACCCTGGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA
AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:66) h#11D5-T6H heavy chain(DNA)
ATGAAACACCTGTGGTTCTTCCTCCTGCTGGTGGCAGCTCCCAGATGGGTGCTGAGCGAGGT
GCAACTGCAAGAGAGCGGCCCTGGCCTGGTGAAGCCTTCCGAGACCCTTAGCCTGACCTGTA
CCGTGTCCGGCTACAGCATCACCTCCAACTACTGGGGCTGGATCAGACAACCTCCTGGCAAG
GGCCTGGAGTGGATCGGACACATCACCAATAGCGGCAACACCACCTACAATCCCTCCCTTAA
GAGCCGGATCTCCATCAGCAGAGACACCTCCAAGAACCAATTTAGCCTGAAGCTGTCCAGCG
TGACCGCCGCCGACACCGCCGTGTACTACTGTGCCAAGGGCGCCTTTGACTACTGGGGCCAA
GGAACCCTTGTGACCGTGAGCTCAGCCTCCACCAAGGGCCCAAGCGTCTTCCCCCTGGCACC
CTCCTCCAAGAGCACCTCTGGCGGCACAGCCGCCCTGGGCTGCCTGGTCAAGGACTACTTCC
CCGAACCCGTGACCGTGAGCTGGAACTCAGGCGCCCTGACCAGCGGCGTGCACACCTTCCCC
GCTGTCCTGCAGTCCTCAGGACTCTACTCCCTCAGCAGCGTGGTGACCGTGCCCTCCAGCAG
CTTGGGCACCCAGACCTACATCTGCAACGTGAATCACAAGCCCAGCAACACCAAGGTGGACA

AGAGAGTTGAGCCCAAATCTTGTGACAAAACTCACACATGCCCACCCTGCCCAGCACCTGAA
CTCCTGGGGGGACCCTCAGTCTTCCTCTTCCCCCCAAAACCCAAGGACACCCTCATGATCTC
CCGGACCCCTGAGGTCACATGCGTGGTGGTGGACGTGAGCCACGAAGACCCTGAGGTCAAGT
TCAACTGGTACGTGGACGGCGTGGAGGTGCATAATGCCAAGACAAAGCCCCGGGAGGAGCAG
TACAACAGCACGTACCGGGTGGTCAGCGTCCTCACCGTCCTGCACCAGGACTGGCTGAATGG
CAAGGAGTACAAGTGCAAGGTCTCCAACAAAGCCCTCCCAGCCCCCATCGAGAAAACCATCT
CCAAAGCCAAAGGCCAGCCCCGGGAACCACAGGTGTACACCCTGCCCCCATCCCGGGAGGAG
ATGACCAAGAACCAGGTCAGCCTGACCTGCCTGGTCAAAGGCTTCTATCCCAGCGACATCGC
CGTGGAGTGGGAGAGCAATGGCCAGCCCGAGAACAACTACAAGACCACCCCTCCCGTGCTGG
ACTCCGACGGCTCCTTCTTCCTCTACAGCAAGCTCACCGTGGACAAGAGCAGGTGGCAGCAG
GGCAACGTCTTCTCATGCTCCGTGATGCATGAGGCTCTGCACAACCACTACACCCAGAAGAG
CCTCTCCCTGTCTCCCGGCAAA
signal sequence(1-57), variable region(58-396), constant
region(297-1386)

(SEQ ID NO:67) h#11D5-T1H heavy chain(AA)
MKHLWFFLLLVAAPRWVLSQVQLQESGPGLVKPSQTLSLTCTVSGYSITSNYWGWIRQPPGM
GLEWIGHITNSGNTTYNPSLKSRVTISVDTSENQFSLKLSSVTPADTAVYYCAKGAFDYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:68) h#11D5-T2H heavy chain(AA)
MKHLWFFLLLVAAPRWVLSEVQLQESGPGLVKPSQTLSLTCTVSGYSITSNYWGWIRQPPGM
GLEWIGHITNSGNTTYNPSLKSRISISVDTSENQFSLKLSSVTPADTAVYYCAKGAFDYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:69) h#11D5-T3H heavy chain(AA)
MKHLWFFLLLVAAPRWVLSEVQLQESGPGLVKPSQTLSLTCTVSGYSITSNYWGWIRKFPGN
KMEWIGHITNSGNTTYNPSLKSRISISRDTSKNQFSLKLSSVTPADTAVYYCAKGAFDYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:70) h#11D5-T4H heavy chain(AA)
MKHLWFFLLLVAAPRWVLSQVQLQQWGAGLLKPSETLSLTCTVSGYSITSNYWGWIRQPPGK
GLEWIGHITNSGNTTYNPSLKSRVTISRDTSKNQFSLKLSSVTAADTAVYYCAKGAFDYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:71) h#11D5-T5H heavy chain(AA)
MKHLWFFLLLVAAPRWVLSEVQLQQWGAGLLKPSETLSLTCTVSGYSITSNYWGWIRQPPGK
GLEWIGHITNSGNTTYNPSLKSRISISRDTSKNQFSLKLSSVTAADTAVYYCAKGAFDYWGQ
GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:72) h#11D5-T6H heavy chain(AA)
MKHLWFFLLLVAAPRWVLSEVQLQESGPGLVKPSETLSLTCTVSGYSITSNYWGWIRQPPGK
GLEWIGHITNSGNTTYNPSLKSRISISRDTSKNQFSLKLSSVTAADTAVYYCAKGAFDYWGQ

GTLVTVSSASTKGPSVFPLAPSSKSTSGGTAALGCLVKDYFPEPVTVSWNSGALTSGVHTFP
AVLQSSGLYSLSSVVTVPSSSLGTQTYICNVNHKPSNTKVDKRVEPKSCDKTHTCPPCPAPE
LLGGPSVFLFPPKPKDTLMISRTPEVTCVVVDVSHEDPEVKFNWYVDGVEVHNAKTKPREEQ
YNSTYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRWQQ
GNVFSCSVMHEALHNHYTQKSLSLSPGK
signal sequence(1-19), variable region(20-132), constant
region(133-462)

(SEQ ID NO:73) h#11B7-T14L light chain variable region(AA)
DIQMTQSPSSLSASVGDRVTITCRASQDIGNYLRWYQQKPGKAPKLMISGATNLAAGVPSRF
SGSGSGSDYTLTISSLQPEDFATYYCLQSKESPWTFGQGTKLEIKRT

(SEQ ID NO:74) h#11B7-T15L light chain variable region(AA)
h#11B7-T15L
DIQMTQSPSSLSASVGDRVTITCRASQDIGNYLRWFQQKPGKAPKLLISGATNLAAGVPSRF
SGSGSGSDYTLTISSLQPEDFATYYCLQSKESPWTFGQGTKLEIKRT

(SEQ ID NO:75) h#11B7-T16L light chain variable region(AA)
h#11B7-T16L
DIQMTQSPSSLSASVGDRVTITCRASQDIGNYLRWFQQKPGKAPKLMISGATNLAAGVPSRF
SGSGSGSDFTLTISSLQPEDFATYYCLQSKESPWTFGQGTKLEIKRT

(SEQ ID NO:76) h#11B7-T17L light chain variable region(AA)
h#11B7-T17L
DIQMTQSPSSLSASVGDRVTITCRASQDIGNYLRWYQQKPGKAPKLLISGATNLAAGVPSRF
SGSGSGSDYTLTISSLQPEDFATYYCLQSKESPWTFGQGTKLEIKRT

(SEQ ID NO:77) h#11B7-T18L light chain variable region(AA)
h#11B7-T18L
DIQMTQSPSSLSASVGDRVTITCRASQDIGNYLRWFQQKPGKAPKLLISGATNLAAGVPSRF
SGSGSGSDFTLTISSLQPEDFATYYCLQSKESPWTFGQGTKLEIKRT

(SEQ ID NO:78) h#11B7-T19L light chain variable region(AA)
h#11B7-T19L
DIQMTQSPSSLSASVGDRVTITCRASQDIGNYLRWYQQKPGKAPKLMISGATNLAAGVPSRF
SGSGSGSDFTLTISSLQPEDFATYYCLQSKESPWTFGQGTKLEIKRT

(SEQ ID NO:79)h#11B7-T20L light chain variable region(AA)

h#11B7-T20L
DIQMTQSPSSLSASVGDRVTITCRASQDIGNYLRWYQQKPGKAPKLLISGATNLAAGVPSRF
SGSGSGSDFTLTISSLQPEDFATYYCLQSKESPWTFGQGTKLEIKRT

(SEQ ID NO:80) h#11B7-T10H heavy chain variable region(AA)
QVQLQQWGAGLLKPSETLSLTCTVSGYSITSNYWGWIRQPPGKGLEWIGYITYSGSTSYNPS
LKSRVSISRDTSKNQFSLKLSSVTAADTAVYYCAITTFYYWGQGTTVTVSS

(SEQ ID NO:81) h#11B7-T11H heavy chain variable region(AA)
QVQLQQWGAGLLKPSETLSLTCTVSGYSITSNYWGWIRQPPGKGLEWMGYITYSGSTSYNPS
LKSRITISRDTSKNQFSLKLSSVTAADTAVYYCAITTFYYWGQGTTVTVSS

(SEQ ID NO:82) h#11B7-T12H heavy chain variable region(AA)
QVQLQQWGAGLLKPSETLSLTCTVSGYSITSNYWGWIRQPPGKGLEWIGYITYSGSTSYNPS
LKSRVTISRDTSKNQFSLKLSSVTAADTAVYYCAITTFYYWGQGTTVTVSS

(SEQ ID NO:83) h#11D5-T7L light chain variable region(AA)
DIQMTQSPSSLSASVGDRVTITCRASQDIGNYLSWYQQKPGKAPKRLIYGAIKLAVGVPSRF
SGSGSGTEFTLTISSLQPEDFATYYCLQYIQFPLTFGQGTKVEIKRT

(SEQ ID NO:84) h#11D5-T8L light chain variable region(AA)
DIQMTQSPSSLSASVGDRVTITCRASQDIGNYLSWFQQKPGKAPKRLIYGAIKLAVGVPSRF
SGSGSGTEFTLTISSLQPEDFATYYCLQYIQFPLTFGQGTKVEIKRT

(SEQ ID NO:85) h#11D5-T9L light chain variable region(AA)
DIQMTQSPSSLSASVGDRVTITCRASQDIGNYLSWYQQKPGKAPRRLIYGAIKLAVGVPSRF
SGSGSGTEFTLTISSLQPEDFATYYCLQYIQFPLTFGQGTKVEIKRT

(SEQ ID NO:86) h#11D5-T10L light chain variable region(AA)
DIQMTQSPSSLSASVGDRVTITCRASQDIGNYLSWYQQKPGKAPKRMIYGAIKLAVGVPSRF
SGSGSGTEFTLTISSLQPEDFATYYCLQYIQFPLTFGQGTKVEIKRT

(SEQ ID NO:87) h#11D5-T11L light chain variable region(AA)
DIQMTQSPSSLSASVGDRVTITCRASQDIGNYLSWYQQKPGKAPKRLIYGAIKLAVGVPSRF
SGSGSGTDFTLTISSLQPEDFATYYCLQYIQFPLTFGQGTKVEIKRT

(SEQ ID NO:88) h#11D5-T12L light chain variable region(AA)
DIQMTQSPSSLSASVGDRVTITCRASQDIGNYLSWYQQKPGKAPKRLIYGAIKLAVGVPSRF
SGSGSGTEYTLTISSLQPEDFATYYCLQYIQFPLTFGQGTKVEIKRT

(SEQ ID NO:89) h#11D5-T13L light chain variable region(AA)
DIQMTQSPSSLSASVGDRVTITCRASQDIGNYLSWFQQKPGKAPRRLIYGAIKLAVGVPSRF
SGSGSGTEFTLTISSLQPEDFATYYCLQYIQFPLTFGQGTKVEIKRT

(SEQ ID NO:90) h#11D5-T14L light chain variable region(AA)
DIQMTQSPSSLSASVGDRVTITCRASQDIGNYLSWFQQKPGKAPKRMIYGAIKLAVGVPSRF
SGSGSGTEFTLTISSLQPEDFATYYCLQYIQFPLTFGQGTKVEIKRT

(SEQ ID NO:91) h#11D5-T15L light chain variable region(AA)
DIQMTQSPSSLSASVGDRVTITCRASQDIGNYLSWFQQKPGKAPKRLIYGAIKLAVGVPSRF
SGSGSGTDFTLTISSLQPEDFATYYCLQYIQFPLTFGQGTKVEIKRT

(SEQ ID NO:92) h#11D5-T16L light chain variable reigon(AA)
DIQMTQSPSSLSASVGDRVTITCRASQDIGNYLSWFQQKPGKAPKRLIYGAIKLAVGVPSRF
SGSGSGTEYTLTISSLQPEDFATYYCLQYIQFPLTFGQGTKVEIKRT

(SEQ ID NO:93) h#11D5-T17L light chain variable region(AA)
DIQMTQSPSSLSASVGDRVTITCRASQDIGNYLSWYQQKPGKAPRRMIYGAIKLAVGVPSRF
SGSGSGTEFTLTISSLQPEDFATYYCLQYIQFPLTFGQGTKVEIKRT

(SEQ ID NO:94) h#11D5-T18L light chain variable region(AA)
DIQMTQSPSSLSASVGDRVTITCRASQDIGNYLSWYQQKPGKAPRRLIYGAIKLAVGVPSRF
SGSGSGTDFTLTISSLQPEDFATYYCLQYIQFPLTFGQGTKVEIKRT

(SEQ ID NO:95) h#11D5-T19L light chain variable region(AA)
DIQMTQSPSSLSASVGDRVTITCRASQDIGNYLSWYQQKPGKAPRRLIYGAIKLAVGVPSRF
SGSGSGTEYTLTISSLQPEDFATYYCLQYIQFPLTFGQGTKVEIKRT

(SEQ ID NO:96) h#11D5-T20L light chain variable region(AA)
DIQMTQSPSSLSASVGDRVTITCRASQDIGNYLSWYQQKPGKAPKRMIYGAIKLAVGVPSRF
SGSGSGTDFTLTISSLQPEDFATYYCLQYIQFPLTFGQGTKVEIKRT

(SEQ ID NO:97) h#11D5-T21L light chain variable region(AA)
DIQMTQSPSSLSASVGDRVTITCRASQDIGNYLSWYQQKPGKAPKRMIYGAIKLAVGVPSRF
SGSGSGTEYTLTISSLQPEDFATYYCLQYIQFPLTFGQGTKVEIKRT

(SEQ ID NO:98) h#11D5-T22L light chain variable region(AA)
DIQMTQSPSSLSASVGDRVTITCRASQDIGNYLSWYQQKPGKAPKRLIYGAIKLAVGVPSRF

SGSGSGTDYTLTISSLQPEDFATYYCLQYIQFPLTFGQGTKVEIKRT

(SEQ ID NO:99) h#11D5-T23L light chain variable region(AA)
DIQMTQSPSSLSASVGDRVTITCRASQDIGNYLSWFQQKPGKAPRRMIYGAIKLAVGVPSRF
SGSGSGTEFTLTISSLQPEDFATYYCLQYIQFPLTFGQGTKVEIKRT

(SEQ ID NO:100) h#11D5-T24L light chain variable region(AA)
DIQMTQSPSSLSASVGDRVTITCRASQDIGNYLSWFQQKPGKAPRRLIYGAIKLAVGVPSRF
SGSGSGTDFTLTISSLQPEDFATYYCLQYIQFPLTFGQGTKVEIKRT

(SEQ ID NO:101) h#11D5-T25L light chain variable region(AA)
DIQMTQSPSSLSASVGDRVTITCRASQDIGNYLSWFQQKPGKAPRRLIYGAIKLAVGVPSRF
SGSGSGTEYTLTISSLQPEDFATYYCLQYIQFPLTFGQGTKVEIKRT

(SEQ ID NO:102) h#11D5-T26L light chain variable region(AA)
DIQMTQSPSSLSASVGDRVTITCRASQDIGNYLSWFQQKPGKAPKRMIYGAIKLAVGVPSRF
SGSGSGTDFTLTISSLQPEDFATYYCLQYIQFPLTFGQGTKVEIKRT

(SEQ ID NO:103) h#11D5-T27L light chain variable region(AA)
DIQMTQSPSSLSASVGDRVTITCRASQDIGNYLSWFQQKPGKAPKRMIYGAIKLAVGVPSRF
SGSGSGTEYTLTISSLQPEDFATYYCLQYIQFPLTFGQGTKVEIKRT

(SEQ ID NO:104) h#11D5-T28L light chain variable region(AA)
DIQMTQSPSSLSASVGDRVTITCRASQDIGNYLSWFQQKPGKAPKRLIYGAIKLAVGVPSRF
SGSGSGTDYTLTISSLQPEDFATYYCLQYIQFPLTFGQGTKVEIKRT

(SEQ ID NO:105) h#11D5-T29L light chain variable region(AA)
DIQMTQSPSSLSASVGDRVTITCRASQDIGNYLSWYQQKPGKAPRRMIYGAIKLAVGVPSRF
SGSGSGTDFTLTISSLQPEDFATYYCLQYIQFPLTFGQGTKVEIKRT

(SEQ ID NO:106) h#11D5-T30L light chain variable region(AA)
DIQMTQSPSSLSASVGDRVTITCRASQDIGNYLSWYQQKPGKAPRRMIYGAIKLAVGVPSRF
SGSGSGTEYTLTISSLQPEDFATYYCLQYIQFPLTFGQGTKVEIKRT

(SEQ ID NO:107) h#11D5-T31L light chain variable region(AA)
DIQMTQSPSSLSASVGDRVTITCRASQDIGNYLSWYQQKPGKAPRRLIYGAIKLAVGVPSRF
SGSGSGTDYTLTISSLQPEDFATYYCLQYIQFPLTFGQGTKVEIKRT

(SEQ ID NO:108) h#11D5-T32L light chain variablre region(AA)

DIQMTQSPSSLSASVGDRVTITCRASQDIGNYLSWYQQKPGKAPKRMIYGAIKLAVGVPSRF
SGSGSGTDYTLTISSLQPEDFATYYCLQYIQFPLTFGQGTKVEIKRT

(SEQ ID NO:109) h#11D5-T33L light chain variable region(AA)
DIQMTQSPSSLSASVGDRVTITCRASQDIGNYLSWFQQKPGKAPRRMIYGAIKLAVGVPSRF
SGSGSGTDFTLTISSLQPEDFATYYCLQYIQFPLTFGQGTKVEIKRT

(SEQ ID NO:110) h#11D5-T34L light chain variable region(AA)
DIQMTQSPSSLSASVGDRVTITCRASQDIGNYLSWFQQKPGKAPRRMIYGAIKLAVGVPSRF
SGSGSGTEYTLTISSLQPEDFATYYCLQYIQFPLTFGQGTKVEIKRT

(SEQ ID NO:111) h#11D5-T35L light chain variable region(AA)
DIQMTQSPSSLSASVGDRVTITCRASQDIGNYLSWFQQKPGKAPRRLIYGAIKLAVGVPSRF
SGSGSGTDYTLTISSLQPEDFATYYCLQYIQFPLTFGQGTKVEIKRT

(SEQ ID NO:112) h#11D5-T36L light chain variable region(AA)
DIQMTQSPSSLSASVGDRVTITCRASQDIGNYLSWFQQKPGKAPKRMIYGAIKLAVGVPSRF
SGSGSGTDYTLTISSLQPEDFATYYCLQYIQFPLTFGQGTKVEIKRT

(SEQ ID NO:113) h#11D5-T37L light chain variable region(AA)
DIQMTQSPSSLSASVGDRVTITCRASQDIGNYLSWYQQKPGKAPRRMIYGAIKLAVGVPSRF
SGSGSGTDYTLTISSLQPEDFATYYCLQYIQFPLTFGQGTKVEIKRT

(SEQ ID NO:114) h#11D5-T7H heavy chain variable region(AA)
QVQLQESGPGLVKPSETLSLTCTVSGYSITSNYWGWIRQPPGKGLEWIGHITNSGNTTYNPS
LKSRVTISRDTSKNQFSLKLSSVTAADTAVYYCAKGAFDYWGQGTLVTVSS

(SEQ ID NO:115) h#11D5-T8H heavy chain variable region(AA)
EVQLQESGPGLVKPSETLSLTCTVSGYSITSNYWGWIRQPPGKGLEWIGHITNSGNTTYNPS
LKSRVTISRDTSKNQFSLKLSSVTAADTAVYYCAKGAFDYWGQGTLVTVSS

(SEQ ID NO:116) h#11D5-T9H heavy chain variable region(AA)
QVQLQESGPGLVKPSETLSLTCTVSGYSITSNYWGWIRQPPGKGLEWIGHITNSGNTTYNPS
LKSRITISRDTSKNQFSLKLSSVTAADTAVYYCAKGAFDYWGQGTLVTVSS

(SEQ ID NO:117) h#11D5-T10H heavy chain variable region(AA)
QVQLQESGPGLVKPSETLSLTCTVSGYSITSNYWGWIRQPPGKGLEWIGHITNSGNTTYNPS
LKSRVSISRDTSKNQFSLKLSSVTAADTAVYYCAKGAFDYWGQGTLVTVSS

(SEQ ID NO:118) h#11D5-T11H heavy chain variable region(AA)
EVQLQESGPGLVKPSETLSLTCTVSGYSITSNYWGWIRQPPGKGLEWIGHITNSGNTTYNPS
LKSRITISRDTSKNQFSLKLSSVTAADTAVYYCAKGAFDYWGQGTLVTVSS

(SEQ ID NO:119) h#11D5-T12H heavy chain variable region(AA)
EVQLQESGPGLVKPSETLSLTCTVSGYSITSNYWGWIRQPPGKGLEWIGHITNSGNTTYNPS
LKSRVSISRDTSKNQFSLKLSSVTAADTAVYYCAKGAFDYWGQGTLVTVSS

(SEQ ID NO:120) h#11D5-T13H heavy chain variable region(AA)
QVQLQESGPGLVKPSETLSLTCTVSGYSITSNYWGWIRQPPGKGLEWIGHITNSGNTTYNPS
LKSRISISRDTSKNQFSLKLSSVTAADTAVYYCAKGAFDYWGQGTLVTVSS

(SEQ ID NO:121) 11B7 Light chain CDRL4
RASQDIGNYLR

(SEQ ID NO:122) 11B7 Light chain CDRL5
GATNLAA

(SEQ ID NO:123) 11B7 Light chain CDRL6
LQSKESPWT

(SEQ ID NO:124) 11B7 Heavy chain CDRH1
SNYWG

(SEQ ID NO:125) 11B7 Heavy chain CDRH2
YITYSGSTSYNPSLKS

(SEQ ID NO:126) 11B7 Heavy chain CDRH3
TTFYY

(SEQ ID NO:127) 11D5 Light chain CDRL4
RASQDIGNYLS

(SEQ ID NO:128) 11D5 Light chain CDRL5

GAIKLAV

(SEQ ID NO:129) 11D5 Light chain CDRL6

LQYIQFPLT

(SEQ ID NO:130) 11D5 Light chain CDRH1

SNYWG

(SEQ ID NO:131) 11D5 Light chain CDRH2

HITNSGNTTYNPSLKS

(SEQ ID NO:132) 11D5 Light chain CDRH3

GAFDY

(SEQ ID NO:133) Leader Sequence (nucleotide sequence)

ATGGGTGACAATGACATCCACTTTGCCTTTCTCTCCACAGGTGTGCATTCC

(SEQ ID NO:134) Leader Sequence (amino acid sequence)

MGDNDIHFAFLSTGVHS

(SEQ ID NO:135) #11B7-chimeric light chain

D I Q M T Q A P S S L P A S L G D R V T I T C R A S Q D I G N Y
L R W F Q Q K P G K S P R L M I S G A T N L A A G V P S R F S G
S R S G S D Y S L T I S S L E S E D M A D Y Y C L Q S K E S P W
T F G G G T K L E L K R T V A A P S V F I F P P S D E Q L K S G
T A S V V C L L N N F Y P R E A K V Q W K V D N A L Q S G N S Q
E S V T E Q D S K D S T Y S L S S T L T L S K A D Y E K H K V Y
A C E V T H Q G L S S P V T K S F N R G E C

(SEQ ID NO:136) #11B7-chimeric heavy chain

E V Q L Q E S G P G L V K P S Q S L S L T C S V T G Y S I T S N
Y W G W I R K F P G D K M E W M G Y I T Y S G S T S Y N P S L K
S R I S I T R D T S K N Q F F L Q L N S V T S E D T A T Y Y C A
I T T F Y Y W G Q G V M V T V S S A S T K G P S V F P L A P S S
K S T S G G T A A L G C L V K D Y F P E P V T V S W N S G A L T
S G V H T F P A V L Q S S G L Y S L S S V V T V P S S S L G T Q
T Y I C N V N H K P S N T K V D K K V E P K S C D K T H T C P P
C P A P E L L G G P S V F L F P P K P K D T L M I S R T P E V T
C V V V D V S H E D P E V K F N W Y V D G V E V H N A K T K P R
E E Q Y N S T Y R V V S V L T V L H Q D W L N G K E Y K C K V S
N K A L P A P I E K T I S K A K G Q P R E P Q V Y T L P P S R D
E L T K N Q V S L T C L V K G F Y P S D I A V E W E S N G Q P E
N N Y K T T P P V L D S D G S F F L Y S K L T V D K S R W Q Q G
N V F S C S V M H E A L H N H Y T Q K S L S L S P G K

(SEQ ID NO:137) #11D5-chimeric light chain

D I Q M T Q S P S S M S T S L G D R V T I T C R A S Q D I G N Y
L S W F Q Q K V G K S P R R M I Y G A I K L A V G V P S R F S G
S R S G S D Y S L T I S S L E S E D M A I Y Y C L Q Y I Q F P L
T F G S G T K L E L K R T V A A P S V F I F P P S D E Q L K S G
T A S V V C L L N N F Y P R E A K V Q W K V D N A L Q S G N S Q
E S V T E Q D S K D S T Y S L S S T L T L S K A D Y E K H K V Y
A C E V T H Q G L S S P V T K S F N R G E C

(SEQ ID NO:138) #11D5-chimeric heavy chain

E V Q L Q E S G P G L V K P S Q S L S L T C S V T G Y S I T S N
Y W G W I R K F P G N K M E W I G H I T N S G N T T Y N P S L K
S R I S I S R D T S R N Q F F L Q L N S V T T E D T A T Y Y C A
K G A F D Y W G Q G V M V T V S S A S T K G P S V F P L A P S S
K S T S G G T A A L G C L V K D Y F P E P V T V S W N S G A L T
S G V H T F P A V L Q S S G L Y S L S S V V T V P S S S L G T Q
T Y I C N V N H K P S N T K V D K K V E P K S C D K T H T C P P
C P A P E L L G G P S V F L F P P K P K D T L M I S R T P E V T
C V V V D V S H E D P E V K F N W Y V D G V E V H N A K T K P R
E E Q Y N S T Y R V V S V L T V L H Q D W L N G K E Y K C K V S

N K A L P A P I E K T I S K A K G Q P R E P Q V Y T L P P S R D
E L T K N Q V S L T C L V K G F Y P S D I A V E W E S N G Q P E
N N Y K T T P P V L D S D G S F F L Y S K L T V D K S R W Q Q G
N V F S C S V M H E A L H N H Y T Q K S L S L S P G K

(SEQ ID NO:139) human Axl (Accession No.P_30530 of NCBI protein
database)
MGRVPLAWCLALCGWACMAPRGTQAEESPFVGNPGNITGARGLTGTLRCQLQVQGEPPEVHW
LRDGQILELADSTQTQVPLGEDEQDDWIVVSQLRITSLQLSDTGQYQCLVFLGHQTFVSQPG
YVGLEGLPYFLEEPEDRTVAANTPFNLSCQAQGPPEPVDLLWLQDAVPLATAPGHGPQRSLH
VPGLNKTSSFSCEAHNAKGVTTSRTATITVLPQQPRNLHLVSRQPTELEVAWTPGLSGIYPL
THCTLQAVLSDDGMGIQAGEPDPPEEPLTSQASVPPHQLRLGSLHPHTPYHIRVACTSSQGP
SSWTHWLPVETPEGVPLGPPENISATRNGSQAFVHWQEPRAPLQGTLLGYRLAYQGQDTPEV
LMDIGLRQEVTLELQGDGSVSNLTVCVAAYTAAGDGPWSLPVPLEAWRPGEAQPVHQLVKEP
STPAFSWPWWYVLLGAVVAAACVLILALFLVHRRKKETRYGEVFEPTVERGELVVRYRVRKS
YSRRTTEATLNSLGISEELKEKLRDVMVDRHKVALGKTLGEGEFGAVMEGQLNQDDSILKVA
VKTMKIAICTRSELEDFLSEAVCMKEFDHPNVMRLIGVCFQGSERESFPAPVVILPFMKHGD
LHSFLLYSRLGDQPVYLPTQMLVKFMADIASGMEYLSTKRFIHRDLAARNCMLNENMSVCVA
DFGLSKKIYNGDYYRQGRIAKMPVKWIAIESLADRVYTSKSDVWSFGVTMWEIATRGQTPYP
GVENSEIYDYLRQGNRLKQPADCLDGLYALMSRCWELNPQDRPSFTELREDLENTLKALPPA
QEPDEILYVNMDEGGGYPEPPGAAGGADPPTQPDPKDSCSCLTAAEVHPAGRYVLCPSTTPS
PAQPADRGSPAAPGQEDGA

(SEQ ID NO:140) 11B7 light chain variable region
DIQMTQAPSSLPASLGDRVTITCRASQDIGNYLRWFQQKPGKSPRLMISGATNLAAGVPSRF
SGSRSGSDYSLTISSLESEDMADYYCLQSKESPWTFGGGTKLELKR

(SEQ ID No:141) 11B7 heavy chain variable region
EVQLQESGPGLVKPSQSLSLTCSVTGYSITSNYWGWIRKFPGDKMEWMGYITYSGSTSYNPS
LKSRISITRDTSKNQFFLQLNSVTSEDTATYYCAITTFYYWGQGVMVTVSS

(SEQ ID NO:142) 11D5 light chain variable region
DIQMTQSPSSMSTSLGDRVTITCRASQDIGNYLSWFQQKVGKSPRRMIYGAIKLAVGVPSRF
SGSRSGSDYSLTISSLESEDMAIYYCLQYIQFPLTFGSGTKLELKR

(SEQ ID NO:143) 11D5 heavy chain variable region
EVQLQESGPGLVKPSQSLSLTCSVTGYSITSNYWGWIRKFPGNKMEWIGHITNSGNTTYNPS
LKSRISISRDTSRNQFFLQLNSVTTEDTATYYCAKGAFDYWGQGVMVTVSS

## Claims

1. A monoclonal humanized antibody that binds to the extracellular domain of AXL and at least partially inhibits AXL activity.

2. The monoclonal humanized antibody of claim 1, which reduces and/or blocks AXL-mediated signal transduction, AXL phosphorylation, cell proliferation, angiogenesis, cell migration, tumor metastasis and/or AXL mediated anti-apoptosis.

3. The monoclonal humanized antibody of claims 1 or 2, which reduces and/or blocks ligand induced phosphorylation of AXL downstream signaling molecules such as ERK1/2, AKT, GSK-3β, TSC2, mTOR and/or S6K1.

4. The monoclonal humanized antibody according to anyone of claims 1-3, which is derived from one of the chimeric anti-AXL antibodies 11 B7 (SEQ ID NO: 135, 136) or 11D5 (SEQ ID NO: 137, 138) and which contains at least one mutation in the frame work region of at least one variable domain of 11 B7 or 11D5.

5. The monoclonal humanized antibody of claims 1 to 4, which comprises a heavy chain amino acid sequence selected from the group consisting of SEQ ID NO:40 to SEQ ID NO:48, or at least the variable domain thereof, or an amino acid sequence having a sequence identity of at least 90% thereto,
or a variable domain of a heavy chain amino acid sequence selected from the group consisting of SEQ ID NO:80 to SEQ ID NO:82, or an amino acid sequence having a sequence identity of at least 90% thereto and/or a light chain amino acid sequence selected from the group consisting of SEQ ID NO: 18 to SEQ ID NO:30, or at least the variable domain thereof or an amino acid sequence having a sequence identity of at least 90% thereto,
or a variable domain of a light chain amino acid sequence selected from the group consisting of SEQ ID NO: 73 to SEQ ID NO:79, or an amino acid sequence having a sequence identity of at least 90% thereto,
or a fragment thereof recognizing the same epitope on the extracellular domain of AXL.

6. The monoclonal humanized antibody of claim 5, selected from the group of humanized h#11B7 antibodies selected from the group consisting of h#11B7-T1, h#11B7-T2, h#11B7-T3, h#11B7-T4, h#11B7-T5, h#11B7-T6, h#11B7-T7, h#11B7-T8, h#11B7-T9, h#11B7-T10, h#11B7-T11, h#11B7-T12, h#11B7-T13, h#11B7-T14, h#11B7-T15, h#11B7-T16, h#11B7-T17, h#11B7-T18, h#11B7-T19, h#11B7-T20, h#11B7-T21, h#11B7-T22, h#11B7-T23, h#11B7-T24, h#11B7-T25, h#11B7-T26, h#11B7-T27 or h#11B7-T28.

7. The monoclonal humanized antibody of claims 1 to 4, which comprises a heavy chain amino acid sequence selected from the group consisting of SEQ ID NO:67 to SEQ ID NO:72, or at least the variable domain thereof or an amino acid sequence having a sequence identity of at least 90% thereto,
or a variable domain of a heavy chain amino acid sequence selected from the group consisting of SEQ ID NO:114 to SEQ ID NO:120 or an amino acid sequence having a sequence identity of at least 90% thereto
and/or a light chain amino acid sequence selected from the group consisting of SEQ ID NO:55 to SEQ ID NO:60, or at least the variable domain thereof or an amino acid sequence having a sequence identity of at least 90% thereto,
or a variable domain of a light chain amino acid sequence selected from the group consisting of SEQ ID NO:83 to SEQ ID NO:113 or an amino acid sequence having a sequence identity of at least 90% thereto,
or a fragment thereof recognizing the same epitope on the extracellular domain of AXL.

8. The monoclonal humanized antibody of claim 7, selected from the group of humanized h#11D5 antibodies selected from the group consisting of h#11D5-T1, h#11D5-T2, h#11D5-T3, h#11D5-T4, h#11D5-T5 or h#11D5-T6.

9. The monoclonal humanized antibody according to anyone of claims 1-8, which is coupled to a labeling group.

10. The monoclonal humanized antibody according to anyone of claims 1-9, which is coupled to an effector-group.

11. An isolated nucleic acid molecule selected from the group consisting of:
(a) a nucleic acid sequence encoding an monoclonal antibody, antibody fragment or a derivative thereof of any of claims 1-10,
(b) a nucleic acid sequence as shown in in one of the SEQ ID NOs. selected from the group consisting of SEQ ID NO:5 to SEQ ID NO:17, SEQ ID NO:31 to SEQ ID NO:39, SEQ ID NO:49 to SEQ ID NO: 54 and SEQ ID NO:61 to SEQ ID NO:66
(c) a nucleic acid sequence encoding a polypeptide selected from the group consisting of SEQ ID NO:18 to SEQ ID NO:30, SEQ ID NO:40 to SEQ ID NO:48, SEQ ID NO:55 to SEQ ID NO:60, SEQ ID NO:66 to SEQ ID NO:73, SEQ ID NO:73 to SEQ ID NO:120 and SEQ ID NO:121 to SEQ ID NO:132.
(d) a nucleic acid complementary to any of the sequences in (a) to (c), or
(e) a nucleic acid sequence capable of hybridizing to (a), (b), (c) or (d) under stringent conditions and encoding a polypeptide, wherein an antibody or functional fragment thereof comprising said polypeptide binds to the extracellular domain of AXL.

12. A vector comprising a nucleic acid sequence of claim 11.

13. The vector according to claim 12, which is an expression vector and the nucleic acid sequence is operably linked to a control sequence.

14. A host comprising the vector of claim 12 or 13.

15. The host of claim 14 which is a human, bacteria, animal, fungal, amphibian or plant cell.

16. The host of claim 14 which is a non-human transgenic animal.

17. A process of manufacturing a monoclonal humanized antibody according to anyone of claims 1-12 comprising the step of obtaining said polypeptide from the host of claim 14, 15 or 16.

18. A pharmaceutical composition comprising a humanized anti-AXL-antibody, preferably the monoclonal humanized antibody of anyone of claims 1-10, the nucleic acid molecule of claim 11 the vector of claim 12 or 13, the host of claim 14, 15 or 16, or a polypeptide generated by the process of claim 17.

19. The pharmaceutical composition of claim 18 comprising pharmaceutically acceptable carriers, diluents and/or adjuvants.

20. The pharmaceutical composition according to claim 18 or 19, comprising a further active agent.

21. The pharmaceutical composition according to anyone of claims 18 to 20, for the diagnosis, prevention or treatment of a hyperproliferative disease.

22. The pharmaceutical composition according to anyone of claims 18 to 21, wherein said hyperproliferative disease is associated with AXL expression, overexpression and/or hyperactivity.

23. The pharmaceutical composition of claim 22, wherein said hyperproliferative disease is selected from the group consisting of breast cancer, lung cancer and other AXL expressing or overexpressing cancers, and formation of tumor metastases.

24. The monoclonal humanized antibody according to anyone of claims 1-10, for the diagnosis, prevention or treatment of a hyperproliferative disease.

25. Use of the monoclonal humanized antibody according to anyone of claims 1-10 for the manufacture of a pharmaceutical composition for the diagnosis, prevention or treatment of a hyperproliferative disease.

26. The use according to claim 24 or 25, wherein said hyperproliferative disease is a hyperproliferative disease as defined in anyone of claims 22 or 23.

27. A method for diagnosing a condition associated with the expression of AXL, comprising contacting a sample with an monoclonal humanized antibody according to anyone of claims 1-10, and detecting the presence of AXL.

28. The method according to claim 27, wherein the condition is a hyperproliferative disease as defined in anyone of claims 22 or 23.

29. A method for preventing or treating a condition associated with the expression of AXL in a patient, comprising administering to a patient in need thereof an effective amount of at least the monoclonal humanized antibody according to anyone of claims 1-10.

30. The method according to claim 29, wherein the condition is a hyperproliferative disease as defined in anyone of claims 27 or 29.

31. The method according to claim 29 or 30, wherein the patient is a mammalian patient, particularly a human patient.

32. A kit comprising an anti-AXL-antibody, preferably a monoclonal antibody according to anyone of claims 1 to 10, a nucleic acid sequence according to claim 11 or a vector according to claim 12 or 13.

33. The kit according to claim 32, further comprising a further antineoplastic agent.

34. Use of a humanized anti-AXL antibody according to anyone of claims 1-10 for the manufacture of a pharmaceutical composition for the treatment of drug resistant cancer.

35. Use of a humanized anti-AXL antibody according to anyone of claims 1-9 for the manufacture of a medicament for the co-administration with an antineoplastic agent for the treatment of a hyperproliferative disease.
